# EUROPEAN PATENT SPECIFICATION

(11) **EP 1 396 270 B1**
(45) Date of publication and mention of the grant of the patent: **10.05.2006**
(21) Application number: 03255629.2
(22) Date of filing: 09.09.2003
(51) Int. Cl.: A61K 31/69, C07F 5/02, C07D 207/08

(54) **Boronic acid salts and use thereof in the preparation of medicaments for treating thrombosis**
Borsäuresalze und deren Verwendung in der Bereitstellung von Medikamente für die Thrombosebehandlung
Sels d'acide boronique et leur utilisation dans la préparation de médicaments pour le traitement de la thrombose

(30) Priority: 09.09.2002 GB 0220764; 09.09.2002 GB 0220822; 04.04.2003 GB 0307817; 16.05.2003 GB 0311237; 04.07.2003 GB 0315691; 08.07.2003 US 485786 P
(43) Date of publication of application: 10.03.2004
(73) Proprietor: TRIGEN LIMITED, London SW1A 1ES (GB)
(72) Inventor: Madge, David Jonathan, NCE-Discovery Limited, London WC1E 6AU (GB); Dolman, Mark, NCE-Discovery Limited, London WC1E 6AU (GB); Combe-Marzelle, Sophie Marie, Trigen Limited, London SW1Y 4EL (GB); Deadman, John Joseph, c/o Susan Bartho, Richmond, Victoria 3121 (AU); Kennedy, Anthony James, Trigen Limited, Lodon SW1Y 4EL (GB); Kakkar, Sanjay Kumar, Trigen Limited, London SW1Y 4EL (GB)
(74) Representative: Harrison Goddard Foote

(56) References cited:
- WO-A-95/09634
- WO-A-95/09859
- WO-A-02/059130
- WO-A-02/059131
- US-A- 5 444 049
- US-A- 5 462 964
- US-A- 5 563 127
- US-A- 5 681 978
- SKORDALAKES EMMANUEL ET AL: "Bifunctional peptide boronate inhibitors of thrombin: Crystallographic analysis of inhibition enhanced by linkage to an exosite 1 binding peptide" BIOCHEMISTRY, vol. 37, no. 41, 13 October 1998 (1998-10-13), pages 14420-14427, XP002262060 ISSN: 0006-2960
- KATZ B A ET AL: "Episelection: novel Ki approximately nanomolar inhibitors of serine proteases selected by binding or chemistry on an enzyme surface." BIOCHEMISTRY, vol. 34, no. 26, 4 July 1995 (1995-07-04), pages 8264-8280, XP002262061 ISSN: 0006-2960
- ELGENDY S ET AL: "New peptide boronic acid inhibitors of thrombin" TETRAHEDRON LETTERS, vol. 33, no. 29, 14 July 1992 (1992-07-14), pages 4209-4212, XP000609104 ISSN: 0040-4039
- SPENCER J ET AL: "Synthesis of ortho-modified mercapto- and piperazino-methyl-phenylboronic acid derivatives" TETRAHEDRON, vol. 58, no. 8, 18 February 2002 (2002-02-18), pages 1551-6, XP004336392 ISSN: 0040-4020
- THROMBOSIS RESEARCH, vol. 101, 2001, pages 171-181,
- JOURNAL OF PHARMACEUTICAL RESEARCH, vol. 89, no. 6, June 2000 (2000-06), pages 758-765,
- JOURNAL OF THE AMERICAN CHEMICAL SOCIETY, vol. 188, no. 5, 1996, pages 950-958,

## Description

### BACKGROUND

The present disclosure relates to pharmaceutically useful products obtainable from organoboronic acids. The disclosure also relates to the use of members of the aforesaid class of products, to their formulation, their preparation, their synthetic intermediates and to other subject matter.

The disclosure further relates to oral pharmaceutical formulations containing the described products.

### Boronic Acid Compounds

It has been known for some years that boronic acid compounds and their derivatives, e.g. esters, have biological activities, notably as inhibitors or substrates of proteases. For example, Koehler et al. *Biochemistry* 10:2477, 1971 report that 2-phenylethane boronic acid inhibits the serine protease chymotrypsin at millimolar levels. The inhibition of chymotrypsin and subtilisin by arylboronic acids (phenylboronic acid, m-nitro-phenylboronic acid, m-aminophenylboronic acid, m-bromophenylboronic acid) is reported by Phillip et al, *Proc. Nat. Acad Sci. USA* 68:478-480, 1971. A study of the inhibition of subtilisin Carlsberg by a variety of boronic acids, especially phenyl boronic acids substituted by Cl, Br, CH₃, H₂N, MeO and others, is described by Seufer-Wasserthal et al, *Biorg. Med. Chem.* 2(1):35-48, 1994.

In describing inhibitors or substrates of proteases, P1, P2, P3, etc. designate substrate or inhibitor residues which are amino-terminal to the scissile peptide bond, and S1, S2, S3, etc., designate the corresponding subsites of the cognate protease in accordance with: Schechter, I. and Berger, A. On the Size of the Active Site in Proteases, *Blochem.Biophys.Res.Comm*., 27:157-162, 1967. In thrombin, the S1 binding site or "specificity pocket" is a well defined slit in the enzyme, whilst the S2 and S3 binding subsites (also respectively called the proximal and distal hydrophobic pockets) are hydrophobic and interact strongly with, respectively, Pro and (R)-Phe, amongst others.

Pharmaceutical research into serine protease inhibitors has moved from the simple arylboronic acids to boropeptides, i.e. peptides containing a boronic acid analogue of an α-amino carboxylic acid. The boronic acid may be derivatised, often to form an ester. Shenvi (EP-A-145441 and US 4499082) disclosed that peptides containing an α-aminoboronic acid with a neutral side chain were effective inhibitors of elastase and has been followed by numerous patent publications relating to boropeptide inhibitors of serine proteases. Specific, tight binding boronic acid inhibitors have been reported for elastase (Kᵢ, 0.25nM), chymotrypsin (Kᵢ, 0.25nM), cathepsin G (Kᵢ, 21nM), α-lytic protease (Kᵢ, 0.25nM), dipeptidyl aminopeptidase type IV (Kᵢ, 16pM) and more recently thrombin (Ac-D-Phe-Pro-boroArg-OH (DuP 714 initial Kᵢ 1.2nM).

Claeson et al (US 5574014 and others) and Kakkar et al (WO 92/07869 and family members including US 5648338) disclose thrombin inhibitors having a neutral C-terminal side chain, for example an alkyl or alkoxyalkyl side chain.

Modifications of the compounds described by Kakkar et al are included in WO 96/25427, directed to peptidyl serine protease inhibitors in which the P2-P1 natural peptide linkage is replaced by another linkage. As examples of non-natural peptide linkages may be mentioned -CO₂-, -CH₂O-, -NHCO-, -CHYCH₂-, -CH=CH-, -CO(CH₂)ₚCO- where p is 1, 2 or 3, -COCHY-, -CO₂-CH₂NH-, -CHY-NX-, -N(X)CH₂-N(X)CO-, -CH=C(CN)CO-, -CH(OH)-NH-, -CH(CN)-NH-, -CH(OH)-CH₂- or -NH-CHOH-, where X is H or an amino protecting group and Y is H or halogen, especially F. Particular non-natural peptide linkages are -CO₂- or -CH₂O-.

Metternich (EP 471651 and US 5288707, the latter being assigned to Trigen Limited) discloses variants of Phe-Pro-BoroArg boropeptides in which the P3 Phe is replaced by an unnatural hydrophobic amino acid such as trimethylsilylalanine, p-tert.butyl-diphenyl-silyloxymethylphenylalanine or p-hydroxymethylphenylalanine and the P1 side chain may be neutral (alkoxyalkyl, alkylthioalkyl or trimethylsilylalkyl).

The replacement of the P2 Pro residue of borotripeptide thrombin inhibitors by an N-substituted glycine is described in Fevig J M et al *Bioorg. Med. Chem.* 8: 301-306 and Rupin A et al *Thromb. Haemost*. 78(4):1221-1227, 1997. See also US 5,585,360 (de Nanteuil et al).

Amparo (WO 96/20698 and family members including US 5698538) discloses peptidomimetics of the structure Aryl-linker-Boro(Aa), where Boro(Aa) may be an aminoboronate residue with a non-basic side chain, for example BoroMpg. The linker is of the formula -(CH₂)ₘCONR- (where m is 0 to 8 and R is H or certain organic groups) or analogues thereof in which the peptide linkage -CONR- is replaced by -CSNR-, -SO₂NR-, -CO₂-, -C(S)O- or -SO₂O-. Aryl is phenyl, naphthyl or biphenyl substituted by one, two or three moieties selected from a specified group. Most typically these compounds are of the structure Aryl-(CH₂)ₙ-CONH-CHR²-BY¹Y², where R² is for example a neutral side chain as described above and n is 0 or 1.

Other aminoboronate or peptidoboronate inhibitors or substrates of serine proteases are described in:
- US 4935493
- EP 341661
- WO 94/25049
- WO 95/09859
- WO 96/12499
- WO 96/20689
- Lee S-L et al, *Biochemistry* 36:13180-13186, 1997
- Dominguez C et al, *Bioorg. Med Chem. Lett*. 7:79-84, 1997
- EP 471651
- WO 94/20526
- WO 95/20603
- WO97/05161
- US 4450105
- US 5106948
- US 5169841.

Unfortunately, organoboronic acids can be relatively difficult to obtain in analytically pure form. Thus, alkylboronic acids and their boroxines are often air-sensitive. Korcek et al, *J. Chem. Soc. Perkin Trans*. 2:242, 1972, teaches that butylboronic acid is readily oxidized by air to generate 1-butanol and boric acid.

It is known that derivatisation of boronic acids as cydic esters provides oxidation resistance. For example, Martichonok V et al *J. Am. Chem. Soc*. 118: 950-958, 1996 state that diethanolamine derivatisation provides protection against possible boronic acid oxidation. US Patent No 5,681,978 (Matteson DS et al) teaches that 1,2-diols and 1,3 diols, for example pinacol, form stable cyclic boronic esters that are not easily oxidised.

Wu et al, *J. Pharm. Sci*., 89:758-765, 2000, discuss the stability of the compound N-(2-pyrazine) carbonyl-phenylalanine-leucine boronic acid (LDP-341, also known as bortezomib), an anti-cancer agent. It is described how "during an effort to formulate [LDP-341] for parenteral administration, the compound showed erratic stability behaviour". The degradation pathways were investigated and it was concluded that the degradation was oxidative, the initial oxidation being attributed to peroxides or molecular oxygen and its radicals.

WO 02/059131 discloses boronic acid products which are described as stable. In particular, these products are certain boropeptides and/or boropeptidomimetics in which the boronic acid group has been derivatised with a sugar.
Some of the disdosed compounds are sugar derivatives of LDP-341.

Many drugs comprise an active moiety which is a carboxylic acid. There are a number of differences between carboxylic acids and boronic acids, whose effects on drug delivery, stability and transport (amongst others) have not been investigated. One feature of trivalent boron compounds is that the boron atom is *sp*² hybridised, which leaves an empty 2*p*_{*z*} orbital on the boron atom. A molecule of the type BX₃ can therefore act as an electron-pair acceptor, or Lewis acid. It can use the empty 2*p*_{*z*} orbital to pick up a pair of nonbonding electrons from a Lewis base to form a covalent bond. BF₃ therefore reacts with Lewis bases such as NH₃ to form acid-base complexes in which all of the atoms have a filled shell of valence electrons.

Boric acid, accordingly, can act as a Lewis acid, accepting OH⁻:

B(OH)₃ + H₂O → B(OH)₄⁻ + H⁺

Further, boronic acids of the type RB(OH)₂ are dibasic and have two pKa's. Another point of distinction about boron compounds is the unusually short length of bonds to boron, for which three factors may be responsible:
1. Formation of pπ-pπ bonds;
2. Ionic-covalent resonance;
3. Reduced repulsions between non-bonding electrons.

The presumed equilibria of boronic and carboxylic acids in aqueous KOH are shown below (excluding formation of RBO₂²⁻):

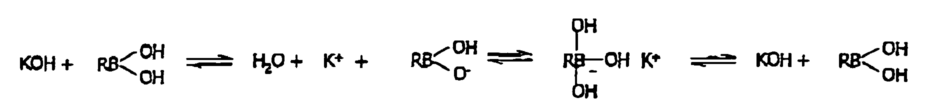

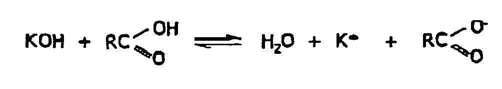

### Thrombosis

Hemostasis is the normal physiological condition of blood in which its components exist in dynamic equilibrium. When the equilibrium is disturbed, for instance following injury to a blood vessel, certain biochemical pathways are triggered leading, in this example, to arrest of bleeding via dot formation (coagulation). Coagulation is a dynamic and complex process in which proteolytic enzymes such as thrombin play a key role. Blood coagulation may occur through either of two cascades of zymogen activations, the extrinsic and intrinsic pathways of the coagulation cascade. The last protease in each pathway is thrombin, which acts to hydrolyze four small peptides (two FpA and two FpB) from each molecule of fibrinogen, thus deprotecting its polymerization sites. Once formed, the linear fibrin polymers may be cross-linked by factor XIIIa, which is itself activated by thrombin. In addition, thrombin is a potent activator of platelets, upon which it acts at specific receptors. Thrombin activation of platelets leads to aggregation of the cells and secretion of additional factors that further accelerate the creation of a hemostatic plug. Thrombin also potentiates its own production by the activation of factors V and VIII.

As indicated above, platelets play two important roles in normal hemostasis. First, by aggregating, they constitute the initial hemostatic plug which immediately curtails bleeding from broken blood vessels. Secondly, the platelet surface can become activated and potentiate blood clotting, a property referred to as platelet procoagulant activity. This may be observed as an increase in the rate of activation of prothrombin by factor Xa in the presence of factor Va and Ca²⁺, referred to as the prothrombinase reaction. Normally, there are few (if any) dotting factors on the surface of unstimulated platelets but, when platelets are activated, negatively charged phospholipids (phosphatidylserine and phospatidylinositol) that are normally on the cytoplasmic side of the membrane become available and provide a surface on which two steps of the coagulation sequence occur. The phospholipid on the surface of activated platelets profoundly accelerates the reactions leading to the formation of thrombin, so that thrombin can be generated at a rate faster than its neutralisation by antithrombin III.

A thrombus can be considered as an abnormal product of a normal mechanism and can be defined as a mass or deposit formed from blood constituents on a surface of the cardiovascular system, for example of the heart or a blood vessel. Thrombosis can be regarded as the pathological condition wherein improper activity of the hemostatic mechanism results in intravascular thrombus formation. Three basic types of thrombi are recognised:
- the white thrombus which is usually seen in arteries and consists chiefly of platelets;
- the red thrombus which is found in veins and is composed predominantly of fibrin and red cells;
- the mixed thrombus which is composed of components of both white and red thrombi.

The composition of thrombi is influenced by the velocity of blood flow at their sites of formation. In general white platelet-rich thrombi form in high flow systems, while red coagulation thrombi form in regions of stasis. The high shear rate in arteries prevents the accumulation of coagulation intermediates on the arterial side of the circulation: only platelets have the capacity to form thrombi binding to the area of damage via von Willebrand factor. Such thrombi composed only of platelets are not stable and disperse. If the stimulus is strong then the thrombi will form again and then disperse continually until the stimulus has diminished. For the thrombus to stabilise, fibrin must form. In this respect, small amounts of thrombin can accumulate within the platelet thrombus and activate factor Va and stimulate the platelet procoagulant activity. These two events lead to an overall increase in the rate of activation of prothrombin by factor Xa of 300,000 fold. Fibrin deposition stabilises the platelet thrombus. Indirect thrombin inhibitors, for example heparin, are not clinically effective at inhibiting stimulation of platelet procoagulant activity. Accordingly, a therapeutic agent which inhibits platelet procoagulant activity would be useful for treating or preventing arterial thrombotic conditions.

On the venous side of circulation, the thrombus is comprised of fibrin: thrombin can accumulate because of the slower flow on the venous side and platelets play only a minor role.

Thrombosis is thus not considered to be a single indication but, rather, is a class of indications embracing distinct sub-classes for which differing therapeutic agents and/or protocols may be appropriate. Thus, regulatory authorities treat disorders such as, for example, deep vein thrombosis, cerebrovascular arterial thrombosis and pulmonary embolism as distinct indications for the purposes of licensing medicines. Two main sub-dasses of thrombosis are arterial thrombosis and venous thrombosis. Arterial thrombosis includes such specific disorders as acute coronary syndromes [for example acute myocardial infarction (heart attack, caused by thrombosis in a coronary artery)], cerebrovascular arterial thrombosis (stroke, caused by thrombosis in the cerebrovascular arterial system) and peripheral arterial thrombosis. Examples of conditions caused by venous thrombosis are deep vein thrombosis and pulmonary embolism.

The management of thrombosis commonly involves the use of antiplatelet drugs (inhibitors of platelet aggregation) to control future thrombogenesis and thrombolytic agents to lyse the newly formed dot, either or both such agents being used in conjunction or combination with anticoagulants. Anticoagulants are used also preventatively (prophylactically) in the treatment of patients thought susceptible to thrombosis.

Currently, two of the most effective classes of drugs in clinical use as anticoagulants are the heparins and the vitamin K antagonists. The heparins are ill-defined mixtures of sulfated polysaccharides that bind to, and thus potentiate, the action of antithrombin III. Antithrombin III is a naturally occurring inhibitor of the activated dotting factors IXa, Xa, XIa, thrombin and probably XIIa (see Jaques, *Pharmacol. Rev*. 31:99-166, 1980). The vitamin K antagonists, of which warfarin is the most well-known example, act indirectly by inhibiting the post-ribosomal carboxylations of the vitamin K dependent coagulation factors II, VII, IX and X (see Hirsch, *Semin. Thromb. Hemostasis* 12:1-11, 1986). While effective therapies for the treatment of thrombosis, heparins and vitamin K antagonists have the unfortunate side effects of bleeding, heparin-induced thrombocytopenia (in the case of heparin) and marked interpatient variability, resulting in a small and unpredictable therapeutic safety margin.

The use of direct acting inhibitors of thrombin and other serine protease enzymes of the coagulation system is expected to alleviate these problems. To that end, a wide variety of serine protease inhibitors have been tested, including boropeptides, i.e. peptides containing a boronic acid analogue of an α-amino acid. Whilst direct acting boronic acid thrombin inhibitors have been discussed earlier in this specification, they are further described in the following section.

### Neutral P1 Residue Boropeptide Thrombin Inhibitors

Claeson et al (US 5574014 and others) and Kakkar et al (WO 92/07869 and family members including US 5648338) disclose lipophilic thrombin inhibitors having a neutral (uncharged) C-terminal (P1) side chain, for example an alkoxyalkyl side chain.

The Claeson et al and Kakkar et al patent families disclose boronate esters containing the amino acid sequence D-Phe-Pro-BoroMpg [(R)-Phe-Pro-BoroMpg], which are highly specific inhibitors of thrombin. Of these compounds may be mentioned in particular Cbz-(R)-Phe-Pro-BoroMpg-OPinacol (also known as TRI 50b). The corresponding free boronic acid is known as TRI 50c. For further information relating to TRI 50b and related compounds, the reader is referred to the following documents:
- Elgendy S et al., in *The Design of Synthetic Inhibitors of Thrombin*, Claeson G et al Eds, *Advances in Experimental Medicine,* 340:173-178, 1993.
- Claeson G et al, *Biochem J*. 290:309-312, 1993
- Tapparelli C et al, *J Biol Chem*, 268:4734-4741, 1993
- Claeson G, in *The Design of Synthetic Inhibitors of Thrombin,* Claeson G et al Eds, *Advances in Experimental Medicine*, 340:83-91, 1993
- Phillip et al, in *The Design of Synthetic Inhibitors of Thrombin,* Claeson G et al Eds, *Advances in Experimental Medicine,* 340:67-77, 1993
- Tapparelli C et al, *Trends Pharmacol, Sci*. 14:366-376, 1993
- Claeson G, *Blood Coagulation and Fibrinolysis* 5:411-436, 1994
- Elgendy et al, *Tetrahedron* 50:3803-3812, 1994
- Deadman J et al, *J. Enzyme Inhibition* 9:29-41, 1995
- Deadman J et al, *J. Medicinal Chemistry* 38:1511-1522, 1995.

The tripeptide sequence of TRI 50b has three chiral centres. The Phe residue is considered to be of (R)-configuration and the Pro residue of natural (5)-configuration, at least in compounds with commercially useful inhibitor activity; the Mpg residue is believed to be of (R)-configuration in isomers with commercially useful inhibitor activity. Thus, the active, or most active, TRI 50b stereoisomer is considered to be of R,S,R configuration and may be represented as:

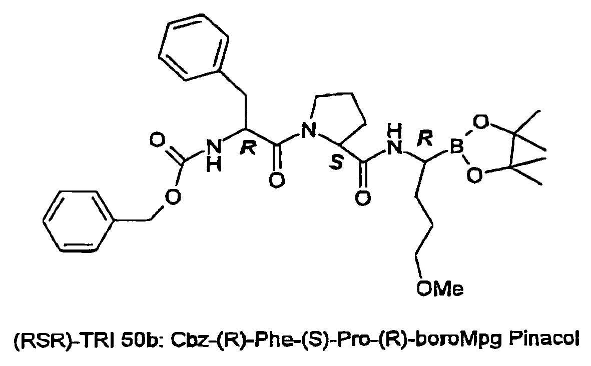

Whilst indirect acting thrombin inhibitors have been found useful for the treatment of patients susceptible to or suffering from venous thrombosis, the same is not true of arterial thrombosis, because it would be necessary to raise the dosage used in the treatment of venous thrombosis by many times in order to treat (prevent) arterial thrombosis. Such raised dosages typically cause bleeding, which makes indirect acting thrombin inhibitors unsuitable or less preferable for treating arterial thrombosis. Heparin and its low molecular weight derivatives are indirect thrombin inhibitors, and so are unsuitable to treat arterial thrombosis. Oral direct thrombin inhibitors are in development for arterial indications but may have lower than desirable therapeutic indices, i.e. may have higher than desirable levels of bleeding at therapeutic doses.

Many organoboronic acid compounds may be classified as lipophilic or hydrophobic. Typically, such compounds include amongst others:
- boropeptides of which all or a majority of the amino acids are hydrophobic
- boropeptides of which at least half of the amino acids are hydrophobic and which have a hydrophobic N-terminal substituent (amino protecting group)
- non-peptides based on hydrophobic moieties.

### Oral Absorption

Absorption in the gastro-intestinal tract can be by an active or a passive route. Active absorption by transport mechanisms tends to be variable between individuals and with intestinal content (Gustafsson et al, *Thrombosis Research*, **2001,** *101,* 171-181). The upper intestine has been identified as the principal site of oral drug absorption. In particular, the duodenum is the customary target site for absorption of orally administered drugs because of its large surface area. The intestinal mucosa acts as a barrier that controls passive transcellular absorption: the absorption of ionic species is blocked whilst the transcellular absorption of lipophilic molecules is favoured (Palm K et al., *J. Pharmacol and Exp. Therapeutics*, **1999,** *291*, 435-443).

Orally administered drugs are required to be consistently and adequately absorbed. Variability of absorption between individuals or between different occasions in the same individual is unwelcome. Similarly, drugs which have a low level of bioavailability (only a small portion of the administered active agent is absorbed) are generally unacceptable.

Non-ionised compounds are favoured for passive absorption, a route associated with invariability, and are therefore preferred for consistent absorption. Lipophilic species are particularly favoured by passive absorption mechanisms and, accordingly, non-ionic, lipophilic drugs are indicated to be most favoured for consistent and high oral absorption.

Typical functionalities required for interaction of drugs with their physiological targets are functional groups such as carboxylic and sulphonic acids. These groups exist as the protonated form in the stomach (at pH 2-3), but will be ionised to some extent at the higher pH of the intestinal fluid. One strategy that has been used to avoid the ionisation of the carboxylates or sulphonates is to present them as ester forms, which are cleaved once absorbed into the vascular lumen.

For example, the direct acting thrombin inhibitor melagatran, which has sub-optimal gastrointestinal absorption, has terminal carboxy and amidino groups and is a pure zwitterion at pH 8-10 when the carboxylic acid and amidino groups are both charged. A prodrug H 376/95 was therefore developed which has protecting groups for the carboxylic acid and for the amidine and is a more lipophilic molecule than melagatran. The prodrug has a permeability coefficient across cultured epithelial Caco-2 cells 80 times higher than that of melagatran and oral bioavailability 2.7-5.5 times higher than that of melagatran as well as much smaller variability in the area under the drug plasma concentration vs. time curve (Gustafsson et al, *Thrombosis Research*, **2001,** *101*, 171-181).

### Oral Absorption of Boropeptides, Boropeptidomimetics and other Organoboronates

The boronate ester group of TRI 50b is rapidly cleaved in the conditions of the plasma to form the corresponding boronic acid group, which is considered to be the active moiety which inhibits the catalytic site of thrombin.

Boronic acids are divalent functional groups, with boron-oxygen bond lengths (1.6Å) more typical of single bonds, unlike superficially comparable C-O and S-O bonds in carboxylic and sulphonic acids. Consequently the boronic acid group has two ionisation potentials. The boronic acid group will be partly ionised at pH's of the duodenal fluid and not suited to the desired passive duodenal uptake. Thus, a charged boronate inhibitor H-D-PheProBoroArg is absorbed by a predominantly active transport mechanism (Saitoh, H. and Aungst, BJ., *Pharm. Res.,* **1999,** *16,* 1786-1789).

The peptide boronic acid formed by such cleavage of TRI 50b (the acid is designated TRI 50c) is relatively insoluble in water, especially at acidic or neutral pH, and tends to be poorly absorbed in the stomach and duodenum. The acid has the structure Cbz-Phe-Pro-BoroMpg-OH.

Whereas the peptide boronic acid Cbz-Phe-Pro-BoroMpg-OH is partly ionised under duodenal conditions and, to that extent, unfavoured for passive transport, esters of the acid are designed for a high rate of passive (thus consistent) transport. The tripeptide sequence Phe-Pro-Mpg has a non-basic P1 side chain (specifically, methoxypropyl), such that the tripeptide consists of three non-polar amino acids. The esters of the peptide boronic acid are non-ionisable and the ester-forming species further impart lipophilic properties, so encouraging a high rate of passive transport.

Computational techniques have confirmed that TRI 50b and other diol esters of Cbz-Phe-Pro-BoroMpg-OH can be predicted to have good bioavailability. Thus, polar surface area (PSAd) is a parameter predictive of bioavailability and PSAd values of greater than 60Å correlate well with passive transcellular transport and with bioavailability of known drugs (Kelder, *J. Pharm. Res*., **1999**, *16*, 1514-1519). Measurements for diol esters of the above peptide boronic acid, including the pinacol ester TRI 50b, show that the diol esters have PSAd values well above 60Å, predictive of passive transport and good bioavailability as shown in Table 1:

**Table 1: PSAd values of selected diol esters of Cbz-Phe-Pro-BoroMpg-OH**

| **Diol** | **PSAd Value** |
|---|---|
| Pinacol | 98.74 |
| Pinanediol | 90.64 |

The corresponding monohydroxy alcohol (e.g. alkanol) esters were considered too unstable, spontaneously cleaving to liberate the acid *in-vitro*. Esters of diols such as pinanediol and pinacol have enhanced kinetic stability over esters of monohydroxy alcohols, in that after partial hydrolysis to the mono-ester derivative they will tend to reassociate by a facile intra-molecular reaction.

### BRIEF SUMMARY OF THE DISCLOSURE

To counterbalance these highly desirable features of TRI 50b, it has been discovered that TRI 50b tends to hydrolyse. Thus in the acid conditions of an HPLC assay, TRI 50b is converted to the acid form with a short half life, which implies potential intraduodenal hydrolysis in the duodenum and elsewhere in the gastro-intestinal tract into ionic species which would resist passive transport and, if anything, be absorbed by active transport, indicative at best of variable bioavailability.

The instability of TRI 50b to hydrolysis also presents potential disadvantages in preparation of the compound and its formulation, as well as in the storage of pharmaceutical formulations containing it.

Another challenging difficulty which has been posed by TRI 50b is that the data show significant variation in bioavailability between subjects. Such variability can make a drug candidate unacceptable and it would therefore be desirable to reduce the observed variability.

An ideal solution to the instability of TRI 50b would be development of a diol ester more stable to hydrolysis, as such a diol ester like TRI 50b can be predicted to be oxidation resistant as compared with TRI 50c. In this regard, it is known that ring size can affect boronate stability and glycolato boron has been shown to have enhanced aqueous stability compared to pinacol (D.S.Matteson, Stereodirected Synthesis with Organoboranes, Springer-Verlag, 1995, ch.1). Similarly, the pinanediol ester is more stable than the pinacol; this is believed to be because the pinanediol group is highly sterically hindered and disfavours nucleophilic attack on the boron. In fact transesterification from pinacol to pinanediol has been reported (Brosz, CS, *Tet. Assym*, 8:1435-1440, 1997) whereas the reverse process is unfavourable. The pinanediol ester however is considered too slow to cleave in plasma and there remains a need to provide an improved diol ester.

Another solution to the instability of TRI 50b would be to administer in its place TRI 50c. However, TRI 50c data suggest that TRI 50c too suffers from variability in bioavailability.

TRI 50c suffers further from instability, in that there is a problematic tendency for the boropeptide moiety itself to degrade via de-boronation (carbon-boron bond cleavage), by a pathway previously considered to be oxidative as taught by the literature (e.g. Wu et al, discussed above). The level of degradation can be remarkably high.

The properties discussed above of TRI 50b and TRI 50c will not be restricted to such compounds but will be shared by other boropeptide esters and acids, even if the properties of such other boropeptides differ quantitatively.

The present disclosure is predicated on, amongst other things, the finding that certain organoboronic acid products are indicated to be of enhanced stability.

The benefits of the present disclosure include a solution to the problem of boronate diol ester and especially TRI 50b instability, that is to say the presently disclosed products provide *inter alia* pharmacologically active compounds which are more stable than TRI 50b and other comparable esters in the sense of stability to hydrolysis. The disdosure further includes a solution to the problem of organoboronic acid instability, that is to say the presently disclosed products provide *inter alia* pharmacologically active compounds which are more stable to deboronation than TRI 50c.

The stability provided within the framework of the disclosure is not absolute but is improved relative to the comparator compounds. The benefits offered by the disclosure further include the provision of unexpected products which have usefulness in oral formulations.

There is disclosed an amino boronic acid derivative which avoids the disadvantages of pinacol esters. The disclosure further indudes a peptide boronic acid derivative which is indicated to be of enhanced stability. In particular, the disclosure includes amongst other subject a pharmaceutical formulation which comprises an amino boronic acid derivative and which can provide in the duodenum an ionic boropeptide species whilst avoiding the disadvantages of pinacol esters, and can provide bioavailability. Further included are boronic acid derivatives which are of relative stability to hydrolysis and deboronation and are useful in oral formulations for inhibiting thrombin.

The disclosure concerns a pharmaceutically acceptable base addition salt of certain organoboronic acid drugs, specifically hydrophobic boropeptides (e.g di- or tri-peptides), and more specifically thrombin inhibitors having a non-basic P1 group. As a class, such salts are not only contrary to the direction of the prior art but additionally have an improved level of stability which cannot be explained or predicted on the basis of known chemistry.

In one aspect, the disclosure relates to pharmaceutically acceptable base addition salts of boronic acids which have a neutral aminoboronic acid residue capable of binding to the thrombin S1 subsite linked through a peptide linkage to a hydrophobic moiety capable of binding to the thrombin S2 and S3 subsites. In a first embodiment, there is disclosed a pharmaceutically acceptable base addition salt of a boronic acid of, for example, formula (I): 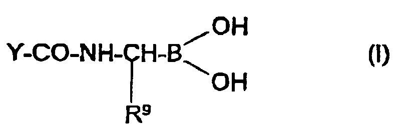 wherein
Y comprises a hydrophobic moiety which, together with the aminoboronic acid residue -NHCH(R⁹)-B(OH)₂, has affinity for the substrate binding site of thrombin; and
R⁹ is a straight chain alkyl group interrupted by one or more ether linkages (e.g. 1 or 2) and in which the total number of oxygen and carbon atoms is 3, 4, 5 or 6 (e.g. 5) or R⁹ is -(CH₂)ₘ-W where m is 2, 3, 4 or 5 (e.g. 4) and W is -OH or halogen (F, Cl, Br or I). R⁹ is an alkoxyalkyl group in one subset of compounds, e.g. alkoxyalkyl containing 4 carbon atoms.

The disclosure comprises pharmaceutically acceptable base addition salts of hydrophobic boronic acid inhibitors of thrombin, and therefore includes such salts of peptide boronic acids which have a partition coefficient between 1-n-octanol and water expressed as log P of greater than 1.0 at physiological pH and 25°C. Some peptide boronic acids useful in the disclosure have a partition coefficient of at least 1.5. A class of hydrophobic peptide boronic acids useful in the disclosure has a partition coefficient of no more than 5.

Disclosed as certain examples are pharmaceutically acceptable base addition salts of hydrophobic boronic acid inhibitors of thrombin. Such inhibitors may contain hydrophobic amino acids, and this class of amino acids includes those whose side chain is hydrocarbyl, hydrocarbyl containing an in-chain oxygen and/or linked to the remainder of the molecule by an in-chain oxygen or heteroaryl, or any of the aforesaid groups when substituted by hydroxy, halogen or trifluoromethyl. Representative hydrophobic side chains include alkyl, alkoxyalkyl, either of the aforesaid when substituted by at least one aryl or heteroaryl, aryl, heteroaryl, aryl substituted by at least one alkyl and heteroaryl substituted by at least one alkyl. Proline and other imino acids which are ring-substituted by nothing or by one of the moieties listed in the previous sentence are also hydrophobic.

Some hydrophobic side chains contain from 1 to 20 carbon atoms, e.g. non-cyclic moieties having 1, 2, 3 or 4 carbon atoms. Side chains comprising a cyclic group typically but not necessarily contain from 5 to 13 ring members and in many cases are phenyl or alkyl substituted by one or two phenyls.

Hydrophobic non-peptides are typically based on moieties which may form a side chain of a hydrophobic amino acid, as described above.

Hydrophobic compounds may contain, for example, one amino group and/or one acid group (e.g. - COOH, -B(OH)₂). Generally, they do not contain multiple polar groups of any one type.

One class of hydrophobic organoboronic acids have a partition coefficient between 1-n-octanol and water expressed as log P of greater than 1 at physiological pH and 25°C. For example, TRI 50c has a partition coefficient of approximately 2.

Some sub-classes of hydrophobic organoboronic acids are those described by Formulae (I) and (III)) below, under the heading "Detailed Description of Several Examples".

Also disclosed as another embodiment is a pharmaceutically acceptable base addition salt of a 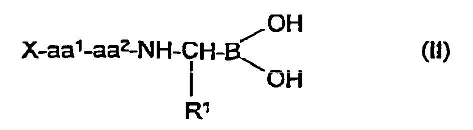 peptide boronic acid of formula (II):
where:
X is a moiety bonded to the N-terminal amino group and may be H to form NH₂. The identity of X is not critical but may be a particular X moiety described above. In one example there may be mentioned benzyloxycarbonyl.
aa¹ is an amino acid having a hydrocarbyl side chain containing no more than 20 carbon atoms (e.g. up to 15 and optionally up to 13 C atoms) and comprising at least one cyclic group having up to 13 carbon atoms. In certain examples, the cyclic group(s) of aa¹ have/has 5 or 6 ring members. For instance, the cyclic group(s) of aa¹ may be aryl groups, particularly phenyl. Typically, there are one or two cyclic groups in the aa¹ side chain. Certain side chains comprise, or consist of, methyl substituted by one or two 5- or 6- membered rings.

More particularly, aa¹ is Phe, Dpa or a wholly or partially hydrogenated analogue thereof. The wholly hydrogenated analogues are Cha and Dcha. The disclosure therefore includes medicaments comprising salts, e.g. metal salts, of organoboronic acids which are thrombin inhibitors, particularly selective thrombin inhibitors, having a neutral P1 (S1-binding) moiety. For more information about moieties which bind to the S3, S2 and S1 sites of thrombin, see for example Tapparelli C et al, *Trends Pharmacol. Sci*. 14: 366-376, 1993; Sanderson P et al, *Current Medicinal Chemistry*, 5: 289-304, 1998; Rewinkel J et al, *Current Pharmaceutical Design,* 5:1043-1075, 1999; and Coburn C *Exp. Opin. Ther. Patents* 11(5): 721-738, 2001. The thrombin inhibitory salts of the disclosure are not limited to those having S3, S2 and S1 affinity groups described in the publications listed in the preceding sentence.

aa² is an imino acid having from 4 to 6 ring members. Alternatively, aa² is Gly N-substituted by a C₃-C₁₃ hydrocarbyl group, e.g. a C₃-C₈ hydrocarbyl group comprising a C₃-C₆ hydrocarbyl ring; the hydrocarbyl group may be saturated, for example exemplary N-substituents are cyclopropyl, cyclobutyl, cyclopentyl and cydohexyl. As a hydrocarbyl group containing one or more unsaturated bonds may be mentioned phenyl and methyl or ethyl substituted by phenyl, e.g. 2-phenylethyl, as well as β,β-dialkylphenylethyl.

There is a debate in the literature as to whether boronates in aqueous solution form the 'trigonal' B(OH)₂ or 'tetrahedral' B(OH)₃⁻ boron species, but NMR evidence seems to indicate that at a pH below the first pKa of the boronic acid the main boron species is the neutral B(OH)₂. In the duodenum the pH is likely to be between 6 and 7, so the trigonal species is likely to be predominant here. In any event, the symbol -B(OH)₂ includes tetrahedral as well as trigonal boron species, and throughout this specification symbols indicating trigonal boron species embrace also tetrahedral species. The symbol further may further include boronic groups in anhydride form.

The salts may be in the form of solvates, particularly hydrates.

The salts may comprise, or consist essentially of, acid salts in which the boronic acid is singly deprotonated. The disclosure therefore includes products having a metal/boronate stoichiometry consistent with the boronate groups in the product predominantly (more than 50 mol %) carrying a single negative charge.

Oral formulations of the salts are also provided herein. In particular, there are provided oral formulations comprising the salts in the solid phase, for example particulate salts formulated as compressed tablets or as capsules.

As described further hereinafter, there are provided also haemodialysis solutions comprising a salt of the disclosure.

The salts described herein include products obtainable by (having the characteristics of a product obtained by) reaction of the boronic acid with a strong base and the term "salt" herein is to be understood accordingly. The term "salt" in relation to the disclosed products, therefore, does not necessarily imply that the products contain discrete cations and anions and is to be understood as embracing products which are obtainable using a reaction of a boronic acid and a base. The disclosure embraces products which, to a greater or lesser extent, are in the form of a coordination compound. The disclosure thus provides also products obtainable by (having the characteristics of a product obtained by) reaction of a boronic acid (I) with a strong base a well as the therapeutic, including prophylactic, use of such products.

The present disclosure is not limited as to the method of preparation of the salts, provided that they contain a boronate species derived from boronic acid (I) and a counter-ion. Such boronate species may be boronate anions in any equilibrium form thereof. The term "equilibrium form" refers to differing forms of the same compounds which may be represented in an equilibrium equation (e.g. boronic acid in equilibrium with a boronic anhydride and in equilibrium with different boronate ions). Boronates in the solid phase may form anhydrides and the disclosed boronate salts when in the solid phase may comprise boronate anhydrides, as a boronic equilibrium species. It is not required that the salts be prepared by reaction of a base containing the counter-ion and the boronic acid (I). Further, the disclosure includes salt products which might be regarded as indirectly prepared by such an acid/base reaction as well as salts obtainable by (having the characteristics of products obtained by) such indirect preparation. As examples of possibly indirect preparation may be mentioned processes in which, after initial recovery of the salt, it is purified and/or treated to modify its physicochemical properties, for example to modify solid form or hydrate form, or both.

In some embodiments, the cations of the salts are monovalent.

In some embodiments the salts comprise anhydride species; in others they are essentially free of anhydride species.

The salts may be in isolated form. The salts may have a purity, e.g. as determined by the method of Example 28, of at least about 90%, e.g. of greater than or equal to about 95%. In the case of pharmaceutical formulations, such salt forms may be combined with pharmaceutically acceptable diluents, excipients or carriers.

The disclosure includes a method for preparing the salts from the corresponding boronic acid as an intermediate, as well as the intermediate boronic acid of Formula (I) and a method for preparing it.

Further aspects and embodiments of the disclosure are set forth in the following description and daims.

Throughout the description and claims of this specification, the words "comprise" and "contain" and variations of the words, for example "comprising" and "comprises", mean "including but not limited to", and are not intended to (and do not) exclude other moieties, additives, components, integers or steps.

This patent contains data indicating that the stability (resistance to deboronation) of organoboronic acids may be increased by providing them in the form of salts, e.g. metal salts. In single experiments, the ammonium salt of TRI 50c appeared to decompose on drying to yield ammonia, whilst the choline salt demonstrated rapid decomposition to a deboronated impurity. Although experiments have not been conducted to reproduce these unrepeated observations, there is provided a sub-dass in which the ammonium and choline salts are excluded. The salt may be an acid salt. In any event, this stabilisation technique forms part of the disclosure and is applicable, *inter alia,* to organoboronic acids described under the heading "BACKGROUND" and to organoboronic acids described in publications mentioned under that heading.

### BRIEF DESCRIPTION OF THE DRAWINGS

Figure 1 is a plot referred to in Example 32, showing oral phase clearance and kinetics following p.o. dosing with TRI 50b or TRI 50c.
Figure 2 is a second plot referred to in Example 32, showing oral phase clearance and kinetics following intraduodenal dosing with TRI 50b or TRI 50c.

### DETAILED DESCRIPTION OF SEVERAL EXAMPLES

### Glossary

The following terms and abbreviations are used in this specification:

The expression "acid salt" as applied to a salt of a boronic acid refers to salts of which a single -OH group of the trigonally-represented acid group -B(OH)₂ is deprotonated. Thus salts wherein the boronate group carries a single negative charge and may be represented as -B(OH)(O⁻) or as [-B(OH)₃]⁻ are acid salts. The expression encompasses salts of a cation having a valency n wherein the molar ratio of boronic acid to cation is approximately n to 1. In practical terms, the observed stoichiometry is unlikely to be exactly n:1 but will be consistent with a notional n:1 stoichiometry. For example, the observed mass of the cation might vary from the calculated mass for a n:1 stoichiometry by no more than about 10%, e.g. no more than about 7.5%; in some cases an observed mass of a cation might vary from the calculated mass by no more than about 1%. Calculated masses are suitably based on the trigonal form of the boronate. (At an atomic level, a salt stoichiometrically consistent with being an acid salt might contain boronates in a mix of protonation states, whose average approximates to single deprotonation and such "mixed" salts are included in the term "acid salt"). Examples of acid salts are monosodium salts and hemizinc salts.

α-Aminoboronic acid or Boro(aa) refers to an amino acid in which the CO₂ group has been replaced by BO₂.

The term "amino-group protecting moiety" refers to any group used to derivatise an amino group, especially an N-terminal amino group of a peptide or amino acid. Such groups include, without limitation, alkyl, acyl, alkoxycarbonyl, aminocarbonyl, and sulfonyl moieties. However, the term "amino-group protecting moiety" is not intended to be limited to those particular protecting groups that are commonly employed in organic synthesis, nor is it intended to be limited to groups that are readily cleavable.

The phrase "pharmaceutically acceptable" is employed herein to refer to those compounds, materials, compositions, and/or dosage forms which are, within the scope of sound medical judgment, suitable for use in contact with the tissues of human beings or animals without excessive toxicity, irritation, allergic response, or other problem or complication, commensurate with a reasonable benefit/risk ratio.

The expression "thrombin inhibitor" refers to a product which, within the scope of sound pharmacological judgement, is potentially or actually pharmaoeutically useful as an inhibitor of thrombin, and includes reference to substance which comprises a pharmaceutically active species and is described, promoted or authorised as a thrombin inhibitor. Such thrombin inhibitors may be selective, that is they are regarded, within the scope of sound pharmacological judgement, as selective towards thrombin in contrast to other proteases; the term "selective thrombin inhibitor" includes reference to substance which comprises a pharmaceutically active species and is described, promoted or authorised as a selective thrombin inhibitor.

The term "heteroaryl" refers to a ring system which has at least one (e.g. 1, 2 or 3) in-ring heteroatoms and has a conjugated in-ring double bond system. The term "heteroatom" includes oxygen, sulfur and nitrogen, of which sulfur is sometimes less preferred.

"Natural amino acid" means an L-amino acid (or residue thereof) selected from the following group of neutral (hydrophobic or polar), positively charged and negatively charged amino acids:

### Hydrophobic amino acids

A = Ala = alanine
V = Val = valine
I = Ile = isoleucine
L = Leu = leucine
M = Met = methionine
F = Phe = phenylalanine
P = Pro = proline
W = Trp = tryptophan

### Polar (neutral or uncharged) amino acids

N = Asn = asparagine
C = Cys = cysteine
Q = Gin = glutamine
G = Gly = glycine
S = Ser = serine
T = Thr = threonine
Y = Tyr = tyrosine

### Positively charged (basic) amino acids

R = Arg = arginine
H = His = histidine
K = Lys = lysine

### Negatively charged amino acids

D = Asp = aspartic acid
E = Glu = glutamic acid.

ACN = acetonitrile
Amino acid = α-amino acid
Base addition salt = a salt which is prepared from addition of an inorganic base or an organic base to a free acid (in this case the boronic acid).
Cbz = benzyloxycarbonyl
Cha = cyclohexylalanine (a hydrophobic unnatural amino acid)
Charged (as applied to drugs or fragments of drug molecules, e.g. amino acid residues) = carrying a charge at physiological pH, as in the case of an amino, amidino or carboxy group
Dcha = dicyclohexylalanine (a hydrophobic unnatural amino acid)
Dpa = diphenylalanine (a hydrophobic unnatural amino acid)
Drug = a pharmaceutically useful substance, whether the active in vivo principle or a prodrug i.v. = intravenous
Mpg = 3-methoxypropylglycine (a hydrophobic unnatural amino acid)
Multivalent = valency of at least two, for example two or three
Neutral (as applied to drugs or fragments of drug molecules, e.g. amino acid residues) = uncharged = not carrying a charge at physiological pH
Pinac = Pinacol = 2,3-dimethyl-2,3-butanediol
Pinanediol = 2,3-pinanediol = 2,6,6-trimethylbicyclo [3.1.1] heptane-2,3-diol
Pip = pipecolinic acid
p.o. = per os = by way of the mouth (thus an oral formulation is administered p.o.)
s.c. = subcutaneous
Strong base = a base having a sufficiently high pKb to react with a boronic acid. Suitably such bases have a pKb of 7 or more, e.g. 7.5 or more, for example about 8 or more
THF = tetrahydrofuran
Thr = thrombin

### The Compounds

The products of the disclosure comprise base addition salts of boronic acids which have a neutral aminoboronic acid residue capable of binding to the thrombin S1 subsite linked through a peptide linkage to a hydrophobic moiety capable of binding to the thrombin S2 and S3 subsites. The invention more specifically provides the subject matter set forth in the claims and in one aspect includes base addition salts of acids of formula (I): 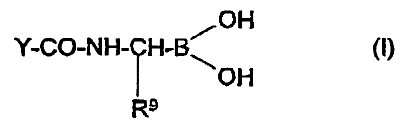 wherein
Y comprises a hydrophobic moiety which, together with the aminoboronic acid residue -NHCH(R⁹)-B(OH)₂, has affinity for the substrate binding site of thrombin; and

R⁹ is a straight chain alkyl group interrupted by one or more ether linkages and in which the total number of oxygen and carbon atoms is 3, 4, 5 or 6 (e.g. 5) or R⁹ is -(CH₂)ₘ-W where m is from 2, 3, 4 or 5 (e.g. 4) and W is -OH or halogen (F, Cl, Br or I). As examples of straight chain alkyl interrupted by one or more ether linkages (-O-) may be mentioned alkoxyalkyl (one interruption) and alkoxyalkoxyalkyl (two interruptions). R⁹ is an alkoxyalkyl group in one subset of compounds, e.g. alkoxyalkyl containing 4 carbon atoms.

Typically, YCO- comprises an amino acid residue (whether natural or unnatural) which binds to the S2 subsite of thrombin, the amino acid residue being N-terminally linked to a moiety which binds the S3 subsite of thrombin.

In one class of Formula (I) acids, YCO- is an optionally N-terminally protected dipeptide residue which binds to the S3 and S2 binding sites of thrombin and the peptide linkages in the acid are optionally and independently N-substituted by a C₁-C₁₃ hydrocarbyl group optionally containing in-chain and/or in-ring nitrogen, oxygen or sulfur and optionally substituted by a substituent selected from halo, hydroxy and trifluoromethyl. The N-terminal protecting group, when present, may be a group X as defined above (other than hydrogen). Normally, the acid contains no N-substituted peptide linkages; where there is an N-substituted peptide linkage, the substituent is often 1C to 6C hydrocarbyl, e.g. saturated hydrocarbyl; the N-substituent comprises a ring in some embodiments, e.g. cycloalkyl, and may be cyclopentyl, for example. One class of acids has an N-terminal protecting group (e.g. an X group) and unsubstituted peptide linkages.

Where YCO- is a dipeptide residue (whether or not N-terminally protected), the S3-binding amino acid residue may be of R configuration and/or the S2-binding residue may of S configuration. The fragment -NHCH(R⁹)-B(OH) may of R configuration. The disclosure is not restricted to chiral centres of these conformations, however.

In one class of compounds, the side chain of the P3 (S3-binding) amino acid and/or the P2 (S2-binding) amino acid is a moiety other than hydrogen selected from a group of formula A or B:

-(CO)ₐ-(CH₂)_{b}-D_{c}-(CH₂)_{d}-E (A)

-(CO)ₐ-(CH₂)_{b}-D_{c}-Cₑ(E¹)(E²)(E³) (B)

wherein
a is 0 or 1;
e is 1;
b and d are independently 0 or an integer such that (b+d) is from 0 to 4 or, as the case may be,
(b+e) is from 1 to 4;
c is 0 or 1;
D is O or S;
E is H, C₁-C₆ alkyl, or a saturated or unsaturated cydic group which normally contains up to 14 members and particularly is a 5-6 membered ring (e.g. phenyl) or an 8-14 membered fused ring system (e.g. naphthyl), which alkyl or cyclic group is optionally substituted by up to 3 groups (e.g. 1 group) independently selected from C₁-C₆ trialkylsilyl, -CN, -R¹³, -R¹²OR¹³, -R¹²COR¹³, -R¹²CO₂R¹³ and -R¹²O₂CR¹³, wherein R¹² is -(CH₂)_{f}- and R¹³ is -(CH₂)_{g}H, or by a moiety whose non-hydrogen atoms consist of carbon atoms and in-ring heteroatoms and number from 5 to 14 and which contains a ring system (e.g. an aryl group) and optionally an alkyl and/or alkylene group,
wherein f and g are each independently from 0 to 10, g particularly being at least 1 (although -OH may also be mentioned as a substituent), provided that (f+g) does not exceed 10, more particularly does not exceed 6 and most particularly is 1, 2, 3 or 4, and provided that there is only a single substituent if the substituent is a said moiety containing a ring system, or E is C₁-C₆ trialkylsilyl; and E¹, E² and E³ are each independently selected from -R¹⁵ and -J-R¹⁵, where J is a 5-6 membered ring and R¹⁵ is selected from C₁-C₆ trialkylsilyl,-CN, -R¹³, -R¹²OR¹³, -R¹²COR¹³, -R¹²CO₂R¹³, -R¹²O₂CR¹³, and one or two halogens (e.g. in the latter case to form a -J-R¹⁵ moiety which is dichlorophenyl), where R¹² and R¹³ are, respectively, an R¹² moiety and an R¹³ moiety as defined above (in some acids where E¹, E² and E³ contain an R¹³ group, g is 0 or 1);
in which moiety of Formula (A) or (B) any ring is carbocyclic or aromatic, or both, and any one or more hydrogen atoms bonded to a carbon atom is optionally replaced by halogen, especially F.

In certain examples, a is 0. If a is 1, c may be 0. In particular examples, (a+b+c+d) and (a+b+c+e) are no more than 4 and are more especially 1, 2 or 3. (a+b+c+d) may be 0.

Exemplary groups for E, E¹, E² and E³ include aromatic rings such as phenyl, naphthyl, pyridyl, quinolinyl and furanyl, for example; non-aromatic unsaturated rings, for example cyclohexenyl; saturated rings such as cydohexyl, for example. E may be a fused ring system containing both aromatic and non-aromatic rings, for example fluorenyl. One class of E, E¹, E² and E³ groups are aromatic (including heteroaromatic) rings, especially 6-membered aromatic rings. In some compounds, E¹ is H whilst E² and E³ are not H; in those compounds, examples of E² and E³ groups are phenyl (substituted or unsubstituted) and C₁-C₄ alkyl, e.g. methyl.

In one class of embodiments, E contains a substituent which is C₁-C₆ alkyl, (C₁-C₅ alkyl)carbonyl, carboxy C₁-C₅ alkyl, aryl (including heteroaryl), especially 5-membered or preferably 6-membered aryl (e.g. phenyl or pyridyl), or arylalkyl (e.g. arylmethyl or arylethyl where aryl may be heterocyclic and is preferably 6-membered).

In another dass of embodiments, E contains a substituent which is OR¹³, wherein R¹³ can be a 6-membered ring, which may be aromatic (e.g. phenyl) or is alkyl (e.g. methyl or ethyl) substituted by such a 6-membered ring.

A class of moieties of formula A or B are those in which E is a 6-membered aromatic ring optionally substituted, particularly at the 2-position or 4-position, by -R¹³ or -OR¹³.

The disclosure includes salts in which the P3 and/or P2 side chain comprises a cyclic group in which 1 or 2 hydrogens have been replaced by halogen, e.g. F or Cl.

The disclosure indudes a class of salts in which the side chains of formula (A) or (B) are of the following formulae (C), (D) or (E):

C_{q}H_{2q}CHT₂ (C)

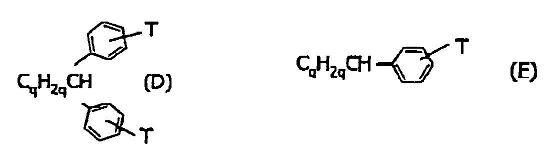

wherein q is from 0 to 5, e.g. is 0, 1 or 2, and each T is independently hydrogen, halogen (e.g. F or Cl), -SiMe₃, -R¹³, -OR¹³, -COR¹³, -CO₂R¹³ or -O₂CR¹³. In some embodiments of structures (D) and (E), T is at the 4-position of the phenyl group(s) and is -R¹³, -OR¹³, -COR¹³, -CO₂R¹³ or -O₂CR¹³, and R¹³ is C₁-C₁₀ alkyl and more particularly C₁-C₆ alkyl. In one sub-class, T is -R¹³ or - OR¹³, for example in which f and g are each independently 0, 1, 2 or 3; in some side chains groups of this sub-class, T is -R¹²-OR¹³ and R¹³ is H.

In one class of the moieties, the side chain is of formula (C) and each T is independently R¹³ or OR¹³ and R¹³ is C₁-C₄ alkyl. In some of these compounds, R¹³ is branched alkyl and in others it is straight chain. In some moieties, the number of carbon atoms is from 1 to 4.

In many dipeptide fragments YCO- (which dipeptides may be N-terminally protected or not), the P3 amino acid has a side chain of formula (A) or (B) as described above and the P2 residue is of an imino acid.

The disclosure therefore includes medicaments comprising salts, e.g. metal salts, of organoboronic acids which are thrombin inhibitors, particularly selective thrombin inhibitors, having a neutral P1 (S1-binding) moiety. For more information about moieties which bind to the S3, S2 and S1 sites of thrombin, see for example Tapparelli C et al, *Trends Pharmacol. Sci*. 14: 366-376, 1993; Sanderson P et al, *Current Medicinal Chemistry*, 5: 289-304, 1998; Rewinkel J et al, *Current Pharmaceutical Design*, 5:1043-1075, 1999; and Coburn C *Exp. Opin. Ther. Patents* 11(5): 721-738, 2001. The thrombin inhibitory salts of the disclosure are not limited to those having S3, S2 and S1 affinity groups described in the publications listed in the preceding sentence.

The boronic acids may have a Ki for thrombin of about 100 nM or less, e.g. about 20 nM or less.

A subset of the Formula (I) acids comprises the acids of Formula (III): 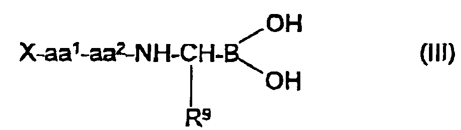 X is a moiety bonded to the N-terminal amino group and may be H to form NH₂. The identity of X is not critical but may be a particular X moiety described above. In one example there may be mentioned benzyloxycarbonyl.

In certain examples X is R⁶-(CH₂)ₚ-C(O)-, R⁶-(CH₂)ₚ-S(O)₂-, R⁶-(CH₂)ₚ-NH-C(O)- or R⁶-(CH₂)ₚ-O-C(O)- wherein p is 0, 1, 2, 3, 4, 5 or 6 (of which 0 and 1 are preferred) and R⁶ is H or a 5 to 13-membered cyclic group optionally substituted by 1, 2 or 3 substituents selected from halogen, amino, nitro, hydroxy, a C₅-C₆ cyclic group, C₁-C₄ alkyl and C₁-C₄ alkyl containing, and/or linked to the 5 to 13-membered cyclic group through, an in-chain O, the aforesaid alkyl groups optionally being substituted by a substituent selected from halogen, amino, nitro, hydroxy and a C₅-C₆ cyclic group. More particularly X is R⁶-(CH₂)ₚ-C(O)- or R⁶-(CH₂)ₚ-O-C(O)- and p is 0 or 1. Said 5 to 13-membered cyclic group is often aromatic or heteroaromatic, for example is a 6-membered aromatic or heteroaromatic group. In many cases, the group is not substituted.

Exemplary X groups are (2-pyrazine) carbonyl, (2-pyrazine) sulfonyl and particularly benzyloxycarbonyl.

aa¹ is an amino acid residue having a hydrocarbyl side chain containing no more than 20 carbon atoms (e.g. up to 15 and optionally up to 13 C atoms) and comprising at least one cyclic group having up to 13 carbon atoms. In certain examples, the cyclic group(s) of aa¹ have/has 5 or 6 ring members. For instance, the cyclic group(s) of aa¹ may be aryl groups, particularly phenyl. Typically, there are one or two cydic groups in the aa¹ side chain. Certain side chains comprise, or consist of, methyl substituted by one or two 5- or 6- membered rings.

More particularly, aa¹ is Phe, Dpa or a wholly or partially hydrogenated analogue thereof. The wholly hydrogenated analogues are Cha and Dcha.

aa² is an imino acid residue having from 4 to 6 ring members.

An exemplary class of products comprises those in which aa² is a residue of an imino acid of formula (IV) 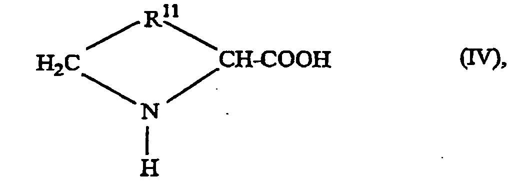 where R¹¹ is -CH₂-, CH₂-CH₂-, -S-CH₂- or -CH₂-CH₂-CH₂-, which group when the ring is 5 or 6-membered is optionally substituted at one or more -CH₂- groups by from 1 to 3 C₁-C₃ alkyl groups, for example to form the R¹¹ group -S-C(CH₃)₂-. Of these imino acids, azetidine-2-carboxylic acid, especially (s)-azetidine-2-carboxylic acid, and more particularly proline are illustrative.

It will be appreciated from the above that a very preferred dass of products consists of those in which aa¹-aa² is Phe-Pro. In another preferred dass, aa¹-aa² is Dpa-Pro. In other products, aa¹⁻aa² is Cha-Pro or Dcha-Pro. Of course, also included are corresponding product classes in which Pro is replaced by (s)-azetidine-2-carboxylic acid.

R⁹ is as defined previously and may be a moiety R¹ of the formula -(CH₂)ₛ-Z. Integer s is 2, 3 or 4 and W is -OH, -OMe, -OEt or halogen (F, Cl, I or, preferably, Br). Particularly illustrative Z groups are -OMe and -OEt, especially -OMe. In certain examples s is 3 for all Z groups and, indeed, for all compounds of the disclosure. Particular R¹ groups are2- bromoethyl, 2-chloroethyl, 2-methoxyethyl, 4-bromobutyl, 4-chlorobutyl, 4-methoxybutyl and, especially, 3-bromopropyl, 3-chloropropyl and 3-methoxypropyl. Most preferably, R¹ is 3-methoxypropyl. 2-Ethoxyethyl is another preferred R¹ group.

Accordingly, a very preferred class of salts consists of those of acids of the formula X-Phe-Pro-Mpg-B(OH)₂, especially Cbz-Phe-Pro-Mpg-B(OH)₂; also preferred are analogues of these compounds in which Mpg is replaced by a residue with another of the particularly preferred R¹ groups and/or Phe is replaced by Dpa or another aa¹ residue.

The aa¹ moiety of the salt is preferably of R configuration. The aa² moiety is preferably of (S)-configuration. Particularly preferred salts have aa¹ of (R)-configurauon and aa² of (S)-configuration. The chiral centre -NH-CH(R¹)-B- is preferably of (R)-configuration. It is considered that commercial formulations will have the chiral centres in (R,S,R) arrangement, as for example in the case of salts of Cbz-Phe-Pro-BoroMpg-OH:

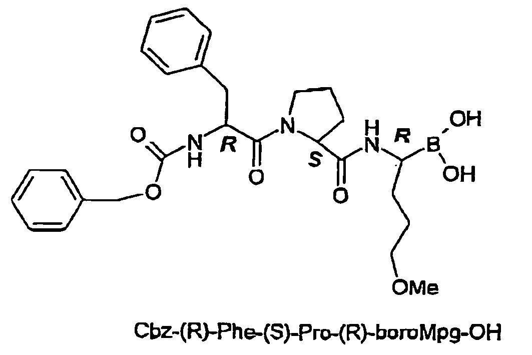

The disclosure includes salts of Cbz-(R)-Phe-(S)-Prtr(R)-boroMpg-OH (and of other compounds of the formula X-(R)-Phe-(5)-Pro-(R)-boroMpg-OH) which are at least 90% pure, e.g. at least 95% pure.

In broad terms, the salts described herein may be considered to correspond to reaction products of an organoboronic acid as described above with a strong base, e.g. a basic metal compound; the salts are however not limited to products resulting from such a reaction and may be obtained by alternative routes.

The salts are therefore obtainable by contacting an acid of formula (I) with a strong base. The disclosure thus contemplates products (compositions of matter) having the characteristics of a reaction product of an acid of formula (I) and a strong base. The base is pharmaceutically acceptable.

As suitable salts may be mentioned:
1. Salts of metals, notably monovalent metals, as which may be mentioned alkali metals;
2. Salts of strongly basic organic nitrogen-containing compounds, including:
   2A. Salts of guanidines and their analogues;
   2B. Salts of strongly basic amine, examples of which include (i) aminosugars and (ii) other amines.

Of the above salts, particularly illustrative are alkali metals, especially Na and Li. Also illustrative are aminosugars.

Specific salts are of the acid boronate though in practice the acid salts may contain a very small proportion of the doubly deportonated boronate. The term "acid boronate" refers to trigonal -B(OH)₂ groups in which one of the B-OH groups is deprotonated as well as to corresponding tetrahedral groups in equilibrium therewith. Acid boronates have a stoichiometry consistent with single deprotonation.

The disclosure includes therefore products (compositions of matter) which comprise salts which may be represented by formula (V): 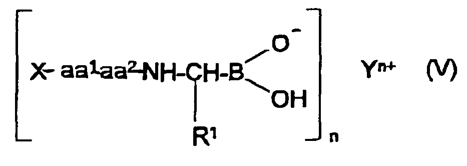 where Yⁿ⁺ is a pharmaceutically acceptable cation obtainable from a strong base, and aa¹, aa², X and R¹ are as defined above. Also included are products in which R¹ is replaced by another R⁹ group.

One class of salts have a solubility of about 10 mM or more, e.g. of at least about 20mM, when their solubility is determined as described in the examples at a dissolution of 25mg/mL More particularly yet they have a solubility of least 50mM when their solubility is determined as described in the examples at a dissolution of 50mg/ml.

The invention includes salts of boronic acids (I) having an observed stoichiometry consistent with the salt being of (being representable by) the formula "(boronate⁻)ₙ cationⁿ⁺". One dass of such salts are represented by the formula:

[Cbz-(R)-Phe-(S)-Pro-(R)-Mpg-B(OH)(O⁻)]M⁺

where M⁺ represents a monovalent cation, especially an alkali metal cation. It will be understood that the above representation is a notional representation of a product whose observed stoichiometry is unlikely to be literally and exactly 1:1. In the above formula, the trigonally-represented boronate represents, as always, boronates which are trigonal, tetrahedral or mixed trigonal/tetrahedral.

The invention also provides a pharmaceutical formulation which comprises a salt of the invention, is adapted for oral administration, and comprises:
a) a first spedes selected from (a) boronic acids of formula (I) or, as the case may be, formula (II), (b) boronate anions thereof, and (c) any equilibrium form of the aforegoing (e.g. anhydride); and
(b) a second species selected from pharmaceutically acceptable metal ions and basic organic nitrogen-containing compounds having a pKb of about 7 or more (which compounds will be understood to comprise the compounds in protonated form),
wherein the formulation has an observed stoichiometry which would be consistent with the first species being boronate ions carrying a single negative charge.

Particularly exemplary are products which comprise:
(i) species selected from (a) acids of formula (VIII): X-(R)-Phe-(S)-Pro-(R)-Mpg-B(OH)₂ where X is H or an amino-protecting group, especially Cbz, and (b) boronate anions thereof; and
(ii) ions having a valency n in combination with said species, the species and said ions having an observed stoichiometry consistent with a notional species:ion stoichiometry of n:1. In one class of salts, V is 1.

Considering the counter-ions in turn:

### 1. Monovalent metal, especially alkali metal salts

Suitable alkali metals include lithium, sodium and potassium. All of these are remarkably soluble. Lithium and sodium are illustrative because of their high solubility. The lithium and particularly sodium salts are of surprisingly high solubility in relation to potassium amongst others. Sodium is most used in many instances. Salts containing mixtures of alkali metals are contemplated by the invention.

The invention includes products comprising salts of the formula (VI) 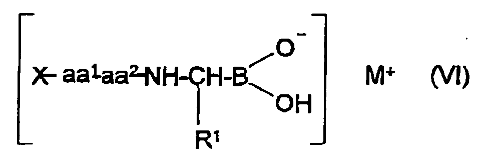 where M⁺ is an alkali metal ion and aa¹, aa², X and R¹ are as defined above, as well as salts in which both hydroxy groups of the boronate group are in salt form (preferably with another identical M⁺ group) and mixtures of such salts. Included also are products wherein R¹ is replaced by another R⁹ group.

### 2. Strongly basic organic nitrogen-containing compounds

The invention includes products obtainable by (having the characteristics of a product obtained by) reaction of a peptide boronic acid as defined above and a strong organic base. Two preferred classes of organic base are described in sections 2A and 2B below. Particularly preferred are acid salts (in which one of the two boronic -OH groups is deprotonated). Most commonly, the salts contain a single type of organic counter-ion (disregarding trace contaminants) but the invention contemplates salts containing mixtures of organic counter-ions; in one sub-dass, the different counter-ions all fall within the section 2A family described below or, as the case may be, in the section 2B family below; in another subclass, the salts comprise a mixture of organic counter-ions which are not all from the same family (2A or 2B).

Suitable organic bases include those with a pKb of 7 or more, e.g. 7.5 or more, for example in the region of 8 or more. Bases which are less lipophilic [e.g. have at least one polar functional group (e.g. 1, 2 or 3 such groups) for example hydroxy] are favoured; thus aminosugars are one favoured class of base.

### 2A. Guanidines and their analogues

The guanidino compound (guanidine) may in principle be any soluble and pharmaceutically acceptable compound having a guanidino or a substituted guanidino group, or a substituted or unsubstituted guanidine analogue. Suitable substituents include aryl (e.g. phenyl), alkyl or alkyl interrupted by an ether or thioether linkage and, in any event, typically contain from 1 to 6 and especially 1, 2, 3, or 4 carbon atoms, as in the case of methyl or ethyl. The guanidino group may have 1, 2, 3 or 4 substituent groups but more usually has 1 or 2 substituent groups, for instance on a terminal nitrogen. One class of guanidines is monoalkylated; another class is dialkylated. As guanidine analogues may be mentioned thioguanidines and 2-amino pyridines. Compounds having unsubstituted guanidino groups, for example guanidine and arginine, form one particular class.

Salts containing mixtures of guanidines are contemplated by the invention.

A particular guanidino compound is L-arginine or an L-arginine analogue, for example D-arginine, or the D- or, preferably, L- isomers of homoarginine or agmatine [(4-aminobutyl) guanidine]. Less preferred arginine analogues are NG-nitro-L-arginine methyl ester, for example, and constrained guanidine analogues, particularly 2-amino pyrimidines, for example 2,6-quinazolinediamines such as 5,6,7,8-tetrahydro-2,6-quinazolinediamine, for example. The guanidino compound may also be a peptide, for example a dipeptide, containing arginine; one such dipeptide is L-tyrosyl-L-arginine.

Some particular guanidino compounds are compounds of formula (VII): 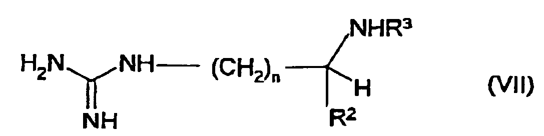 where n is from 1 to 6 and for example at least 2, e.g. 3 or more, and in many instances no more than 5. Most particularly, n is 3, 4 or 5, R² is H or carboxylate or derivatised carboxylate, for example to form an ester (e.g. a C₁-C₄ alkyl ester) or amide. R³ is H, C₁-C₄ alkyl or a residue of a natural or unnatural amino acid (e.g. tyrosine). The compounds of formula (IV) are usually of L-configuration. The compounds of formula (IV) are arginine (n=3; R²=carboxyl; R³=H) and arginine derivatives or analogues.

The invention includes products comprising salts of the formula (IX) 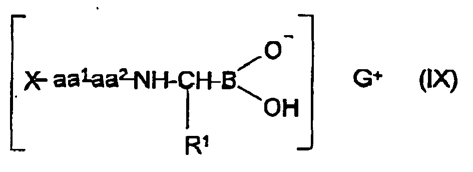 where aa¹, aa², X and R¹ are as defined previously and G⁺ is the protonated form of a pharmaceutically acceptable organic compound comprising a guanidino group or an analogue thereof, as well as salts in which both hydroxy groups of the boronate group are in salt form (preferably with another identical G⁺ group) and mixtures of such salts. Also included are products wherein R¹ is replaced by another R⁹ group.

### 2B. Strongly basic amines

The invention includes products obtainable by (having the characteristics of a product obtained by) reaction of a peptide boronic acid as defined above and a strong organic base which is an amine. The amine may in principle be any soluble and pharmaceutically acceptable amine.

It is envisaged that a desirable class of amine includes those having polar functional groups in addition to a single amine group, as such compounds will be more hydrophilic and thus more soluble than others. In certain salts, the or each additional functional group is hydroxy. Some amines have 1, 2, 3, 4, 5 or 6 additional functional groups, especially hydroxy groups. In one illustrative class of amines the ratio of (amino plus hydroxy groups):carbon atoms is from 1:2 to 1:1, the latter ratio being particularly preferred. These amines with one or more additional polar functional groups may be a hydrocarbon, especially an alkane, substituted by the amino group and the additional polar group(s). The amino group may be substituted or unsubstituted and, excluding amino substituents, the polar base may contain, for example, up to 10 carbon atoms; usually there are no less than three such carbon atoms, e.g. 4, 5 or 6. Aminosugars are included in this category of polar bases.

The invention includes products comprising salts of the formula (X) 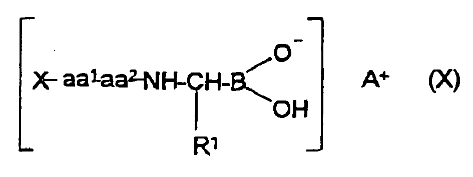 where aa¹, aa², X and R¹ are as defined previously and A⁺ is the protonated form of a pharmaceutically acceptable amine, as well as salts in which both hydroxy groups of the boronate group are in salt form (preferably with another identical A⁺ group) and mixtures of such salts. In one class of such products, A⁺ is the protonated form of an amine described in section 2B(i) below; in another class A⁺ is the protonated form of an amine described in 2B(ii) below. Also included are products in which R¹ is replaced by another R⁹ group.

Two illustrative classes of amine base are described in sections 2B(i) and 2B(ii) below. Particularly preferred are acid salts (in which one of the two boronic -OH groups is deprotonated). Most commonly, the salts contain a single type of amine counter-ion (disregarding trace contaminants) but the invention contemplates salts containing mixtures of amine counter-ions; in one sub-class, the different counter-ions all fall within the sub-section 2B(i) family described below or, as the case may be, in the sub-section 2B(ii) family below; in another subclass, the salts comprise a mixture of organic counter-ions which are not all from the same family (2B(i) or 2B(ii)).

### 2B(i) Aminosugars

The identity of the aminosugar is not critical to the invention. Preferred aminosugars include ring-opened sugars, especially glucamines. Cyclic aminosugars are also envisaged as useful. One class of the aminosugars is N-unsubstituted and another, preferred, class is N-substituted by one or two N-substituents (e.g. one). Suitable substituents are hydrocarbyl groups, for example and without limitation containing from 1 to 12 carbon atoms; the substituents may comprise alkyl or aryl moieties or both. Exemplary substituents are C₁, C₂, C₃, C₄, C₅, C₆, C₇ and C₈ alkyl groups, in particular methyl and ethyl, of which methyl is illustrative. Data indicate that aminosugars, especially N-methyl-D-glucamine, are of surprisingly high solubility.

A most preferred aminosugar is N-methyl-D-glucamine:

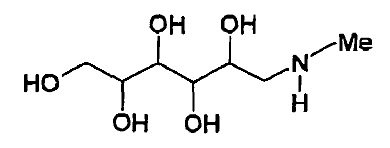

### 2B(ii) Other amines

Other suitable amines include amino acids (whether naturally occurring or not) whose side chain is substituted by an amino group, especially lysine.

Some amines are compounds of formula (XI): 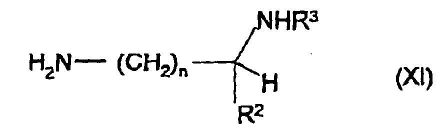 where n, R² and R³ are as defined in relation to formula (IV). The compounds of formula (VI) are usually of L-configuration. The compounds of formula (VI) are lysine (n=4; R²=carboxyl; R³=H) and lysine derivatives or analogues. A most preferred amine is L-lysine.

Other suitable amines are nitrogen-containing heterocycles. At least usually, such heterocyclic compounds are alicyclic; one class of the heterocyclic compounds is N-substituted and another, preferred, class is N-unsubstituted. The heterocycles may contain 6 ring-forming atoms, as in the cases of piperidine, piperazine and morpholine. One class of amines includes N-containing heterocycles substituted by polar substituents, especially hydroxy, e.g. 1, 2 or 3 times.

The invention therefore includes amines other than aminosugars which have one or more (e.g. 1, 2, 3, 4, 5 or 6) polar substituents, especially hydroxy, in action to one amine group. Such compounds may have a ratio of (amino plus hydroxy gro) carbon atoms of 1:2 to 1:1, the latter ratio being particularly preferred.

The invention indudes mixed salts, i.e. salts containing a mixture of boropetide moieties and/or counterions but single salts are preferred.

The salts in solid form may contain a solvent, e.g. water. There are included a dass of products in which the salts are essentially anhydrous. Also included is a class in which the salts are hydrates.

### Use of the Products of the Disclosure

The salts of the disdosure are thrombin inhibitors. They are therefore useful for inhibiting thrombin. There are therefore provided compounds which have potential for controlling haemostasis and especially for inhibiting coagulation, for example in the treatment or prevention of secondary events after myocardial infarction. The medical use of the compounds may be prophylactic (induding to treat thrombosis as well as to prevent occurrence of thrombosis) as well as therapeutic (including to prevent re-occurrence of thrombosis or secondary thrombotic events).

The salts may be employed when an anti-thrombogenic agent is needed. Further, it has been found that the salts, including those of boronic acids of Formula (III), are beneficial in that the class is useful for treating arterial thrombosis by therapy or prophylaxis. The disclosed salts are thus indicated in the treatment or prophylaxis of thrombosis and hypercoagulability in blood and tissues of animals induding man. The term "thrombosis" includes *inter alia* atrophic thrombosis, arterial thrombosis, cardiac thrombosis, coronary thrombosis, creeping thrombosis, infective thrombosis, mesenteric thrombosis, placental thrombosis, propagating thrombosis, traumatic thrombosis and venous thrombosis.

It is known that hypercoagulability may lead to thromboembolic diseases.

Examples of venous thromboembolism which may be treated or prevented with compounds of the disclosure include obstruction of a vein, obstruction of a lung artery (pulmonary embolism), deep vein thrombosis, thrombosis associated with cancer and cancer chemotherapy, thrombosis inherited with thrombophilic diseases such as Protein C deficiency, Protein S deficiency, antithrombin III deficiency, and Factor V Leiden, and thrombosis resulting from acquired thrombophilic disorders such as systemic lupus erythematosus (inflammatory connective tissue disease). Also with regard to venous thromboembolism, compounds of the disclosure are useful for maintaining patency of indwelling catheters.

Examples of cardiogenic thromboembolism which may be treated or prevented with compounds of the disclosure include thromboembolic stroke (detached thrombus causing neurological affliction related to impaired cerebral blood supply), cardiogenic thromboembolism associated with atrial fibrillation (rapid, irregular twitching of upper heart chamber muscular fibrils), cardiogenic thromboembolism associated with prosthetic heart valves such as mechanical heart valves, and cardiogenic thromboembolism associated with heart disease.

Examples of conditions involving arterial thrombosis include unstable angina (severe constrictive pain in chest of coronary origin), myocardial infarction (heart muscle cell death resulting from insufficient blood supply), ischemic heart disease (local ischemia due to obstruction (such as by arterial narrowing) of blood supply), reocdusion during or after percutaneous transluminal coronary angioplasty, restenosis after percutaneous transluminal coronary angioplasty, occlusion of coronary artery bypass grafts, and occlusive cerebrovascular disease. Also with regard to arterio-venous (mixed) thrombosis, anti-thrombotic compounds of the disdosure are useful for maintaining patency in arteriovenous shunts.

Other conditions associated with hypercoagulability and thromboembolic diseases which may be mentioned inherited or acquired deficiencies in heparin cofactor II, circulating antiphospholipid antibodies (Lupus anticoagulant), homocysteinemia, heparin induced thrombocytopenia and defects in fibrinolysis.

Particular uses which may be mentioned include the therapeutic and/or prophylactic treatment of venous thrombosis and pulmonary embolism. Preferred indications envisaged for the products of the disclosure (notably the salts of TRI 50c) include:
- Prevention of venous thromboembolic events (e.g. deep vein thrombosis and/or pulmonary embolism). Examples include patients undergoing orthopaedic surgery such as total hip replacement, total knee replacement, major hip or knee surgery; patients undergoing general surgery at high risk for thrombosis, such as abdominal or pelvic surgery for cancer; and in patients bedridden for more than 3 days and with acute cardiac failure, acute respiratory failure, infection.
- Prevention of thrombosis in the haemodialysis circuit in patients, in patients with end stage renal disease.
- Prevention of cardiovascular events (death, myocardial infarction, etc) in patients with end stage renal disease, whether or not requiring haemodialysis sessions.
- Prevention of venous thrombo-embolic events in patients receiving chemotherapy through an indwelling catheter.
- Prevention of thromboembolic events in patients undergoing lower limb arterial reconstructive procedures (bypass, endarteriectomy, transluminal angioplasty, etc).
- Treatment of venous thromboembolic events.
- Prevention of cardiovascular events in acute coronary syndromes (e.g. unstable angina, non Q wave myocardial ischaemia/infarction), in combination with another cardiovascular agent, for example aspirin (acetylsalicylic acid; aspirin is a registered trade mark in Germany), thrombolytics (see below for examples), antiplatelet agents (see below for examples).
- Treatment of patients with acute myocardial infarction in combination with acetylsalicylic acid, thrombolytics (see below for examples).

The thrombin inhibitors of the disclosure are thus indicated both in the therapeutic and/or prophylactic treatment of all the aforesaid disorders.

In one method, the products of the disclosure are used for the treatment of patients by haemodialysis, by providing the product in the dialysis solution, as described in relation to other thrombin inhibitors in WO 00/41715. The disclosure therefore includes dialysing solutions and dialysing concentrates which comprise a product of the disclosure, as well as a method of treatment by dialysis of a patient in need of such treatment, which method comprises the use of a dialysing solution including a low molecular weight thrombin inhibitor. Also included is the use of an anti-thrombotic product of the disclosure for the manufacture of a medicament for the treatment by dialysis of a patient in which the anti-thrombotic product of the disclosure is provided in the dialysing solution.

In another method, the products of the disclosure are used to combat undesirable cell proliferation, as described in relation to other thrombin inhibitors in WO 01/41796. The undesirable cell proliferation is typically undesirable hyperplastic cell proliferation, for example proliferation of smooth muscle cells, especially vascular smooth muscle cells. The products of the disclosure particularly find application in the treatment of intimal hyperplasia, one component of which is proliferation of smooth musde cells. Restenosis can be considered to be due to neointimal hyperplasia; accordingly intimal hyperplasia in the context of the disclosure includes restenosis.

The products of the disclosure are also contemplated for the treatment of ischemic disorders. More particularly, they may be used in the treatment (whether therapeutic or prophylactic) of an ischemic disorder in a patient having, or at risk of, non-valvular atrial fibrillation (NVAF) as described in relation to other thrombin inhibitors in WO 02/36157. Ischemic disorders are conditions whose results include a restriction in blood flow to a part of the body. The term will be understood to include thrombosis and hypercoagulability in blood, tissues and/or organs. Particular uses that may be mentioned include the prevention and/or treatment of ischemic heart disease, myocardial infarction, systemic embolic events in e.g. the kidneys or spleen, and more particularly of cerebral ischemia, including cerebral thrombosis, cerebral embolism and/or cerebral ischemia associated with non-cerebral thrombosis or embolism (in other words the treatment (whether therapeutic or prophylactic) of thrombotic or ischemic stroke and of transient ischemic attack), particularly in patients with, or at risk of, NVAF.

The products of the disclosure are also contemplated for the treatment of rheumatic/arthritic disorders, as described in relation to other thrombin inhibitors in WO 03/007984. Thus, the products of the disclosure may be used in the treatment of chronic arthritis, rheumatoid arthritis, osteoarthritis or ankylosing spondylitis

Moreover, the products of the disclosure are expected to have utility in prophylaxis of re-occlusion (i.e. thrombosis) after thrombolysis, percutaneous trans-luminal angioplasty (PTA) and coronary bypass operations; the prevention of re-thrombosis after microsurgery and vascular surgery in general. Further indications include the therapeutic and/or prophylactic treatment of disseminated intravascular coagulation caused by bacteria, multiple trauma, intoxication or any other mechanism; anticoagulant treatment when blood is in contact with foreign surfaces in the body such as vascular grafts, vascular stents, vascular catheters, mechanical and biological prosthetic valves or any other medical device; and anticoagulant treatment when blood is in contact with medical devices outside the body such as during cardiovascular surgery using a heart-lung machine or in haemodialysis.

The products of the disclosure are further indicated in the treatment of conditions where there is an undesirable excess of thrombin without signs of hypercoagulability, for example in neurodegenerative diseases such as Alzheimer's disease. In addition to its effects on the coagulation process, thrombin is known to activate a large number of cells (such as neutrophils, fibroblasts, endothelial cells and smooth muscle cells). Therefore, the compounds of the disclosure may also be useful for the therapeutic and/or prophylactic treatment of idiopathic and adult respiratory distress syndrome, pulmonary fibrosis following treatment with radiation or chemotherapy, septic shock, septicaemia, inflammatory responses, which include, but are not limited to, edema, acute or chronic atherosclerosis such as coronary arterial disease, cerebral arterial disease, peripheral arterial disease, reperfusion damage, and restenosis after percutaneous transluminal angioplasty (PTA).

The salts may also be useful in the treatment of pancreatitis.

The salts described herein are further considered to be useful for inhibiting platelet procoagulant activity. The disclosure provides a method for inhibiting platelet pro-coagulant activity by administering a salt of a boronic acid described herein to a mammal at risk of, or suffering from, arterial thrombosis, particularly a human patient. Also provided is the use of such salts for the manufacture of medicaments for inhibiting platelet procoagulant activity.

The use of products of the disclosure as inhibitors of platelet pro-coagulant activity is predicated on the observation that the boronic acids described herein are indicated to be effective at inhibiting arterial thrombosis as well as venous thrombosis.

Indications involving arterial thrombosis include acute coronary syndromes (especially myocardial infarction and unstable angina), cerebrovascular thrombosis and peripheral arterial occlusion and arterial thrombosis occurring as a result of atrial fibrillation, valvular heart disease, arterio-venous shunts, indwelling catheters or coronary stents. Accordingly, in another aspect there is provided a method of treating a disease or condition selected from this group of indications, comprising administering to a mammal, especially a human patient, a salt of the disclosure. The disclosure includes products for use in an arterial environment, e.g. a coronary stent or other arterial implant, having a coating which comprises a salt according to the disclosure.

The salts of the disclosure may be used prophylactically to treat an individual believed to be at risk of suffering from arterial thrombosis or a condition or disease involving arterial thrombosis or therapeutically (including to prevent re-occurrence of thrombosis or secondary thrombotic events).

There is therefore included the use of selective thrombin inhibitors (organoboronic acid salts) described herein for treatment of the above disorders by prophylaxis or therapy as well as their use in pharmaceutical formulations and the manufacture of pharmaceutical formulations.

### Administration and Pharmaceutical Formulations

The salts may be administered to a host, for example, in the case where the drug has anti-thrombogenic activity, to obtain an anti-thrombogenic effect. In the case of larger animals, such as humans, the compounds may be administered alone or in combination with pharmaceutically acceptable diluents, excipients or carriers. The term "pharmaceutically acceptable" includes acceptability for both human and veterinary purposes, of which acceptability for human pharmaceutical use is preferred. In the case of oral administration, the compounds are preferably administered in a form which prevents the salt of the disclosure from contact with the acidic gastric juice, such as enterically coated formulations, which thus prevent release of the salt of the disclosure until it reaches the duodenum.

The enteric coating is suitably made of carbohydrate polymers or polyvinyl polymers, for example. Examples of enteric coating materials indude, but are not limited to, cellulose acetate phthalate, cellulose acetate succinate, cellulose hydrogen phthalate, cellulose acetate trimellitate, ethyl cellulose, hydroxypropyl-methylcellulose phthalate, hydroxypropylmethylcellulose acetate succinate, carboxymethyl ethylcellulose, starch acetate phthalate, amylose acetate phthalate, polyvinyl acetate phthalate, polyvinyl butyrate phthalate, styrene-maleic acid copolymer, methyl-acrylate-methacrylic acid copolymer (MPM-05), methylacrylate-methacrylic acid-methylmethacrylate copolymer (MPM-06), and methylmethacrylate-methacrylic acid co-polymer (Eudragit® L & S). Optionally, the enteric coating contains a plasticiser. Examples of the plasticiser include, but are not limited to, triethyl citrate, triacetin, and diethyl phthalate.

The salts of the disclosure may be combined and/or co-administered with any cardiovascular treatment agent. There are large numbers of cardiovascular treatment agents available in commercial use, in clinical evaluation and in pre-clinical development, which could be selected for use with a product of the disclosure for the prevention of cardiovascular disorders by combination drug therapy. Such agent can be one or more agents selected from, but not limited to several major categories, namely, a lipid-lowering drug, including an IBAT (ileal Na⁺/bile acid cotransporter) inhibitor, a fibrate, niacin, a statin, a CETP (cholesteryl ester transfer protein) inhibitor, and a bile acid sequestrant, an anti-oxidant, including vitamin E and probucol, a IIb/IIIa antagonist (e.g. abciximab, eptifibatide, tirofiban), an aldosterone inhibitor (e.g. spirolactone and epoxymexrenne). an adenosine A2 receptor antagonist (e.g. losartan), an adenosine A3 receptor agonist, a beta-blocker, acetylsalicylic acid, a loop diuretic and an ACE (angiotensin converting enzyme) inhibitor.

The salts of the disclosure may be combined and/or co-administered with any antithrombotic agent with a different mechanism of action, such as the antiplatelet agents acetylsalicylic acid, ticlopidine, dopidogrel, thromboxane receptor and/or synthetase inhibitors, prostacydin mimetics and phosphodiesterase inhibitors and ADP-receptor (P₂ T) antagonists.

The products of the disclosure may further be combined and/or co-administered with thrombolytics such as tissue plasminogen activator (natural, recombinant or modified), streptokinase, urokinase, prourokinase, anisoylated plasminogen-streptokinase activator complex (APSAC), animal salivary gland plasminogen activators, and the like, in the treatment of thrombotic diseases, in particular myocardial infarction.

The salts of the disclosure may be combined and/or co-administered with a cardioprotectant, for example an adenosine A1 or A3 receptor agonist.

There is also provided a method for treating an inflammatory disease in a patient that comprises treating the patient with a product of the disclosure and an NSAID, e.g., a COX-2 inhibitor. Such diseases include but are not limited to nephritis, systemic lupus, erythematosus, rheumatoid arthritis, glomerulonephritis, vasculitis and sarcoidosis. Accordingly, the anti-tnrombotic salts of the disclosure may be combined and/or co-administered with an NSAID.

Typically, therefore, the salts described herein may be administered to a host to obtain a thrombin-inhibitory effect, or in any other thrombin-inhibitory or anti-thrombotic context mentioned herein.

Actual dosage levels of active ingredients in the pharmaceutical compositions of this disclosure may be varied so as to obtain an amount of the active compound(s) that is effective to achieve the desired therapeutic response for a particular patient, composition, and mode of administration (referred to herein as a "therapeutically effective amount"). The selected dosage level will depend upon the activity of the particular compound, the severity of the condition being treated and the condition and prior medical history of the patient being treated. However, it is within the skill of the art to start doses of the compound at levels lower than required for to achieve the desired therapeutic effect and to gradually increase the dosage until the desired effect is achieved.

For example, it is currently contemplated that, in the case of oral administration of salts of TRI 50c, the salts might for instance be administered in an amount of from 0.5 to 2.5mg/Kg twice daily, calculated as TRI 50c. Other salts might be administered in equivalent molar amounts. The disclosure is not limited to administration in such quantities or regimens and includes dosages and regimens outside those described in the previous sentence.

According to a further aspect of the disclosure there is provided an oral pharmaceutical formulation including a product of the disclosure, in admixture with a pharmaceutically acceptable adjuvant, diluent or carrier.

Solid dosage forms for oral administration include capsules, tablets (also called pills), powders and granules. In such solid dosage forms, the active compound is typically mixed with at least one inert, pharmaceutically acceptable excipient or carrier such as sodium citrate or dicalcium phosphate and/or one or more: a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite day and i) lubricants such as talc, calcium stearate, magnesium stearate, solid polyethylene glycols, sodium lauryl sulfate and mixtures thereof. In the case of capsules and tablets, the dosage form may also comprise buffering agents. Solid compositions of a similar type may also be employed as fillers in soft and hard-filled gelatin capsules using such excipients as lactose or milk sugar as well as high molecular weight polyethylene glycol, for example.

Suitably, the oral formulations may contain a dissolution aid. The dissolution aid is not limited as to its identity so long as it is pharmaceutically acceptable. Examples include nonionic surface active agents, such as sucrose fatty acid esters, glycerol fatty acid esters, sorbitan fatty acid esters (e.g., sorbitan trioleate), polyethylene glycol, polyoxyethylene hydrogenated castor oil, polyoxyethylene sorbitan fatty acid esters, polyoxyethylene alkyl ethers, methoxypolyoxyethylene alkyl ethers, polyoxyethylene alkylphenyl ethers, polyethylene glycol fatty acid esters, polyoxyethylene alkylamines, polyoxyethylene alkyl thioethers, polyoxyethylene polyoxypropylene copolymers, polyoxyethylene glycerol fatty acid esters, pentaerythritol fatty acid esters, propylene glycol monofatty acid esters, polyoxyethylene propylene glycol monofatty acid esters, polyoxyethylene sorbitol fatty acid esters, fatty acid alkylolamides, and alkylamine oxides; bile acid and salts thereof (e.g., chenodeoxycholic acid, cholic acid, deoxycholic acid, dehydrocholic acid and salts thereof, and glycine or taurine conjugate thereof); ionic surface active agents, such as sodium laurylsulfate, fatty acid soaps, alkylsulfonates, alkylphosphates, ether phosphates, fatty acid salts of basic amino acids; triethanolamine soap, and alkyl quaternary ammonium salts; and amphoteric surface active agents, such as betaines and aminocarboxylic acid salts.

The active compounds may also be in micro-encapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan and mixtures thereof. Besides inert diluents, the oral compositions may also include adjuvants such as wetting agents, emulsifying and suspending agents, sweetening, flavouring and perfuming agents. Suspensions, in addition to the active compounds, may contain suspending agents such as ethoxylated isostearyl alcohols, polyoxyethylene sorbitol and sorbitan esters, microcrystalline cellulose, aluminium metahydroxide, bentonite, agar-agar, and tragacanth and mixtures thereof.

The product of the disclosure may be presented as solids in finely divided solid form, for example they may be micronised. Powders or finely divided solids may be encapsulated.

The active compound may be given as a single dose, in multiple doses or as a sustained release formulation.

It will be understood from the aforegoing that there are provided pharmaceutical products comprising a metal salt, e.g. an alkali metal salt, of a boronic acid of Formula (I) in dry fine particle form, suitable for administration. The metal salt may be in a form appropriate for oral administration. The metal salt is suitably an acid salt.

### Synthesis

### 1. Peptide/Peptidomimetic Synthesis

The synthesis of boropeptides, including, for example, Cbz-D-Phe-Pro-BoroMpg-OPinacol is familiar to those skilled in the art and described in the prior art mentioned above, including Claeson et al (US 5574014 and others) and Kakkar et al (WO 92/07869 and family members induding US 5648338). It is described also by Elgendy et al *Adv, Exp, Med. Biol. (USA)* 340:173-178,1993; Claeson,G. et al *Biochem.J*. 290:309-312, 1993; Deadman et al *J. Enzyme Inhibition* 9:29-41, 1995, and by Deadman et al *J. Med. Chem*. 38:1511-1522, 1995.

Stereoselective synthesis with S or R configuration at the chiral B-terminal carbon may be conducted using established methodology (Elgendy et al *Tetrahedron. Lett*. 33:4209-4212, 1992; WO 92/07869 and family members including US 5648338) using (+) or (―)- pinanediol as the chiral director (Matteson et al *J. Am. Chem. Soc*. 108:810-819, 1986; Matteson et al *Organometallics*, 3:1284-1288, 1984). Another approach is to resolve the requisite aminoboronate intermediate (e.g. Mpg-BOPinacol) to selectively obtain the desired (R)-isomer and couple it to the dipeptide moiety (e.g. Cbz-(R)-Phe-(S)-Pro, which is the same as Cbz-D-Phe-L-Pro) which will form the remainder of the molecule.

The boropeptides may be synthesised initially in the form of boronic acid esters, particularly esters with diols. Such diol esters may be converted to the peptide boronic acid as described next.

### 2. Ester to Acid Conversion

A peptide boronate ester such as Cbz-(R)Phe-Pro-BoroMpg-OPinacol may be hydrolysed to form the corresponding acid.

A novel technique for converting a diol ester of a peptide boronic acid of formula (I) into the acid comprises dissolving the diol ester in an ether and particularly a dialkyl ether, reacting the thus-dissolved diol with a diolamine, for example a dialkanolamine, to form a product precipitate, recovering the precipitate, dissolving it in a polar organic solvent and reacting the thus-dissolved product with an aqueous medium, e.g. an aqueous acid, to form the peptide boronic acid. The boronic acid may be recovered from the organic layer of the mixture resulting from the reaction, for example by removing the solvent, e.g. by evaporation under vacuum or distillation. The reaction between the diol ester and the diolamine may be carried out under reflux, for example.

The identity of the diol is not critical. As suitable diols may be mentioned aliphatic and aromatic compounds having hydroxy groups that are substituted on adjacent carbon atoms or on carbon atoms substituted by another carbon. That is to say, suitable diols include compounds having at least two hydroxy groups separated by at least two connecting carbon atoms in a chain or ring. One class of diols comprises hydrocarbons substituted by exactly two hydroxy groups. One such diol is pinacol and another is pinanediol; there may also be mentioned neopentylglycol, 1,2-ethanediol, 1,2-propanediol, 1,3-propanediol, 2,3-butanediol, 1,2-diisopropylethanediol, 5,6-decanediol and 1,2-dicyclohexylethanediol.

The alkyl groups of the dialkyl ether preferably have 1, 2, 3 or 4 carbon atoms and the alkyl groups may be the same or different. An exemplary ether is diethyl ether.

The alkyl groups of the dialkanolamine preferably have 1, 2, 3 or 4 carbon atoms and the alkyl groups may be the same or different. An exemplary dialkanolamine is diethanolamine. The diethanolamine/boronic acid reaction product hydrolyses in water at room temperature and the rate of hydrolysis may be accelerated by adding acid or base.

The polar organic solvent is preferably CHCl₃. Other examples are polyhalogenated alkanes generally and ethyl acetate. In principle, any polar organic solvent is acceptable other than alcohols.

The aqueous acid is suitably a strong inorganic acid at a pH in the region of 1 such as hydrochloric acid, for example.

After reaction with the acid, the reaction mixture is suitably washed with, for example, NH₄Cl or another mild base.

An example of a specific procedure is as follows
1. The pinacol or pinanediol ester of the selected peptide boronic acid is dissolved in diethylether.
2. Diethanolamine is added and the mixture is refluxed at 40 °C.
3. The precipitated product is removed (filtered), washed (usually several times) with diethyl ether or another polar organic solvent other than an alcohol, and dried (e.g. by evaporation under vacuum).
4. The dry product is dissolved in a polar organic solvent other than an alcohol, e.g. CHCl₃. Aqueous acid or base is added ,e.g hydrochloric acid (pH 1), and the mixture is stirred for e.g. approximately 1h at room temperature.
5. The organic layer is removed and washed with NH₄Cl solution.
6. The organic solvent is distilled off and the residual solid product is dried.

It will be appreciated that the aforegoing technique comprises an example of a method for recovering an organoboronic acid product, the method comprising providing in a solvent a dissolved mixture comprising the organoboronic acid in a soluble form and a compound having two hydroxy groups and an amino group (i.e. a diolamine), causing or allowing the organoboronic acid and the diolamine to react to form a precipitate, and recovering the precipitate. The soluble form of the organoboronic acid may be a diol ester, as discussed above. The solvent may be an ether, as discussed above. The organoboronic acid may be one of the organoboronic acids referred to in this specification, for example it may be of Formula (I), (II) or (III). A recovery method is filtration.

The reaction between the diolamine and the soluble form of the organoboronic acid is suitable carried out at an elevated temperature, for example under reflux.

One aspect provided by the invention relates to the use of the method of the preceding paragraph in the manufacture of a salt of the invention. Specifically this aspect resides in:
dissolving diethanolamine in an ether solution of a diol ester of an acid of formula (I);
allowing or causing a precipitate to form and recovering the precipitate;
converting the precipitated material into the free boronic acid; and
converting the acid to a base addition salt as described below.

The diethanolamine precipitation technique described above is an example of another novel method, which is a method for recovering from ether solution a pinacol or pinanediol ester of a peptide boronic acid of Formula I, comprising dissolving diethanolamine in the solution, allowing or causing a precipitate to form and recovering the precipitate. The disclosure encompasses variants of this methods in which another diol than pinacol or pinanediol is used.

The precipitated material, i.e. the "adduct", may be converted into the free organoboronic acid, for example by contacting it with an acid. The acid may be an aqueous acid, for example an aqueous inorganic acid, e.g. as described above. The precipitate may be dissolved, for example in an organic solvent, prior to being contacted with the acid.

### 3. Salt Synthesis

In general, the salts may be prepared by contacting the relevant peptide boronic acid with a strong base appropriate to form the desired salt. In the case of metal salts, the metal hydroxides are suitable bases (alternatively, metal carbonates might be used, for example), whilst sometimes it is more convenient to contact the acid with a relevant metal alkoxide (eg methoxide), for which purpose the corresponding alkanol is a suitable solvent. Salts with organic bases may be prepared by contacting the peptide boronic acid with the organic base itself. Illustrative salts are acid salts (one -BOH proton replaced) and, to make acid salts with a monovalent cation, the acid and the base are suitably reacted in substantially equimolar quantities. Generally stated, therefore, the usual acid:base molar ratio is substantially n:1, where n is the valency of the cation of the base.

In one procedure, a solution of the peptide boronic acid in a water-miscible organic solvent, for example acetonitrile or an alcohol (e.g. ethanol, methanol, a propanol, for example iso-propanol, or another alkanol), is combined with an aqueous solution of the base. The acid and the base are allowed to react and the salt is recovered. The reaction is typically carried out at ambient temperature (e.g. at a temperature of from 15 to 30°C, e.g. 15 to 25°C), but an elevated temperature may be used, for example up to the boiling point of the reaction mixture but more usually lower, e.g. a temperature of up to 40°C or 50°C. The reaction mixture may be allowed to stand or be agitated (usually stirred).

The time during which the acid and the base are allowed to react is not critical but it has been found desirable to maintain the reaction mixture for at least one hour. A period of from one to two hours is usually suitable but longer reaction times may be employed.

The salt may be recovered from the reaction mixture by any suitable method, for example evaporation or precipitation. Precipitation may be carried out by adding an excess of a miscible solvent in which the salt has limited solubility. In one preferred technique, the salt is recovered by evacuating the reaction mixture to dryness. The salt is preferably thereafter purified, for example by redissolving the salt before filtering the resulting solution and drying it, for example by evacuating it to dryness. The redissolution may be performed using water, e.g. distilled water. The salt may then be further purified, for example in order to remove residual water by further redissolution in a suitable solvent, which is advantageously ethyl acetate or THF followed by evaporating to dryness.

The purification procedure may be carried out at ambient temperature (say, 15 to 30°C, e.g. 15 to 25°C), or at a modestly elevated temperature, such as e.g. a temperature not exceeding 40°C or 50°C; for example the salt may be dissolved in water and/or solvent by agitating with or without warming to, for example, 37°C.

Generally, preferred solvents for use in purifying the salts are ethyl acetate or THF, or perhaps another organic solvent.

A general procedure for synthesising salts of Cbz-Phe-Pro-BoroMpg-OH is as follows:

Cbz-Phe-Pro-BoroMpg-OH (20.00g, 38.1mM) is dissolved in acetonitrile (200ml) with stirring at room temperature. To this solution is added the requisite base in solution in distilled water (190ml); the base is added as a 0.2M solution for a monovalent cation. The resultant dear solution is allowed to react for example by being left to stand or being agitated, for a usual period, In either case, of from one to two hours. The reaction is typically carried out at ambient temperature (e.g. 15-30°C, e.g. 15 to 25°C) but alternatively the temperature may be elevated (e.g. up to 30°C, 40°C or 50°C). The reaction mixture is then evacuated to dryness under vacuum with its temperature not exceeding 37°C, typically to yield a white brittle solid or an oil/tacky liquid. The oil/tacky liquid is redissolved in the minimum amount of distilled water necessary (200ml to 4L), typically with warming (e.g. to 30-40°C), usually for up to 2 hours. The solution is filtered, suitably through filter paper, and evacuated to dryness, again with the temperature of the solution not exceeding 37°C, or freeze dried. The resultant product is dried under vacuum overnight to normally yield a white brittle solid. If the product is present as an oil or tacky solid then it is dissolved in ethyl acetate and evacuated to dryness to produce the product as a white solid. The white solid is typically a coarse, amorphous powder.

In variations of the aforegoing general procedure, the acetonitrile is replaced by another water-miscible organic solvent, notably an alcohol, as discussed above, especially ethanol, methanol, iso-propanol or another propanol.

Where a boronic acid salt is less soluble in a selected reaction medium for salt formation such that its direct preparation from the corresponding acid and base is inconvenient, the less soluble salt may be prepared from a salt more soluble in the reaction medium.

### Separation of Stereoisomers

The stereoisomers of a peptide boronic ester or a synthetic intermediate aminoboronate may be resolved in, for example, any known way. In particular, stereoisomers of boronic esters may be resolved by HPLC.

### EXAMPLES

### EXAMPLES 1 TO 3 AND 33 - INTRODUCTORY REMARKS

### Apparatus

Throughout the following procedures of Examples 1 to 3 and 33, standard laboratory glassware and, where appropriate, specialised apparatus for handling and transferring of air sensitive reagents are used.

All glassware is heated at 140-160°C for at least 4 hours before use and then cooled either in a desiccator or by assembling hot and purging with a stream of dry nitrogen.

### Solvents

The organic solvents used in the procedures of Examples 1 to 3 and 33 are all dry. Suitably, they are dried over sodium wire before use.

### Dryness

In the drying procedures of Example 1 to 3 and 33, products are tested for dryness (including dryness in terms of organic solvent) by observing weight loss on drying. The following procedure was followed to determine loss on drying: a sample was placed in a vacuum drier and dried at 40°C at 100 mbar for 2 hours. Products are considered dry when the decrease in weight upon drying is less than 0.5% of the total weight of the starting material.

Examples 1 to 3 describe performance of the following reaction scheme and conversion of the resultant TRI 50c to the sodium salt thereof:

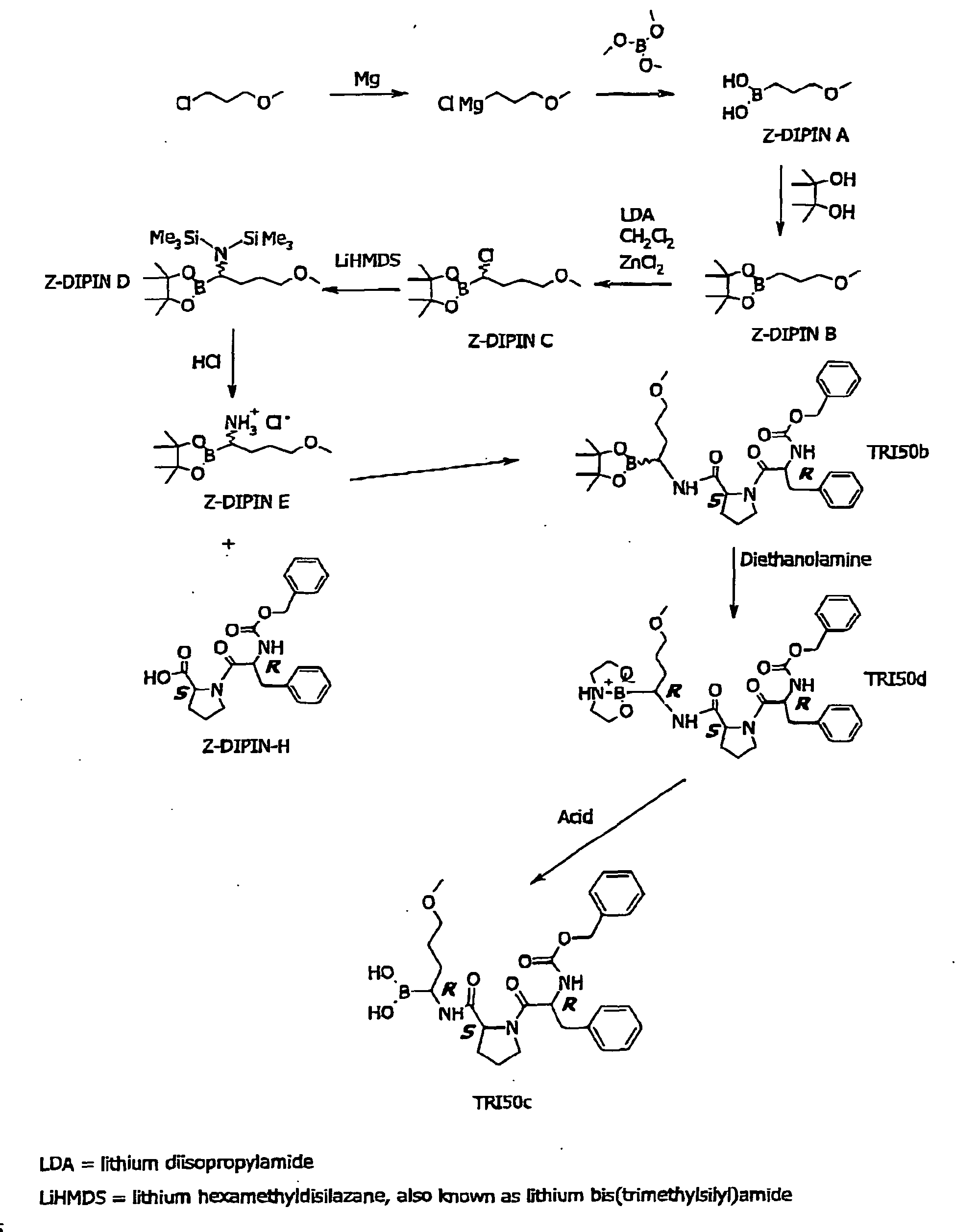

### EXAMPLE 1 - SYNTHESIS OF TRI 50D

### Step 1: Z-DIPIN B

### Procedure A

17.8 g (732.5 mmole) magnesium turnings, 0.1 g (0.4 mmole) iodine and 127 ml dry tetrahydrofuran are charged and heated to reflux. Then 15 ml of a solution of 66 g (608 mmole) 1-chloro-3-methoxypropane in 185 ml dry tetrahydrofuran are added and stirred under reflux until the vigorous reaction starts. After the initial exotherm ceases, the solution of 1-chloro-3-methoxypropane is added slowly to maintain gentle reflux until all the magnesium is consumed. After the reaction is finished, the reaction mixture is cooled to ambient temperature and slowly added to a solution of 64.4 g (620 mmole) trimethylborate in 95 ml dry tetrahydrofuran; the latter solution is cooled to below 0°C and, if it warms up during the course of the reaction, the reaction mixture must be added to it sufficiently slowly to maintain the temperature of this solution below 65°C. Upon complete addition, the reaction mixture is allowed to warm to about 0°C and stirred for another 60 minutes. Then a solution of 22.4 ml sulfuric acid in 400 ml water is added slowly so as to maintain the temperature below 20°C. The layers are allowed to settle and the phases are separated. The aqueous layer is rewashed three times with 200 ml tert.-butylmethylether. The combined organic layers are allowed to settle and additional water separated from this solution is removed. The organic layer is dried over magnesium sulfate, filtered and evaporated to dryness. The evaporation residue is filtered from the precipitated solid and the filtrate dissolved in 175 ml toluene. 34.8 g (292 mmole) pinacol is charged to the solution followed by stirring at ambient temperature for not less than 10 hours. The solution is evaporated to dryness, dissolved in 475 ml n-heptane and washed three times with 290 ml saturated aqueous solution of sodium hydrogen carbonate. The n-heptane solution is evaporated to dryness and the evaporation residue distilled and the fraction with Bp 40-50°C at 0.1-0.5 mbar recovered.
Boiling point: 40-50°C / 0.1-0.5 mbar
Yield: 40.9 g (70%) Z-DIPIN B (oil)

### Procedure B

17.8 g (732.5 mmole) magnesium turnings, 0.1 g (0.4 mmole) iodine and 127 ml dry tetrahydrofuran are charged and heated to reflux. Then 15 ml of a solution of 66 g (608 mmole) 1-chloro-3-methoxypropane in 185 ml dry tetrahydrofuran are added and stirred under reflux until the vigorous reaction starts. After the initial exotherm ceases, the solution of 1-chloro-3-methoxypropane is added slowly to maintain gentle reflux. After the reaction is finished, the reaction mixture is cooled to ambient temperature and slowly added to a solution of 64.4 g (620 mmole) trimethylborate in 95 ml dry tetrahydrofuran, maintaining the temperature of this solution below minus 65°C. Upon complete addition, the reaction mixture is allowed to warm to about 0°C and stirred for another 60 minutes. Then a solution of 22.4 ml sulfuric acid in 400 ml water is added slowly so as to maintain the temperature below 20°C. The organic solvent is removed by distillation under vacuum. 300 ml n-heptane is charged to the aqueous solution of the evaporation residue followed by addition of 34.8 g (292 mmole) pinacol. The two-phase-mixture is stirred at ambient temperature for not less than 2 hours. After allowing the layers to settle, the aqueous phase is separated. 300 ml n-heptane is charged to the aqueous solution and the two-phase-mixture is stirred at ambient temperature for not less than 2 hours. After allowing the layers to settle, the aqueous phase is separated. The organic layers are combined and washed once with 200 ml water, followed by 200 ml saturated sodium hydrogen carbonate solution and two further washes with 200 ml water each. The n-heptane solution is evaporated to dryness and the evaporation residue distilled and the fraction with Bp 40-50°C at 0.1-0.5 mbar recovered.
Boiling point: 40-50°C / 0.1-0.5 mbar
Yield: 40.9 g (70-85%) Z-DIPIN B (oil)

### Step 2: Z-DIPIN C

16.6 g (164 mmole) diisopropylamine and 220 ml tetrahydrofuran are charged and cooled to -30 to -40°C. To this solution 41.8 g (163 mmole) n-butyl lithium, 25% in n-heptane is added, followed by stirring at 0 to -5°C for one hour. This freshly prepared solution of lithium diisopropylamide is cooled to -30°C and then added to a solution of 27.9 g (139 mmole) Z-DIPIN B in 120 ml tetrahydrofuran and 35.5 g (418 mmole) dichloromethane at a temperature between -60 and -75°C. The solution is stirred at that temperature for half an hour followed by addition of 480 ml (240 mmole) 0.5N anhydrous Zinc(II)-chloride in tetrahydrofuran or 32.5 g (240 mmole) anhydrous solid Zinc(II)-chloride. After stirring at -65°C for one hour, the reaction mixture is allowed to warm to ambient temperature and stirred for another 16-18 hours. The reaction mixture is evaporated to dryness (i.e. until solvent is removed) and followed by addition of 385 ml n-heptane. The reaction mixture is washed with 150 ml 5% sulfuric acid, with 190 ml saturated sodium hydrogen carbonate solution, and 180 ml saturated sodium chloride solution. The organic layer is dried over magnesium sulfate, filtered and evaporated to dryness (i.e. until solvent is removed). The oily residue is transferred into the next step without further purification.
Yield: 19 g (55%) Z-DIPIN C

### Step 3: Z-DIPIN D

To a solution of 23.8 g (148 mmole) hexamethyldisilazane in 400 ml tetrahydrofuran at -15°C is added 34.7 g (136 mmole) n-butyl lithium, 25% in n-heptane and stirred for one hour. The solution is cooled to -55°C followed by the addition of 30.6 g (123 mmole) Z-DIPIN C dissolved in 290 ml tetrahydrofuran and 35 ml tetrahydrofuran to this freshly prepared solution of LiHMDS. The solution is allowed to warm to ambient temperature and stirred for 12 hours. The reaction mixture is evaporated to dryness, the evaporation residue dissolved in 174 ml n-heptane, washed with 170 ml water and 75 ml saturated sodium chloride solution. The organic phase is dried over magnesium sulfate, filtered and evaporated to complete dryness (i.e. until solvent is removed). The oily residue is dissolved in 100 g n-heptane. This solution is carried over into the next step without further purification.
Yield: 32.2 g (70%) Z-DIPIN D

### Step 4: Z-DIPIN (TRI50b, crude)

A solution of 26.6 g (71 mmole) Z-DIPIN D in 82.6 g n-heptane is diluted with 60 ml n-heptane and cooled to -60°C followed by introduction of 10.5 g (285 mmole) hydrogen chloride. The reaction mixture is subsequently evacuated and flushed with nitrogen, while the temperature is increased in increments of about 20°C to ambient temperature. The solvent is removed from the oily precipitate and replaced several times by 60 ml fresh n-heptane. The oily residue is dissolved in 60 ml tetrahydrofuran (Solution A).

To a different flask 130 ml tetrahydrofuran, 24.5 g (61.5 mmole) Z-D-Phe-Pro-OH and 6.22 g (61.5 mmole) N-methylmorpholine are charged and cooled to -20°C. To this solution a solution of 8.4 g (61.5 mmole) isobutylchloroformate in 20 ml tetrahydrofuran is added and stirred for 30 minutes, followed by addition of Solution A at 25°C. Upon complete addition, up to 16 ml (115 mmole) triethylamine is added to adjust the pH to 9-10, measured using a pH stick. The reaction mixture is allowed to warm to ambient temperature and stirred for 3 hours, still under nitrogen. The solvent is evaporated to dryness and the evaporation residue dissolved in 340 ml tert.-butylmethylether (t-BME). The solution of Z-DIPIN in t-BME is washed twice with 175 ml 1.5% hydrochloric acid. The combined acidic washes are given a rewash with 175 ml t-BME. The combined organic layers are washed with 175 ml water, with 175 ml saturated sodium hydrogen carbonate solution, with 175 ml 25% sodium chloride solution, dried over magnesium sulfate and filtered. This solution is carried over into the next step without further purification.
Yield: 29.9 g (80%) Z-DIPIN

### EXAMPLE 2 - SYNTHESIS OF TRI 50D (DIETHANOLAMINE ADDUCT OF TRI 50C)

The starting material used in this Example is the solution of TRI 50b ("Z-DIPIN") obtained in Example 1. The solution is carried forward to the synthesis of TRI 50d without further purification. The solution of Z-DIPIN in t-BME (containing 7.0 g (11.5 mmole) (R,S,R) TRI50b, calculated based on HPLC results of Z-DIPIN) is evaporated to dryness and the evaporation residue dissolved in 80 ml diethylether. 1.51 g (14.4 mmole) diethanolamine is added and the mixture heated at reflux for at least 10 hours, during which process the product precipitates. The suspension is cooled to 5-10°C, filtered and the filter residue washed with diethylether.

To improve chiral and chemical purity the wet filter cake (7 g) is dissolved in 7 ml dichloromethane, cooled to 0-5°C and the product precipitated by addition of 42 ml diethylether and filtered. The isolated wet product is dried at 35°C in vacuum or at least 4 hours, until day.
Yield: 5.5 g (80%) Tri50d
Melting Point: 140-145°C

### EXAMPLE 3 - PREPARATION OF SODIUM SALT OF TRI50C

1.5 kg (2.5 mole) TRI50d Example 2 is dissolved in 10.5 L dichloromethane. 11 L 2% hydrochloric acid is added and the mixture is stirred for at most 30 minutes (optimally about 20 minutes) at room temperature. A precipitate forms in the organic phase. After stirring, the layers are allowed to settle and separated. The aqueous layer is rewashed twice with 2.2 L dichloromethane. The combined organic layers are washed with a solution of 625 g ammonium chloride in 2.25 L water. (The ammonium chloride buffers the pH of the aqueous extractions to be within a range of from about pH 1-2 to about pH 4-5, as strongly acidic conditions might cleave peptide bonds). The organic phase is dried over magnesium sulfate, filtered and the filtrate evaporated to dryness. An assay of the free boronic acid is performed (by the RP HPLC method of Example 28 for at most 30 mins (optionally about 20 min) at room temperature) and the amounts of the solvents and base for conversion of the acid to the salt are calculated. If 2.5 mol of the free acid is obtained, the evaporation residue is dissolved in 5 L acetonitrile followed by addition of a solution of 100 g (2.5 mole) sodium hydroxide as a 5% solution in 2.2 L water. The solution is stirred for two hours at ambient temperature (e.g. 15-30°C, optimally room temperature) and then evaporated in vacuum (of ca. 10 mmHg) at a temperature not exceeding 35°C. The evaporation residue is repeatedly dissolved in 3.5 L fresh acetonitrile and evaporated to dryness to remove traces of water. If the evaporation residue is dry, it is dissolved in 3 L acetonitrile (or alternatively in 6 L THF) and slowly added to a mixture of 32 L n-heptane and 32 L diethylether. The addition is performed slowly enough to avoid lumping or sticking of the product and is carried out over a period of not less than 30 minutes. The precipitated product is filtered off, washed with n-heptane and dried under vacuum at a temperature initially of about 10°C and then increasing to a limit of about 35°C, until dry.
Yield: 1.0 kg (70%) Tri50c sodium salt.

The procedures of Examples 1 to 3 may be scaled up and, if operated carefully, will produce highly pure salts. In the diethanolamine precipitation step it is important to use 1.25 equivalents of diethanolamine per equivalent of (R,S,R) TRI 50b. In the hydrolysis of the diethanolamine ester, it is important to avoid excessively long contact with the aqueous acid. Likewise the TRI 50b should be synthesised via the Grignard reaction to Z-DIPIN A.

### EXAMPLE 4 - ALTERNATIVE CONVERSION OF TRI 50B TO TRI 50C

The synthetic procedures described in this and subsequent synthetic examples were generally performed under nitrogen and using dry solvents as supplied from commercial sources.
1. Approximately 300 g of TRI 50b, obtained by the HPLC purification of racemic TRI 50b, were dissolved in approximately 2.5 L diethylether. It is estimated that different batches of TRI 50b had isomeric purities ranging from 85% R,S,R to in excess of 95% R,S,R.
2. Approximately 54 ml diethanolamine were added (1:1 stoichiometry with total TRI 50b content), and the mixture was refluxed at 40 °C.
3. The precipitated product was removed, washed several times with diethylether and dried.
4. The dry product was dissolved in CHCl₃. Hydrochloric acid (pH 1) was added and the mixture was stirred approximately 1h at room temperature.
5. The organic layer was removed and washed with NH₄Cl solution.
6. The organic solvent was distilled off and the residual solid product was dried.
Typical yield: Approximately 230 g

### EXAMPLE 5 - PREPARATION OF LITHIUM SALT OF TRI50C

Cbz-Phe-Pro-BoroMpg-OH obtained by the method of Example 4 (20.00g, 38.1mM) is dissolved in acetonitrile (200ml) with stirring at room temperature. To this solution is added LiOH as a 0.2M solution in distilled water (190ml). The resultant dear solution is stirred for 2 hours at room temperature and then evacuated to dryness under vacuum with its temperature not exceeding 37°C. The resultant oil/tacky liquid is redissolved in 500ml distilled water necessary with light warming for about 20 minutes. The solution is filtered through filter paper and evacuated to dryness, again with the temperature of the solution not exceeding 37°C. The resultant product is dried under vacuum overnight to normally yield a white brittle solid.

The salt was then dried under vacuum over silica to constant weight (72 h).

Yield 17.89g.

**Microanalysis:**

| C % Found | H % Found | N % Found | B % Found | Metal % Found |
|---|---|---|---|---|
| (Calc.) | (Calc.) | (Calc.) | (Calc.) | (Calc.) |
| 57.14 | 6.60 | 7.34 | 2.07 | Li 1.26 |
| (61.03) | (6.64) | (7.90) | (2.03) | (1.31) |

### EXAMPLE 6 - UV/VISIBLE SPECTRA OF LITHIUM SALT OF TRI50C

UV/Visible spectra of the salt resulting from the procedure of Example 5 were recorded in distilled water at 20°C from 190nm to 400nm. The salt gave λₘₐₓ at 210 and 258nm. The weight of the dried salt was then measured for the purposes of calculating the extinction coefficient. The λₘₐₓ at 258nm was used. The extinction coefficient was calculated using the formula:-
A = εcl where A is the absorbance
   C is the concentration
   l the path length of the UV cell
   and ε is the extinction coefficient.
Extinction coefficient: 451

### EXAMPLE 7 - AQUEOUS SOLUBILITY OF LITHIUM SALT OF TRI50C

The salt used in this Example was made using a modification of the process described in Example 5. The modified process differs from that described in that 100mg of TRI 50c was used as starting material, the product of the redissolution in water was dried by freeze drying and the filtration was carried out through a 0.2µm filter. The salt is believed to contain about 85% of R,S,R isomer.

To determine maximum aqueous solubility 25mg of the dried salt were shaken in water at 37°C, the sample filtered and the UV spectrum measured. The salt left a white residue of undissolved material. The lithium salt was comparatively soluble and so was redissolved at 50mg/ml in the same manner previously described.

Solubility when dissolved at 25mg/ml: 43mM (23 mg/ml).
Solubility when dissolved at 50mg/ml: 81mM (43 mg/ml).

### EXAMPLE 8 - PREPARATION OF SODIUM SALT OF TRI50C (TGN 255)

Cbz-Phe-Pro-BoroMpg-OH obtained by the method of Example 4 (20.00g, 38.1mM) is dissolved in acetonitrile (200ml) with stirring at room temperature. To this solution is added NaOH as a 0.2M solution in distilled water (190ml). The resultant dear solution is stirred for 2 hours at room temperature and then evacuated to dryness under vacuum with its temperature not exceeding 37°C. The resultant oil/tacky liquid is redissolved in 500ml distilled water with light warming for about 15-20 minutes. The solution is filtered through filter paper and evacuated to dryness, again with the temperature of the solution not exceeding 37°C. The resultant product is dried under vacuum overnight to normally yield a white brittle solid. The product may be present as an oil or tacky solid due to residual water, in which case it is dissolved in ethyl acetate and evacuated to dryness to produce the product as a white solid.

The salt was then dried under vacuum over silica to constant weight (72 h).

Yield: Over 50%.

**Microanalysis:**

| C % Found | H % Found | N % Found | B % Found | Metal % Found |
|---|---|---|---|---|
| (Calc.) | (calc.) | (Calc.) | (Calc.) | (Calc.) |
| 59.93 | 6.47 | 7.31 | 1.91 | Na 3.81 |
| (59.24) | (6.44) | (7.67) | (1.98) | (4.20) |

### EXAMPLE 9 - UV/VISIBLE SPECTRA OF SODIUM SALT OF TRI50C

UV/Visible spectra of the sodium salt resulting from the procedure of Example 8 were recorded in distilled water at 20°C from 190nm to 400nm. The salt gave λₘₐₓ at 210 and 258nm. The weight of the dried salt was then measured for the purposes of calculating the extinction coefficient. The λₘₐₓ at 258nm was used. The extinction coefficient was calculated using the formula:-
A = εcl where A is the absorbance
   C is the concentration
   l the path length of the UV cell
   and ε is the extinction coefficient.
Extinction coefficient: 415.

### EXAMPLE 10 - AQUEOUS SOLUBILITY OF SODIUM SALT OF TRISOC

The salt used in this Example was made using a modification of the process described in Example 8. The modified process differs from that described in that 100mg of TRI 50c was used as starting material, the product of the redissolution in water was dried by freeze drying and the filtration was carried out through a 0.2µm filter. The salt is believed to contain about 85% of R,S,R isomer.

To determine maximum aqueous solubility 25mg of the dried salt were shaken in water at 37°C, the sample filtered and the UV spectrum measured. The salt left a white residue of undissolved material. The sodium salt was comparatively soluble and so was redissolved at 50mg/ml in the same manner previously described.

Solubility when dissolved at 25mg/ml: 44mM (25 mg/ml).
Solubility when dissolved at 50mg/ml: 90mM (50 mg/ml).

### EXAMPLE 11 - PREPARATION OF POTASSIUM SALT OF TRI50C

Cbz-Phe-Pro-BoroMpg-OH obtained by the method of Example 4 (20.00g, 38.1mM) is dissolved in acetonitrile (200ml) with stirring at room temperature. To this solution is added KOH as a 0.2M solution in distilled water (190ml). The resultant clear solution is stirred for 2 hours at room temperature and then evacuated to dryness under vacuum with its temperature not exceeding 37°C. The resultant oil/tacky liquid is redissolved in 1L distilled water with warming to 37°C for about 2 hours. The solution is filtered through filter paper and evacuated to dryness, again with the temperature of the solution not exceeding 37°C. The resultant product is dried under vacuum overnight to normally yield a white brittle solid.
Yield: 14.45 mg.

The salt was then dried under vacuum over silica to constant weight (72 h).

**Microanalysis:**

| C % Found | H % Found | N % Found | B % Found | Metal % Found |
|---|---|---|---|---|
| (Calc.) | (Calc.) | (Calc.) | (Calc.) | (Calc.) |
| 54.84 | 6.25 | 7.02 | 2.01 | K 4.29 |
| (57.55) | (6.26) | (7.45) | (1.92) | (6.94) |

### EXAMPLE 12 - UVAQSIBLE ZGCTRA OF POTASSIUM SALT OF TRI50C

UV/Visible spectra of the potassium salt resulting from the procedure of Example 11 were recorded in distilled water at 20°C from 190nm to 400nm. TRI50C and the salt gave λₘₐₓ at 210 and 258nm. The weight of the dried salt was then measured for the purposes of calculating the extinction coefficient. The λₘₐₓ at 258nm was used. The extinction coefficient was calculated using the formula:-
A = εcl where A is the absorbance
   C is the concentration
   l the path length of the UV cell
   and ε is the extinction coefficient.
Extinction coefficient: 438.

### EXAMPLE 13 -AQUEOUS SOLUBILITY OF POTASSIUM SALT OF TRI50C

The salt used in this Example was made using a modification of the process described in Example 11. The modified process differs from that described in that 100mg of TRI 50c was used as starting material, the product of the redissolution in water was dried by freeze drying and the filtration was carried out through a 0.2µm filter. The salt is believed to contain about 85% of R,S,R isomer.

To determine maximum aqueous solubility 25mg of the dried salt were shaken in water at 37°C, the sample filtered and the UV spectrum measured. The salt left a white residue of undissolved material.

Solubility when dissolved at 25mg/ml: 29mM (16 mg/ml).

### EXAMPLE 14 - PREPARATION OF ZINC SALT OF TRI 50C

The relative solubility of zinc hydroxide is such that, if the hydroxide had been used to prepare the corresponding TRI 50c salt using the procedure of Example 5, they would not have resulted in homogeneous salt formation. A new method was therefore developed to prepare the zinc salt, as described in this and the next examples.

TRI 50c sodium salt (2.24g, 4.10mM) was dissolved in distilled water (100ml) at room temperature and zinc chloride in THF (4.27ml, 0.5M) was carefully added with stirring. A white precipitate that immediately formed was filtered off and washed with distilled water. This solid was dissolved in ethyl acetate and washed with distilled water (2 x 50ml). The organic solution was evacuated to dryness and the white solid produced dried over silica in a desiccator for 3 days before microanalysis. Yield 1.20g.

¹H NMR 400MHz, δ_{H}(CD₃OD) 7.23-7.33 (20H, m, ArH), 5.14 (4H, m, PhCH₂O), 4.52 (4H, m, αCH), 3.65 (2H, m), 3.31 (12H, m), 3.23 (6H, s, OCH₃), 2.96 (4H, d, *J* 7.8Hz), 2.78 (2H, m), 2.58 (2H, m), 1.86 (6H, m), 1.40 (10H, m).

¹³C NMR 75MHz δ_{C}(CD₃OD) 178.50, 159.00, 138.05, 137.66, 130.54, 129.62, 129.50, 129.07, 128.79, 128.22, 73.90, 67.90, 58.64, 58.18, 56.02, 38.81, 30.06, 28.57, 28.36, 25.29.
FTIR (KBr disc) νₘₐₓ (cm⁻¹) 3291.1, 3062.7, 3031.1, 2932.9, 2875.7, 2346.0, 1956.2, 1711.8, 1647.6, 1536.0, 1498.2, 1452.1, 1392.4, 1343.1, 1253.8, 1116.8, 1084.3, 1027.7, 916.0, 887.6, 748.6, 699.4, 595.5, 506.5.

### EXAMPLE 15 - PREPARATION OF ARGININE SALT OF TRI50C

Cbz-Phe-Pro-BoroMpg-OH obtained by the method of Example 4 (20.00g, 38.1mM) is dissolved in acetonitrile (200ml) with stirring at room temperature. To this solution is added arginine as a 0.2M solution in distilled water (190ml). The resultant clear solution is stirred for 2 hours at room temperature and then evacuated to dryness under vacuum with its temperature not exceeding 37°C. The resultant oil/tacky liquid is redissolved in 2L distilled water with warming to 37°C for 2 hours. The solution is filtered through filter paper and evacuated to dryness, again with the temperature of the solution not exceeding 37°C. The resultant product is dried under vacuum overnight to normally yield a white brittle solid.

The salt was then dried under vacuum over silica to constant weight (72 h).

Yield: 10.54g.

**Microanalysis;**

| C % Found | H % Found | N % Found | B % Found |
|---|---|---|---|
| (Calc.) | (Calc.) | (Calc.) | (Calc.) |
| 52.47 | 7.12 | 15.25 | 1.52 |
| (56.65) | (7.20) | (14.01) | (1.54) |

### EXAMPLE 16 - UV/VISIBLE SPECTRA OF ARGININE SALT OF TRI50C

UV/Visible spectra of the salt resulting from the procedure of Example 15 were recorded in distilled water at 20°C from 190nm to 400nm. TRI50C and the salt gave λₘₐₓ at 210 and 258nm. The weight of the dried salt was then measured for the purposes of calculating the extinction coefficient. The λₘₐₓ at 258nm was used. The extinction coefficient was calculated using the formula:-
A = εcl where A is the absorbance
   C is the concentration
   l the path length of the UV cell
   and ε is the extinction coefficient.
Extinction coefficient: 406.

### EXAMPLE 17 - AQUEOUS SOLUBILITY OF ARGININE SALT OF TRI50C

The salt used in this Example was made using a modification of the process described in Example 15. The modified process differs from that described in that 100mg of TRI 50c was used as starting material, the product of the redissolution in water was dried by freeze drying and the filtration was carried out through a 0.2µm filter. The salt is believed to contain about 85% of R,S,R isomer.

To determine maximum aqueous solubility 25mg of the dried salt were shaken in water at 37°C, the sample filtered and the UV spectrum measured. The salt left a white residue of undissolved material.

Solubility when dissolved at 25mg/ml: 14mM (10 mg/ml).

### EXAMPLE 18 - PREPARATION OF LYSINE SALT OF TRI50C

Cbz-Phe-Pro-BoroMpg-OH obtained by the method of Example 4 (20.00g, 38.1mM) is dissolved in acetonitrile (200ml) with stirring at room temperature. To this solution is added L-lysine as a 0.2M solution in distilled water (190ml). The resultant clear solution is stirred for 2 hours at room temperature and then evacuated to dryness under vacuum with its temperature not exceeding 37°C. The resultant oil/tacky liquid is redissolved in 3L distilled water with warming to 37°C for 2 hours. The solution is filtered through filter paper and evacuated to dryness, again with the temperature of the solution not exceeding 37°C. The resultant product is dried under vacuum overnight to normally yield a white brittle solid. The product may be present as an oil or tacky solid (due to residual water), in which case it is then dissolved in ethyl acetate and evacuated to dryness to produce the product as a white solid.

The salt was then dried under vacuum over silica to constant weight (72 h).

Yield: 17.89.

**Microanalysis:**

| C % Found | H % Found | N % Found | B % Found |
|---|---|---|---|
| (Calc.) | (Calc.) | (Calc.) | (Calc.) |
| 57.03 | 7.43 | 10.50 | 1.72 |
| (59.11) | (7.36) | (10.44) | (1.61) |

### EXAMPLE 19 - UV/VISIBLE SPECTRA OF LYSINE SALT OF TRI50C

UV/Visible spectra of the salt resulting from the procedure of Example 18 were recorded in distilled water at 20°C from 190nm to 400nm. TRI50C and the salt gave λₘₐₓ at 210 and 258nm. The weight of the dried salt was then measured for the purposes of calculating the extinction coefficient. The λₘₐₓ at 258nm was used. The extinction coefficient was calculated using the formula:-
A = εcl where A is the absorbance
   C is the concentration
   l the path length of the UV cell
   and ε is the extinction coefficient.
Extinction coefficient: 437.

### EXAMPLE 20 - AQUEOUS SOLUBILITY OF LYSINE SALT OF TRI50C

The salt used in this Example was made using a modification of the process described in Example 18. The modified process differs from that described in that 100mg of TRI 50c was used as starting material, the product of the redissolution in water was dried by freeze drying and the filtration was carried out through a 0.2µm filter. The salt is believed to contain about 85% of R,S,R isomer.

To determine maximum aqueous solubility 25mg of the dried salt were shaken in water at 37°C, the sample filtered and the UV spectrum measured. The salt left a white residue of undissolved material.

Solubility when dissolved at 25mg/ml: 13mM (8.6 mg/ml).

### EXAMPLE 21 - PREPARATION OF N-METHYL-D-GLUCAMINE SALT OF TRI50C

Cbz-Phe-Pro-BoroMpg-OH obtained by the method of Example 4 (20.00g, 38.1mM) is dissolved in acetonitrile (200ml) with stirring at room temperature. To this solution is added N-methyl-D-glucamine as a 0.2M solution in distilled water (190ml). The resultant dear solution is stirred for 2 hours at room temperature and then evacuated to dryness under vacuum with its temperature not exceeding 37°C. The resultant oil/tacky liquid is redissolved in 500ml distilled water with light warming for about 20 minutes. The solution is filtered through filer paper and evacuated to dryness, again with the temperature of the solution not exceeding 37°C, or freeze dried. The resultant product is dried under vacuum overnight to normally yield a white brittle solid.

The salt was then dried under vacuum over silica to constant weight (72 h).

Yield: 21.31g.

**Microanalysis:**

| C % Found | H % Found | N % Found | B % Found |
|---|---|---|---|
| (Calc.) | (Calc.) | (Calc.) | (Calc.) |
| 56.67 | 7.28 | 7.74 | L63 |
| (56.67) | (7.41) | (7.77) | (1.50) |

### EXAMPLE 22 - UV/VISIBLE SPECTRA OF N-METHYL-D-GLUCAMINE SALT OF TRI50C

UV/Visible spectra of the salt resulting from the procedure of Example 21 were recorded in distilled water at 20°C from 190nm to 400nm. TRI50C and the salt gave λₘₐₓ at 210 and 258nm. The weight of the dried salt was then measured for the purposes of calculating the extinction coefficient. The λₘₐₓ at 258nm was used. The extinction coefficient was calculated using the formula:-
A = εcl where A is the absorbance
   C is the concentration
   l the path length of the UV cell
   and ε is the extinction coefficient.
Extinction coefficient: 433.

### EXAMPLE 23 - AQUEOUS SOLUBILITY OF N-METHYL-D-GLUCAMINE SALT OF TRI50C

The salt used in this Example was made using a modification of the process described in Example 21. The modified process differs from that described in that 100mg of TRI 50c was used as starting material, the product of the redissolution in water was dried by freeze drying and the filtration was carried out through a 0.2µm filter. The salt is believed to contain about 85% of R,S,R isomer.

To determine maximum aqueous solubility 25mg of the dried salt were shaken in water at 37°C, the sample filtered and the UV spectrum measured. The salt was observed to fully dissolve. The salt was comparatively soluble and so was redissolved at 50mg/ml in the same manner previously described.

Solubility when dissolved at 25mg/ml: 35mM (25 mg/ml).
Solubility when dissolved at 50mg/ml: 70mM (50 mg/ml).

### EXAMPLE 24 - ALTERNATIVE PREPARATION OF ARGININE SALT OF TRI50C

The arginine salt is formed simply by adding a slight molar excess of L-arginine to a solution of 0.2-0.3mmol of TRI50c in 10ml of ethyl acetate. The solvent is evaporated after one hour, and the residue is triturated twice with hexane to remove excess arginine.

### EXAMPLE 25 - SOLUBILITY OF TRI50C

The UV/visible spectra of TRI 50c resulting from the procedure of Example 5 and its solubility were obtained as described above in relation to the salts. The solubility of TRI50c when dissolved at 50mg/ml was 8mM (4mg/ml).

¹³C NMR 75MHz δ_{C}(CD₃C(O)CD₃) 206.56, 138.30, 130.76, 129.64, 129.31, 129.19, 129.09, 128.20, 128.04, 74.23, 73.55, 67.78, 58.76, 56.37, 56.03, 48.38, 47.87, 39.00, 25.42, 25.29.
FTIR (KBr disc) νₘₐₓ (cm⁻¹) 3331.3, 3031.4, 2935.3, 2876.9, 2341.9, 1956.1, 1711.6, 1639.9, 1534.3, 1498.1, 1453.0, 1255.3, 1115.3, 1084.6, 1027.6, 917.3, 748.9, 699.6, 594.9, 504.5, 467.8.

### EXAMPLE 26 - ANALYSIS OF SODIUM AND ZINC SALTS OF(R,S,R) TRI50C

The following salts were prepared using a boronate:metal stoichiometry of n:1, where n is the valency of the metal, using (R,S,R) TRI 50c of higher chiral purity than that used to prepare the salt described in Example 10.

**A. Sodjum Salt**

| **Analytical data** | | | | **Physical Properties** |
|---|---|---|---|---|
| HPLC or LC/MS: HPLC betabasic C18 Column, | | | | Form; Amorphous solid |
| CH₃CN, Water | | | | |
| | | | | Colour: White |
| Estimated Purity: >95% by UV (λ₂₁₅ₙₘ) | | | | |
| | | | | Melting Point: N/A |

| Micro analysis: | | | | |
|---|---|---|---|---|
| | | *Calcd.* | *Found.* | Solubility: Soluble in aqueous media |
| C: | | 59.24 | 59.93 | *ca*~50mg/ml |
| H: | | 6.44 | 6.47 | |
| N: | | 7.67 | 7.31 | M_{w}: 547.40 |
| Other: | B: | 1.98 | 1.91 | |
| | Na: | 4.20 | 3.81 | |

**B. Zinc Salt**

| **Analytical data** | | | | **Physical Properties** |
|---|---|---|---|---|
| HPLC or LC/MS: HPLC betabasic C18 Column, CH₃CN, Water | | | | Form: Amorphous solid |
| | | | | Colour: White |
| Estimated Purity: >95% by UV (λ₂₁₅ₙₘ) | | | | |
| | | | | Melting Point: N/A |
| Micro analysis: | | | | |
| | | *Calcd.* | *Found.* | Solubility: Soluble in aqueous media |
| C: | | 58.21 | 56.20 | *ca*~2mg/ml |
| H: | | 6.33 | 6.33 | |
| N: | | 7.54 | 7.18 | M_{w}: 1114.18 |
| Other: | B: | 1.94 | 1.84 | |
| | Zn: | 5.87 | 7.26 | |

| | | | | |
|---|---|---|---|---|
| *Notes:* The trigonal formula of the acid boronate is used in the calculated microanalyses. It is believed that a lower sodium salt solubility is reported in example 11 because the salt tested in example 11 had lower chiral purity. | | | | |

### Conclusion

The sodium and zinc salts have all been prepared with a stoichiometry of, respectively, one metal ion to one molecule of TRI 50c and one metal ion to two molecules of TRI 50c. The value found for the sodium salt is dose to and thus consistent with that calculated for this 1:1 stoichiometry. For the zinc salt an excess of zinc was found; nonetheless, the zinc salt comprises a significant proportion of acid boronate.

### EXAMPLE 27 - STABILITY

An assay of TRI 50c and its sodium and lysine salts before and after drying.

### Method

TRI 50c and its Na, Ca and Lys salts were weighed into HPLC vials and stored in a desiccator over phosphorus pentoxide for 1 week. For sample analysis, 5 mg of dried and non-dried material was weighed in a 5 mL volumetric flask and dissolved in 1 mL acetonitrile and filled up with water to 5 mL.

The compounds were investigated by HPLC. For the impurity profiles, an HPLC peak area percentage was calculated. The results are shown in Table 1.

**Table 1**

| **Compound** | **Amount [µg/mL]** | **Purity (% area)** |
|---|---|---|
| TRI 50c dry | 1000.0 | 82.00 |
| TRI 50c non-dried | 947.3 | 85.54 |
| TRI 50c Na salt dry | 1024 | 98.81 |
| TRI 50c Na salt non-dried | 1005.8 | 98.61 |
| TRI 50c Lys salt dry | 813.3 | 90.17 |
| TRI 50c Lys salt non-dried | 809.8 | 92.25 |

The purity of the acid was lowered by the drying process but the purity of the salts was less affected; the purity of the sodium salt was not significantly reduced. Large differences in response factors will reduce the actual impurity levels, however.

This example indicates that the salts of the disclosure, particularly the metal salts, e.g. alkali metal salts, are more stable than the acids, notably TRI 50c.

### EXAMPLE 28 - STABILITY

This Example compares the stability of TRI 50c and TRI 50c lysine salt when filled into enteric-coated hard gelatin capsules.

### 1. Tabulated Results

| **Compound** | **Packing** | **Climatic conditions 1.5 month** ^{**0)**} | **Purity (HPLC %Area) T0** | **Purity (HPLC %Area)**^{**a**} **T1** |
|---|---|---|---|---|
| TRI50C | capsules in blister | 25°C/60% r.h.⁴ | 99 | 73.9 |
| TRI50c in | capsules blister | 40°C / 75% r.h. | 99 | 73.9 |
| TRI50c | capsules¹ | 40°C / 75% r.h | 99 | 75.3 |
| TRI50c Lysine Salt | capsules in blister | 25°C / 60% r.h. | 90.2²⁾ | 90.5 |
| TRI50c Lysine Salt | capsules in blister | 40°C/75% r.h | 90.2² | 91.8 |
| TRI50c Lysine Salt | capsules¹ | 40°C / 75% r.h | 90.2² | 90.6 |

| | | | | |
|---|---|---|---|---|
| **Notes:** 0) 1.5 month storage at given condinons, samples were then stored at room temperature until analytical testing. | | | | |
| 1) capsules stored at the respective dimatic conditions without blister. | | | | |
| 2) purity of the batch before storage. | | | | |
| 3) purity of the stored batch (capsules were poured out, the contents of the capsules were then analyzed). | | | | |
| 4) r.h. = relative humidity | | | | |

### Conclusion

There was no significant difference in the purities of the lysine salt at T₀ and T₁.

### 2. Analytical procedure

### 2.1 Sample preparation

### 2.1.1 Assay of TRI 50c and salts

TRI 50c-standard (free acid) was stored in a desiccator over phosphorus pentoxide for 2 days for drying. Afterwards, the reference standard was weighed in a volumetric flask and dissolved in a mixture of acetonitrile and water (25/75 v/v %). Aliquots of the resulting solution (ST 1A) were diluted successively with water as shown in the dilution scheme of table 4.

**Stock- and Calibration solutions of Tri 50c**

| | **Net weight** | **Purity** | **Salt-Factor** | **Dissolved** | **Solvent** | **Conc.** | **Calibr.** |
|---|---|---|---|---|---|---|---|
| | **mg** | **%** | | **in ml** | | **[µg/ml]** | **[µg/ml]** |
| **ST 1 A** | 40.8 | 98.23 | 1 | 10 | ACN/water 25/75 (v/v %) | 4007.8 | **C4000** |
| | | | | | | | |

| | **ml** | **ST** | **[µg/ml]** | **ad ml** | **Solvent** | **[µg/ml]** | |
|---|---|---|---|---|---|---|---|
| **ST 2 A** | 5 | 1 A | 4007,8 | 10 | water | 2003.9 | **C2000** |
| **ST 3 A** | 5 | 2 A | 2003,9 | 10 | water | 1001.9 | **C1000** |
| **ST 4 A** | 5 | 3 A | 1001,9 | 10 | water | 501.0 | **C500** |
| **ST 5 A** | 5 | 4 A | 500,9 | 10 | water | 250.5 | **C250** |
| **ST 6 A** | 1 | 3 A | 1001,9 | 10 | water | 100.2 | **C100** |
| **ST 7 A** | 1 | 6 A | 100,2 | 10 | water | 10.0 | **C10** |

### 2.1.2 Impurity profile of the stored capsules

The stored capsules of every batch at corresponding dimatic condition were removed and 10 mg of the content was weighed in a 10 ml volumetric flask and dissolved in 10 ml of a mixture of aoetonitrile/water (25/75 v/v%). These solutions were injected for impurity profile analysis and for quantification respectively.

### 3. Data evaluation

The quantitative evaluation and the impurity profile analysis were performed using an HPLC-PDA method. The processing wavelength was set as 258 nm.

### 4.Analytical parameters

### 4.1 Equipment and software

| | |
|---|---|
| Autosampler | Waters Alliance 2795 |
| Pump | Waters Alliance 2795 |
| Column oven | Waters Alliance 2795 |
| Detection | Waters 996 diode array, extracted wavelength 258 nm |
| Software version | Waters Millennium Release 4.0 |

### 4.2 Stationary phase

| | |
|---|---|
| Analytical Column ID | 571 |
| Material | X-Terra™ MS C₁₈, 5 µm |
| Supplier | Waters, Eschbom, Germany |
| Dimensions | 150 mm x 2.1 mm (length, internal diameter) |

### 4.3 Mobile Phase

Aqueous phase: A: 0.1% HCOOH in water
Organic phase: C: ACN
Gradient conditions:

| Time | Flow | % A | % C |
|---|---|---|---|
| 0.00 | 0.5 | 90 | 10 |
| 27.0 | 0.5 | 10 | 90 |
| 27.1 | 0.5 | 90 | 10 |
| 30.0 | 0.5 | 90 | 10 |

### EXAMPLE 29 - TRI 50B INHIBITION OF PLATELET PROCOAGULANT ACTIVITY

Platelet pro-coagulant activity may be observed as the increase, in rate of activation of prothrombin by factor Xa in the presence of factor Va upon the addition of platelets pretreated with thrombin, caused by thrombin alone, collagen alone or a mixture of thrombin and collagen. This property is due to an increase in anionic phospholipid on the surface of the platelet with concomitant release of microvesicle from the surface. This is an essential physiological reaction and people whose platelets have reduced ability to generate procoagulant activity (Scott syndrome) show an increased tendency for bleeding.

### Method:

Washed platelets were treated with either 1.15nM thrombin, 23µg/ml collagen or a mixture of both at the same concentration at 37°C. TRI 50b was added either for 1 minute prior to the addition of activator or immediately after the incubation with activator. Platelet procoagulant activity was determined as described previously (Goodwin C A et al, *Biochem J*. **1995** 8, 308: 15-21).

TRI 50b proved to be a potent inhibitor of platelet procoagulant activity with IC₅₀'s as summarised below:
Table 2: Influence of TRI 50b on the induction of platelet procoagulant activity by various agonists:

**Table 2**

| **Agonist** | **Fold acceleration without TRI 50b** | **IC50 plus pre-incubation (nM)** | **IC50 without incubation (nM)** |
|---|---|---|---|
| Thrombin | 30 | 8 | 3000 |
| Collagen | 45 | 200 | 300 |
| Thrombin/Collagen | 110 | 3 | 80 |

Table 2 records, for example, that when platelets were treated with thrombin they caused a 30-fold acceleration of the rate of activation of prothrombin in comparison with control platelets. Treatment with TRI 50 reduced such acceleration by half at the various TRI 50 concentration levels given. The significant potency of TRI 50 is evidenced by the fact that the IC₅₀ values are in the nanomolar range.

TRI 50b does not have an effect on ADP, collagen or epinephrine induced aggregation of washed platelets.

### EXAMPLE 30 - RABBIT EXTRACORPOREAL SHUNT MODEL

### Introduction

The technique describes an animal model in which a platelet rich thrombus is produced. The activity of TRI 50b and heparin are compared.

The carotid artery and jugular vein of anaesthetised rabbits were used to create an extracorporeal circuit containing a suspended foreign surface (silk thread). Thrombus deposition is initiated by creation of high sheer stress turbulent arterial blood flow, platelet activation, followed by coagulation in the presence of thrombagenic surfaces. Histopathological studies have shown that the thrombus is platelet rich.

### Materials and Methods

### Animals:

NZW rabbits (males 2.5-3.5 kg) were used. The animals were allowed food and water up to the induction of anaesthesia.

### Anaesthesia:

Animals were premedicated with fontanel/fluanisone (Hypnorm) 0.15 ml total by intramuscular injection. General anaesthesia was induced with methohexitone (10 mg/ml) to effect, followed by endotracheal intubation. Anaesthesia was maintained with isoflurane (1-2.0 %) carried in oxygen /nitrous oxide.

### Surgical Preparation:

The animals were placed in dorsal recumbency and the ventral cervical region prepared for surgery. The left carotid artery and right jugular vein were exposed. The artery was cannulated with a large Portex® catheter (yellow gauge), cut to a suitable length. The vein was cannulated with a Silastic® catheter. The shunt comprised of a 5 cm length of 'auto analyser' line (purple /white gauge). Joins to the shunt on the arterial side were made with intermediate size Silastic® tubing. The shunt was filled with saline before exposure to the circulation. The right femoral artery was cannulated for the measurement of blood pressure.

### Thread Preparation and insertion:

The central section of the shunt contained a thread 3 centimetres in length. This consisted of 000 gauge Gutterman sewing silk so as to give four strands with a single knot at the end. (The knot section was outside the shunt).

### Blood Flow

Blood flow velocity was determined by use of 'Doppler' probes (Crystal Biotech). A silastic probe was positioned over the carotid artery at the point of insertion of the arterial catheter. Flow was recorded on a chart recorder using heat sensitive paper.

### RESULTS

**Table 3**

| TREATMENT | DOSE | THROMBUS WEIGHT AFTER 20 minute run | ANTITHROMBOTIC ACTIVITY |
|---|---|---|---|
| Control | N/A | 22.4 ±2.2 mg (n=5) | |
| TRI 50b | 10mg/kg iv | 9.78 ±1.9 mg(n=5) | Active |
| | 3.0mg/kg iv | 15.3 ±2.2 mg(n=5) | Active |
| HEPARIN | 100 u/kg iv | 22.9 ±1.65 mg(n=4) | Inactive |
| | 300 u/kg iv | 10.5 ±1.4 mg (n=4) | Active (Severe bleeding) |

### Discussion

Table 3 shows that, under high arterial shear conditions, a TRI 50b dose of 3mg/kg to 10mg/kg iv significantly inhibits thrombus formation without bleeding, whereas a heparin dose within the normal clinical range for treating venous thrombosis (100u/kg iv heparin) was ineffective. The higher dose of heparin, though active, caused severe bleeding. These results, which show TRI 50b effectively inhibiting arterial thrombosis without causing bleeding, are consistent with TRI 50b inhibiting platelet procoagulant activity. In contrast, the thrombin inhibitor heparin, when administered at an approximately equi-effective dose (in terms of inhibition of arterial thrombosis), produced the severe bleeding normal when thrombin inhibitors are used to treat arterial thrombosis.

### EXAMPLE 31 - COMPARISON OF BLEEDING TIMES

The aim of the study was to compare the bleeding times of heparin with TRI 50b in a suitable model. It is accepted that heparin is a poor inhibitor of platelet procoagulant activity (*J. Biol. Chem*. **1978** Oct 10; 253(19):6908-16; Miletich JP, Jackson CM, Majerus PW1: *J. Clin. Invest*. **1983** May; 71(5):1383-91).

Bleeding times were determined in a rat tail bleeding model following intravenous administration of heparin and TRI 50b. The doses employed were chosen on the basis of their efficacy in the rat Wessler and dynamic models and were as follows:

| | |
|---|---|
| TRI 50b: | 5 and 10 mg/kg |
| Heparin: | 100 units/kg |

### MATERIALS AND METHODS

### Anaesthesia

Rats were anaesthetised with sodium pentabarbitone at 60 mg/kg (2.0 ml/kg of 30 mg/ml solution by ip. injection). Supplemental anaesthetic was given ip. as required.

### Surgical preparation

A jugular vein was cannulated for the administration of test compound. The trachea was also cannulated with a suitable cannula and the animals allowed to breathe 'room air' spontaneously.

### Compound administration

These were given in the appropriate vehicle at 1.0 ml/kg intravenously. Heparin was administered in saline, whilst TRI 50b was dissolved in ethanol, and then the resultant solution added to water for injection (1 part ethanol to 5 parts water).

### Technique

Two minutes following compound administration the distal 2mm of the animal's tail was sectioned with a new scalpel blade and the tail immersed in warm saline (37°C) contained in a standard 'universal' container, so that the blood stream was clearly visible. The bleeding time recording was started immediately following transection until the cessation of blood flow from the tip of the tail. A period of 30 seconds was allowed after the blood flow from the tail had stopped to ensure that bleeding did not re-commence, if bleeding did start again the recording time was continued for up to a maximum of 45 minutes.

### Results

Table 4 gives a summary of the bleeding results and shows the increases above base line values.

**Table 4**

| **Treatment** | **Bleeding time min (± SEM**^{**†**}**)** |
|---|---|
| Saline | 5.1 ± 0.6 |
| Heparin 100 u/kg iv | >40* |
| TRI 50b 5 mg/kg iv | 11.3 ± 1.2 |
| TRI 50b 10 mg/kg iv | 30.4 ± 5.2 |

| | |
|---|---|
| *Severe bleeding in all animals, with no cessation after 40 minutes. | |
| ^{†}SEM = standard error of the mean | |

### Discussion

The results show that TRI 50b was superior to heparin (produced less bleeding) at all doses. It should be noted that when 100 u/kg heparin is compared with 5 mg/kg TRI 50b, heparin-treated animals bled more extensively than those receiving TRI 50b; it was previously established (Example 25) that heparin at a dose of 100 u/kg is a less effective inhibitor of arterial thrombosis than TRI 50b at a dose of 3.0 mg/kg. Heparin is primarily a thrombin inhibitor and a poor inhibitor of platelet procoagulant activity; the results are therefore consistent with TRI 50b exerting anti-coagulant activity by inhibition of platelet coagulant activity in addition to thrombin inhibiting activity.

### EXAMPLE 32 - TRI 50B AS A PRODRUG FOR TRI 50C: PHARMACOKINETICS AND ABSORPTION

### MATERIALS AND METHODS

### Animals

Rats, body weight circa 250-300g were used. The animals were fasted only on the day of use for the iv stage. Animals were fasted on the night prior to study for the oral and intraduodenal studies, water was allowed up to the time of anaesthesia.

**Table 5: oral phase**

| **Treatment** | **Dose mg/kg po** | **n** |
|---|---|---|
| TRI 50b | 20mg/kg | 2 |
| TRI 50c | 20mg/kg | 2 |

**Table 6: intraduodenal phase**

| **Treatment** | **Dose mg/kg po** | **n** |
|---|---|---|
| TRI 50b | 20mg/kg | 3 |
| TRI 50c | 20mg/kg | 3 |

### Dose

### Formulation (TRI 5Db/TRI 50c)

These were dosed in a formulation prepared as follows: 48 mg/ml of TRI 50b is dissolved in ethanol: PEG 300 (2:3 vol: vol). Just before administration, 5 volumes of this solution is mixed with 3 volumes of 5% kollidon 17 8F.
1) Both compounds were dosed by oral gavage, or directly into the duodenum, at 20mg/kg,

The compounds were dosed in a PEG/ethanol/kollidon formulation which was prepared immediately before, as described immediately under the heading Stock 15.0mg/ml. This was dosed at 1.33ml/kg (equivalent to 30mg/kg).

### Methods

### Oral gavage

Rats were dosed at 20mg/kg. Approximately 30 minutes following dosing the rats were anaesthetised.

### Intraduodenal administration

The compounds were instilled directly into the duodenum after anaesthesia and surgical procedures had been completed.

### Blood sampling

### Oral phase

Blood (0.81ml) was taken from the carotid cannula into (0.09ml) of 3.8% w/v tri sodium citrate following anaesthesia and surgery. The first samples were taken one-hour post dose. Then at, 1.5, 2, 4 hours post dose.

### Intraduodenal phase

Blood samples were taken: Pre dose, then at 0.25, 0.5, 0.75, 1.0, 2, 3 and 4 hours post dosing.

### Plasma

This was obtained by centrifugation (3000 RPM for 10 min) and stored at -20°C prior to analysis.

### RESULTS

### PHARMACOKINETIC ANALYSIS

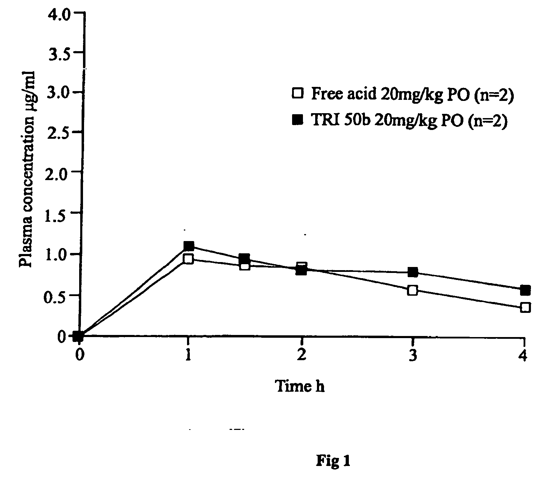

- Fig 1:: oral phase clearance and kinetics following dosing with TRI 50b or its free acid (TRI 50c).

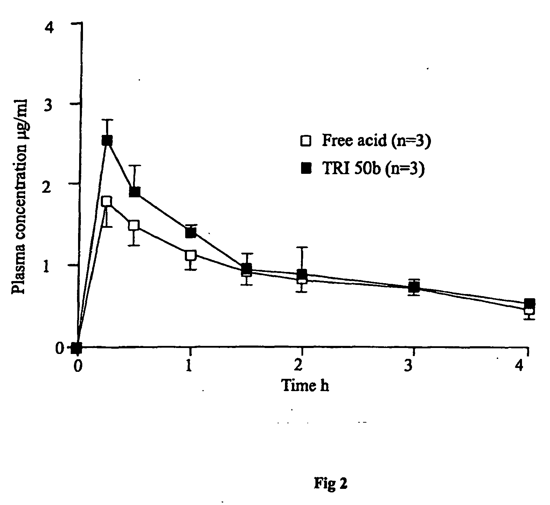

- Fig 2:: oral phase dearance and kinetics following intraduodenal dosing with TRI 50b or its free acid (TRI 50c).

### CONCLUSION

When given by the intraduodenal route TRI 50b achieved a higher bioavailability (peak plasma concentration) than the free acid. The data are consistent with TRI 50b being rapidly hydrolysed in plasma to TRI 50c and with TRI 50c being the active principle.

The results of examples 29 to 32 indicate that administration of TRI 50c as a salt will provide a way to treat arterial thrombosis and/or venous thrombosis.

### EXAMPLE 34 - Human Clinical Studies

In human clinical volunteer studies with doses of up to 2.5mg/kg i.v. (dosages which significantly prolong the thrombin clotting time), TRI 50b had no effect on Simplate bleeding time (i.e. bleeding time measured using a Simplate® bleeding time device).

It will be appreciated from the foregoing that the disclosure provides boronic acid salts useful for pharmaceutical purposes and which feature one or more of the following attributes: (1) improved amount of oral bioavailability; (2) improved consistency of oral bioavailability; (3) improved stability; and (4), in any event, not suggested by the prior art.

The selection of active ingredient for a pharmaceutical composition is a complex task, which requires consideration not only of biological properties (including bioavailability) but also of physicochemical properties desirable for processing, formulation and storage. Bioavailability itself is dependent on various factors, often including in vivo stability, solvation properties and absorption properties, each in turn potentially dependent on multiple physical, chemical and/or biological behaviours.

Advantageously, at least preferred products of the disclosure have adequate absorption and bioavailability. For commercial utility, a product having less good solubility may be selected by virtue of a superior overall combination of properties.

## Claims

1. A pharmaceutically acceptable base addition salt of a boronic acid of formula (I): 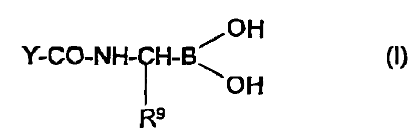 wherein
Y comprises a hydrophobic moiety which, together with the aminoboronic acid residue -NHCH(R⁹)-B(OH)₂, has affinity for the substrate binding site of thrombin; and
R⁹ is a straight chain alkyl group interrupted by one or more ether linkages and in which the total number of oxygen and carbon atoms is 3, 4, 5 or 6 or R⁹ is -(CH₂)ₘ-W where m is 2, 3, 4 or 5 and W is -OH or halogen (F, Cl, Br or I),
and, when YCO- is an optionally N-terminally protected dipeptide residue, the peptide linkages in the acid are optionally and independently N-substituted by a C₁-C₁₃ hydrocarbyl group optionally containing in-chain or in-ring nitrogen, oxygen or sulfur and optionally substituted by a substituent selected from halo, hydroxy and trifluoromethyl.

2. A salt of claim 1 wherein R⁹ is an alkoxyalkyl group.

3. A salt of claim 1 or claim 2 wherein all the peptide linkages in the acid are unsubstituted.

4. A salt of claim 1 or claim 2 wherein said C₁-C₁₃ hydrocarbyl group is a C₁-C₆ saturated hydrocarbyl group.

5. A salt of any of claims 1 to 4 wherein YCO- comprises an amino acid which binds to the S2 subsite of thrombin, the amino acid being N-terminally linked to a moiety which binds the 53 subsite of thrombin.

6. A salt of any of claims 1 to 4 wherein YCO- is an optionally N-terminally protected dipeptide which binds to the S3 and S2 binding sites of thrombin.

7. A salt of claim 6 wherein said dipeptide is N-terminally protected.

8. A salt of claim 6 or claim 7 wherein Y comprises a P3 residue which has a side chain of formula (B):
-(CO)ₐ-(CH₂)_{b}-D_{c}-Cₑ(E¹)(E²)(E³) (B)
wherein
a is 0 or 1;
e is 1;
b is 0 or an integer such that (b+e) is from 1 to 4;
c is 0 or 1;
D is O or S;
E¹, E² and E³ are each independently selected from -R¹⁵ and -J-R¹⁵, where J is a 5-6 membered ring and R¹⁵ is selected from C₁-C₆ trialkylsilyl, -CN, -R¹³, -R¹²OR¹³, -R¹²COR¹³, -R¹²CO₂R¹³, -R¹²O₂CR¹³, and one or two halogen atoms, wherein R¹² is -(CH₂)_{f}- and R¹³ is -(CH₂)_{g}H wherein f and g are each independently from 0 to 10, provided that (f+g) is 1, 2, 3 or 4.

9. A salt of daim 8 wherein, when E¹, E² or E³ contains an R¹³ group, g is 0 or 1.

10. A salt of any of claims 6 to 9 wherein YCO- comprises a P2 residue which is a residue of an imino acid.

11. A salt of any of claims 6 to 10 wherein the dipeptide residue comprises a P3 amino acid residue of (R)-configuration and a P2 residue of (S)-configuration, and wherein the residue -NHCH(R⁹)-B(OH) is of (R)-configuration.

12. A salt of any of claims 1 to 11 wherein the boronic acid has a Ki for thrombin of about 100 nM or less.

13. A salt of claim 12 wherein the boronic acid has a Ki for thrombin of about 20 nM or less.

14. A salt of claim 1 wherein the boronic acid is of formula (II): 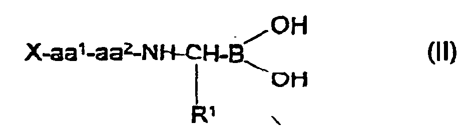 where:
X is H (to form NH₂) or an amino-protecting group;
aa¹ is an amino acid having a hydrocarbyl side chain containing no more than 20 carbon atoms and comprising at least one cyclic group having up to 13 carbon atoms;
aa² is an imino acid having from 4 to 6 ring members or is Gly N-substituted by a C₃-C₁₃ hydrocarbyl group;
R¹ is a group of the formula -(CH₂)ₛ-Z, where s is 2, 3 or 4 and Z is -OH, -OMe, -OEt or halogen (F, Cl, Br or I).

15. A salt of claim 14 wherein aa¹ is selected from Phe, Dpa and wholly or partially hydrogenated analogues thereof.

16. A salt of claim 14 wherein aa¹ is selected from Dpa, Phe, Dcha and Cha.

17. A salt of any of claims 14 to 16 wherein aa¹ is of (R)-configuration.

18. A salt of claim 14 wherein aa¹ is (R)-Phe or (R)-Dpa.

19. A salt of claim 14 wherein aa¹ is (R)-Phe.

20. A salt of any of claims 14 to 19 wherein aa² is a residue of an imino acid of formula (IV) 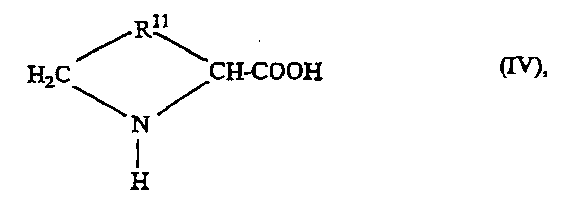 where R¹¹ is -CH₂-, -CH₂-CH₂-, -S-CH₂-, -S-C(CH₃)₂- or -CH₂-CH₂-CH₂-, which imino acid, when the ring is 5- or 6- membered, is optionally substituted at one or more -CH₂- groups by from 1 to 3 C₁-C₃ alkyl groups.

21. A salt of any of claims 14 to 20 wherein aa² is of (S)-configuration.

22. A salt of claim 20 wherein aa² is an (S)-proline residue.

23. A salt of claim 14, wherein aa¹-aa² is (R)-Phe-(S)-Pro.

24. A salt of any of claims 14 to 23 wherein the residue -NH-CH(R¹)-B(OH)₂ is of (R)-configuration.

25. A salt of any of claims 14 to 24 wherein R¹ is 2-bromoethyl, 2-chloroethyl, 2-methoxyethyl, 3-bromopropyl, 3-chloropropyl or 3-methoxypropyl.

26. A salt of any of claims 14 to 24 wherein R¹ is 3-methoxypropyl.

27. A salt of claim 14 which is a salt of a compound of formula (VIII):
X-(R)-Phe-(S)-Pro-(R)-Mpg-B(OH)₂ (VIII).

28. A salt of any of claims 14 to 27 where X is R⁶-(CH₂)ₚ-C(O)-, R⁶-(CH₂)ₚ-S(O)₂-, R⁶-(CH₂)ₚ₋NH-C(O)- or R⁶-(CH₂)ₚ-O-C(O)- wherein p is 0, 1, 2, 3, 4, 5 or 6 and R⁶ is H or a 5 to 13-membered cyclic group optionally substituted by 1, 2 or 3 substituents selected from halogen, amino, nitro, hydroxy, a C₅-C₆ cydic group, C₁-C₄ alkyl and C₁-C₄ alkyl containing, and/or linked to the cyclic group through, an in-chain O, the aforesaid alkyl groups optionally being substituted by a substituent selected from halogen, amino, nitro, hydroxy and a C₅-C₆ cyclic group.

29. A salt of claim 28 wherein said 5 to 13-membered cyclic group is aromatic or heteroaromatic.

30. A salt of claim 29 wherein said 5 to 13-membered cyclic group is phenyl or a 6-membered heteroaromatic group.

31. A salt of any of claims 28 to 30 wherein X is R⁶-(CH₂)ₚ-O-C(O)- and p is 0 or 1.

32. A salt of any of claims 14 to 26 wherein X is benzyloxycarbonyl.

33. A salt of claim 14 wherein the boronic acid is Cbz-(R)-Phe-(S)-Pro-(R)-Mpg-B(OH)₂.

34. A salt of any of claims 1 to 33 which does not comprise a choline or ammonium salt.

35. A salt of any of claims 1 to 34 which comprises boronate ions derived from the boronic acid and monovalent counter-ions.

36. A salt of any of claims 1 to 34 which is a salt of the peptide boronic acid with an alkali metal or a strongly basic organic nitrogen-containing compound.

37. A salt of claim 36 wherein the strongly basic organic nitrogen compound has a pKb of about 7 or more, e.g. about 7.5 or more.

38. A salt of any of claims 1 to 35 which is a salt of the boronic acid with a metal.

39. A salt of any of claims 1 to 33 or 37 which is a salt of the boronic acid with an alkali metal, an aminosugar, a guanidine or an amine of formula (XI): 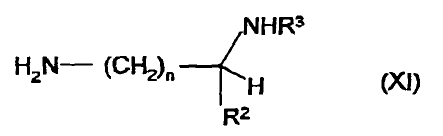 where n is from 1 to 6, R² is H, carboxylate or derivatised carboxylate, R³ is H, C₁-C₄ alkyl or a residue of a natural or unnatural amino acid.

40. A salt of any of claims 1 to 33 which is an L-arginine salt.

41. A salt of any of daims 1 to 33 which is an L-lysine salt.

42. A salt of any of daims 1 to 33 which is a potassium salt.

43. A salt of any of claims 1 to 33 which is a sodium salt.

44. A salt of any of claims 1 to 33 which is a lithium salt.

45. A salt of any of claims 1 to 33 which is a glucamine salt.

46. A salt of daim 45 wherein the glucamine is N-methyl-D-glucamine.

47. A salt of any of claims 1 to 46 which comprises boronate ions derived from the peptide boronic acid and has a stoichiometry consistent with the boronate ions carrying a single negative charge.

48. A salt of any of claims 1 to 47 wherein the salt comprises a boronate ion derived from the peptide boronic acid and a counter-ion and wherein the salt consists essentially of a salt having a single type of counter-ion.

49. A salt of any of claims 1 to 48 which is a monolithium, monosodium or monopotassium salt.

50. A salt of any of claims 28 to 33 which is a monolithium or monosodium salt.

51. A salt of any of daims 49 to 51 which is the monosodium salt.

52. A mono alkali metal salt of Cbz-(R)-Phe-(5)-Pro-(R)-Mpg-B(OH)₂.

53. The monosodium salt of Cbz-(R)-Phe-(S)-Pro-(R)-Mpg-B(OH)₂.

54. A salt of any of claims 1 to 53 which is in the solid phase.

55. A salt of any of claims 1 to 54 which comprises the boronate in anhydride form.

56. A product for use as a pharmaceutical, comprising a salt of any of claims 1 to 55.

57. A pharmaceutical formulation in oral dosage form comprising a salt of any of daims 1 to 56.

58. A formulation of claim 57 which further comprises a pharmaceutically acceptable diluent, excipient or carrier.

59. A formulation of claim 57 or claim 58 which is a solid formulation.

60. A pharmaceutical formulation which comprises a salt of any of claims 1 to 55, is adapted for oral administration, and comprises:
a) a first species selected from (a) boronic acids of formula (I) or, as the case may be, formula (II), (b) boronate anions thereof, and (c) any equilibrium form of the aforegoing (e.g. anhydride); and
(b) a second species selected from pharmaceutically acceptable metal ions and basic organic nitrogen-containing compounds having a pKb of about 7 or more (which compounds will be understood to comprise the compounds in protonated form),
wherein the formulation has an observed stoichiometry which would be consistent with the first species being boronate ions carrying a single negative charge.

61. The use of a salt of any of daims 1 to 55 or a formulation of any of claims 57 to 60 for the manufacture of a medicament for treating thrombosis by way of prophylaxis or therapy.

62. The use of claim 61 wherein the medicament is for treating a venous thromboembolic event, e.g: deep vein thrombosis or pulmonary embolism.

63. The use of a salt of any of claims 1 to 55 or a formulation of any of claims 57 to 60 for the manufacture of an oral medicament for preventing thrombosis in a haemodialysis circuit of a patient, for preventing a cardiovascular event in a patient with end stage renal disease, for preventing venous thromboembolic events in a patient receiving chemotherapy through an indwelling catheter, or for preventing thromboembolic events in a patient undergoing a lower limb arterial reconstructive procedures, or for treating by way of therapy or prophylaxis an arterial disease selected from acute coronary syndromes, cerebrovascular thrombosis, peripheral arterial occlusion and arterial thrombosis resulting from atrial fibrillation, valvular heart disease, arterio-venous shunts, indwelling catheters or coronary stents.

64. The use of a salt of any of claims 1 to 55 or a formulation of any of claims 57 to 60 for the manufacture of a medicament for anticoagulant treatment when blood is in contact with medical devices outside the body such as during cardiovascular surgery using a heart-lung machine or in haemodialysis.

65. An oral pharmaceutical formulation comprising a combination of (i) a salt of any of claims 1 to 55 and (ii) a further pharmaceutically active agent, e.g. another cardiovascular treatment agent, for example a lipid-lowering drug, a fibrate, niacin, a statin, a CETP inhibitor, a bile acid sequestrant, an anti-oxidant, a IIb/IIIa antagonist, an aldosterone inhibitor, an A2 antagonist, an A3 agonist, a beta-blocker, acetylsalicylic acid, a loop diuretic, an ace inhibitor, an antithrombotic agent with a different mechanism of action, an antiplatelet agent, a thromboxane receptor and/or synthetase inhibitor, a fibrinogen receptor antagonist, a prostacydin mimetic, a phosphodiesterase inhibitor, an ADP-receptor (P₂ T) antagonist, a thrombolytic, a cardioprotectant or a COX-2 inhibitor.

66. The use of a salt of any of claims 1 to 55 for the manufacture of a medicament for treating, for example preventing, a cardiovascular disorder in co-administration with another cardiovascular treatment agent, for example one listed in daim 65.

67. A oral medicament comprising a salt of a boronic acid which is a selective thrombin inhibitor and has a neutral aminoboronic acid residue capable of binding to the thrombin S1 subsite linked through a peptide linkage to a hydrophobic moiety capable of binding to the thrombin S2 and S3 subsites, the salt comprising a boronate species derived from the boronic acid and a cation having a valency n and having an observed stoichiometry consistent with a notional stoichiometry (boronic acid:cation) of n:1.

68. A medicament of claim 67 wherein the boronic acid has a Ki for thrombin of about 100 nM or less, and optionally of about 20 nM or less.

69. A pharmaceutical formulation adapted for oral administration and comprising a compound which is a source of boronate species corresponding to the acid Cbz-(R)-Phe-(S)-Pro-(R)-Mpg-B(OH)₂ and a source of pharmaceutically acceptable cations other than choline and ammonium.

70. The formulation of daim 69 wherein said compound is in the solid phase.

71. The formulation of claim 69 or claim 70 wherein the compound comprises anhydride species.

72. A method for forming a boronic acid salt as defined in any of claims 1 to 55, 67 or 68 comprising:
dissolving diethanolamine in an ether solution of a diol ester of the acid;
allowing or causing a precipitate to form and recovering the precipitate;
converting the precipitated material into the free organoboronic acid; and
reacting the organoboronic acid with a pharmaceutically acceptable base to form the salt.

73. A method of claim 72 wherein the ether is diethylether and the ester is a pinacol ester.

74. A method for making an oral pharmaceutical formulation, comprising:
contacting a boronic acid as defined in any of claims 1 to 33 with a pharmaceutically acceptable base having a pKb of 7 or more; and
formulating the resultant product into the formulation.

75. A method of claim 74 which further comprises obtaining the organoboronic acid by:
forming a solution of a diol ester of the boronic acid as defined in any of claims 1 to 33;
combining the solution with diethanolamine and allowing or causing the diethanolamine to react with the ester to form an insoluble precipitate;
recovering the precipitate;
converting the precipitate into the free boronic acid.

76. A method of daim 75 wherein the solvent of said solution is an ether.

77. A method of claim 75 or claim 76 wherein the ester is a pinacol ester.

78. A method of any of daims 74 to 77 wherein the base is an organic nitrogen-containing compound having a pKb of 7 or more or is a base of a metal.

79. A method of claim 78 wherein the organic base has a pKb of 7.5 or more and is not choline or ammonia.

80. A method of any of claims 74 to 77 wherein the base is of an alkali metal.

81. A method of daim 80 wherein the base is a sodium base.

82. A method of any of claims 74 to 81 wherein the boronic acid is as defined in any of daims 27 to 32.

83. A method of any of claims 74 to 81 wherein the boronic acid is Cbz-(R)-Phe-(S)-Pro-(R)-Mpg-B(OH)₂.

84. A method of any of claims 74 to 83 wherein said product comprises boronate anhydride species.

85. The use of a diol ester of a boronic acid as defined in any of claims 1 to 33 to make an oral pharmaceutical formulation by allowing the boronic acid, produced by hydrolysis of the diol ester or of a diethanolamine ester made by transesterification of said diol ester, to react with a pharmaceutically acceptable base as defined in any of claims 78 to 81 and formulating the resultant product into a pharmaceutical formulation.

86. The use of claim 85 wherein the diol ester is a pinacol, pinanediol or diethanolamine ester.

## Patentansprüche

1. Ein pharmazeutisch akzeptables Salz einer Boronsäure nach Zusatz einer Base mit der Formel (I): 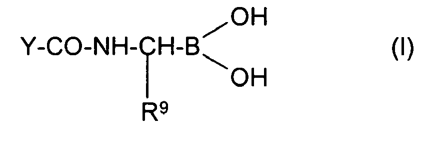 wobei
Y einen hydrophoben Anteil umfasst, der zusammen mit dem Aminoboronsäurerest -NHCH(R⁹)-B(OH)₂ Affinität zur Substratbindungsstelle von Thrombin aufweist; und
R⁹ eine geradkettige Alkylgruppe ist, die durch eine oder mehrere Etherbindungen unterbrochen wird und in der die Gesamtzahl von Sauerstoff- und Kohlenstoffatomen 3, 4, 5 oder 6 ist oder R⁹-(CH₂)ₘ-W ist, wobei m 2, 3, 4 oder 5 ist und W -OH oder Halogen (F, Cl, Br oder I) ist,
und, wenn YCO- ein optional N-terminal geschützter Dipeptidrest ist, die Peptidbindungen in der Säure optional und unabhängig durch eine C₁-C₁₃ Hydrocarbylgruppe N-substituiert werden, welche optional kettenförmigen oder ringförmigen Stickstoff, Sauerstoff oder Schwefel enthält, welche optional durch einen aus Halo-, Hydroxy- und Trifluormethyl ausgewählten Substituent substituiert werden können.

2. Ein Salz gemäß Anspruch 1, wobei R⁹ eine Alkoxyalkylgruppe ist.

3. Ein Salz gemäß Anspruch 1 oder Anspruch 2, wobei alle Peptidbindungen in der Säure unsubstituiert sind.

4. Ein Salz gemäß Anspruch 1 oder Anspruch 2 wobei die besagte C₁-C₁₃ Hydrocarbylgruppe eine C₁-C₆ gesättigte Hydrocarbylgruppe ist.

5. Ein Salz gemäß einem der Ansprüche 1 bis 4, wobei YCO- eine Aminosäure umfasst, die sich an die S2-Subsite von Thrombin bindet, wobei die Aminosäure N-terminal mit einem Anteil verknüpft ist, der die S3-Subsite von Thrombin bindet.

6. Ein Salz gemäß einem der Ansprüche 1 bis 4 wobei YCO- ein optional N-terminal geschütztes Dipeptid ist, das sich an die S3- und S2-Bindungsstellen von Thrombin bindet.

7. Ein Salz gemäß Anspruch 6 wobei das besagte Dipeptid N-terminal geschützt ist.

8. Ein Salz gemäß Anspruch 6 oder Anspruch 7, wobei Y einen P3-Rest umfasst, der eine Seitenkette der folgenden Formel (B) aufweist:
-(CO)ₐ-(CH₂)_{b}-D_{c}-Cₑ(E¹)(E²)(E³) (B)
wobei
a 0 oder 1 ist;
e 1 ist;
b 0 oder eine Ganzzahl ist, so dass (b+e) zwischen 1 und 4 liegt;
c 0 oder 1 ist;
D O oder S ist;
E¹, E² und E³ jeweils unabhängig voneinander aus -R¹⁵ and -J-R¹⁵ ausgewählt wurden, wobei J ein Ring mit 5-6 Gliedern ist und R¹⁵ aus C₁-C₆ Trialkylsilyl, -CN, -R¹³, -R¹²OR¹³, -R¹²COR¹³, -R¹²CO₂R¹³, -R¹²O₂CR¹³ und einem oder zwei Halogenatomen ausgewählt wird, wobei R¹² -(CH₂)_{f}- ist und R¹³ -(CH₂)_{g}H ist, wobei f und g jeweils unabhängig zwischen 0 und 10 liegen, vorausgesetzt, dass (f+g) 1, 2, 3 oder 4 ist.

9. Ein Salz gemäß Anspruch 8, wobei, wenn E¹, E² oder E³ eine R¹³-Gruppe enthalten, g 0 oder 1 ist.

10. Ein Salz gemäß einem der Ansprüche 6 bis 9, wobei YCO- einen P2-Rest umfasst, der ein Rest einer Iminosäure ist.

11. Ein Salz gemäß einem der Ansprüche 6 bis 10, wobei der Dipeptidrest einen Rest einer P3-Aminosäure mit (R)-Konfiguration und einen P2-Rest mit (S)-Konfiguration umfasst, wobei der Rest -NHCH(R⁹)-B(OH) eine (R)-Konfiguration aufweist.

12. Ein Salz gemäß einem der Ansprüche 1 bis 11, wobei die Boronsäure für Thrombin einen Ki-Wert von ungefähr 100nM oder weniger aufweist.

13. Ein Salz gemäß Anspruch 12, wobei die Boronsäure für Thrombin einen Ki-Wert von ungefähr 20nM oder weniger aufweist.

14. Ein Salz gemäß Anspruch 1, wobei die Boronsäure die folgende Formel (II) aufweist: 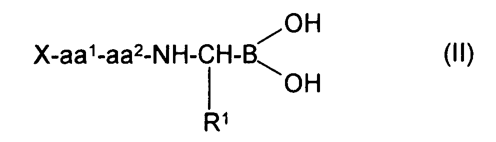 wobei:
X H (um NH₂ zu formen) oder eine Aminoschutzgruppe ist:
aa¹ eine Aminosäure ist, die eine Hydrocarbyl-Seitenkette aufweist, welche nicht mehr als 20 Kohlenstoffatome enthält und mindestens eine zyklische Gruppe umfasst, die bis zu 13 Kohlenstoffatome aufweist;
aa² eine Iminosäure mit 4 bis 6 Ringgliedern oder Gly ist, die durch eine C₃-C₁₃ Hydrocarbylgruppe N-substituiert wurde;
R¹ eine Gruppe mit der Formel -(CH₂)ₛ-Z ist, wobei s 2, 3 oder 4 ist und Z -OH, -OMe, -OEt oder Halogen (F, Cl, Br oder I) ist.

15. Ein Salz gemäß Anspruch 14, wobei aa¹ aus Phe, Dpa und ganz oder teilweise hydrogenisierten Analogen dieser ausgewählt wird.

16. Ein Salz gemäß Anspruch 14, wobei aa¹ aus Dpa, Phe, Dcha und Cha ausgewählt wird.

17. Ein Salz gemäß einem der Ansprüche 14 bis 16, wobei aa¹ eine (R)-Konfiguration aufweist.

18. Ein Salz gemäß Anspruch 14, wobei aa¹ (R)-Phe oder (R)-Dpa ist.

19. Ein Salz gemäß Anspruch 14, wobei aa¹ (R)-Phe ist.

20. Ein Salz gemäß einem der Ansprüche 14 bis 19, wobei aa² ein Rest einer Iminosäure mit der folgenden Formel (IV) ist: 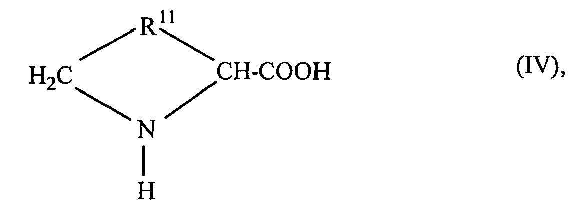 wobei R¹¹ -CH₂-, -CH₂-CH₂-, -S-CH₂-, -S-C(CH₃)₂- oder -CH₂-CH₂-CH₂- ist, wobei die Iminosäure, wenn der Ring 5 oder 6 Glieder aufweist, optional an einer oder mehreren -CH₂- Gruppen durch 1 bis 3 C₁-C₃ Alkylgruppen substituiert wird.

21. Ein Salz gemäß einem der Ansprüche 14 bis 20, wobei aa² eine (S)-Konfiguration aufweist.

22. Ein Salz gemäß Anspruch 20, wobei aa² ein (S)-Prolinrest ist.

23. Ein Salz gemäß Anspruch 14, wobei aa¹-aa² (R)-Phe-(S)-Pro ist.

24. Ein Salz gemäß einem der Ansprüche 14 bis 23, wobei der Rest -NH-CH(R¹)-B(OH)₂ eine (R)-Konfiguration aufweist.

25. Ein Salz gemäß einem der Ansprüche 14 bis 24, wobei R¹ 2-Bromethyl, 2-Chlorethyl, 2-Methoxyethyl, 3-Brompropyl, 3-Chlorpropyl oder 3-Methoxypropyl ist.

26. Ein Salz gemäß einem der Ansprüche 14 bis 24, wobei R¹ 3-Methoxypropyl ist.

27. Ein Salz gemäß Anspruch 14, das ein Salz einer Verbindung mit der Formel (VIII) ist:
X-(R)-Phe-(S)-Pro-(R)-Mpg-B(OH)₂ (VIII).

28. Ein Salz gemäß einem der Ansprüche 14 bis 27, wobei X R⁶-(CH₂)ₚ-C(O)-, R⁶-(CH₂)ₚ₋S(O)₂-, R⁶-(CH₂)ₚ-NH-C(O)- oder R⁶-(CH₂)ₚ-O-C(O)- ist, wobei p 0, 1, 2, 3, 4, 5 oder 6 ist und R⁶ H oder eine zyklische Gruppe mit 5 bis 13 Gliedern ist, die optional durch 1, 2 oder 3 Substituenten substituiert werden kann, die aus Halogen, Amino, Nitro, Hydroxy, einer zyklischen C₅-C₆-Gruppe, C₁-C₄-Alkyl, oder C₁-C₄-Alkyl, das entweder ein in der Kette enthaltenes O enthält und/oder durch dieses mit der zyklischen Gruppe verknüpft ist, ausgewählt werden, wobei die erwähnten Alkylgruppen optional durch einen Substituent substituiert werden können, der aus Halogen, Amino, Nitro, Hydroxy und einer zyklischen C₅-C₆-Gruppe ausgewählt werden kann.

29. Ein Salz gemäß Anspruch 28, wobei die besagte 5- bis 13-gliedrige zyklische Gruppe aromatisch oder heteroaromatisch ist.

30. Ein Salz gemäß Anspruch 29, wobei die besagte 5- bis 13-gliedrige zyklische Gruppe Phenyl oder eine 6-gliedrige heteroaromatische Gruppe ist.

31. Ein Salz gemäß einem der Ansprüche 28 bis 30, wobei X R⁶-(CH₂)ₚ-O-C(O)- und p 0 oder 1 ist.

32. Ein Salz gemäß einem der Ansprüche 14 bis 26, wobei X Benzyloxycarbonyl ist.

33. Ein Salz gemäß Anspruch 14, wobei die Boronsäure Cbz-(R)-Phe-(S)-Pro-(R)-Mpg-B(OH)₂ ist.

34. Ein Salz gemäß einem der Ansprüche 1 bis 33, das kein Cholin oder Ammoniumsalz umfasst.

35. Ein Salz gemäß einem der Ansprüche 1 bis 34, das von der Boronsäure derivierte Boronsäure-Ione und monovalente Gegenione umfasst.

36. Ein Salz gemäß einem der Ansprüche 1 bis 34, das ein Salz der Peptid-Boronsäure mit einem Alkalimetall oder einer stark basischen, organischen, Stickstoff enthaltenden Verbindung ist.

37. Ein Salz gemäß Anspruch 36, wobei die stark basische, organische Stickstoffverbindung einen pKb von ungefähr 7 oder mehr aufweist, z. B. ungefähr 7,5 oder mehr.

38. Ein Salz gemäß einem der Ansprüche 1 bis 35, das ein Salz der Boronsäure mit einem Metall ist.

39. Ein Salz gemäß einem der Ansprüche 1 bis 33 oder 37, das ein Salz der Boronsäure mit einem Alkalimetall, einem Aminozucker, einem Guanidin oder einem Amin mit der folgenden Formel (XI) ist: 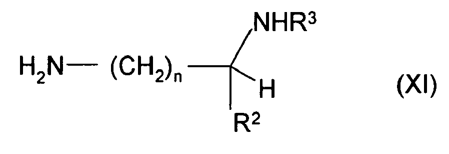 wobei n zwischen 1 uns 6 liegt, R² H, Carboxylat oder derivatisiertes Carboxylat ist, R³ H, C₁-C₄ Alkyl oder ein Rest einer natürlichen oder unnatürlichen Aminosäure ist.

40. Ein Salz gemäß einem der Ansprüche 1 bis 33, das ein L-Arginin-Salz ist.

41. Ein Salz gemäß einem der Ansprüche 1 bis 33, das ein L-Lysin-Salz ist.

42. Ein Salz gemäß einem der Ansprüche 1 bis 33, das ein Kaliumsalz ist.

43. Ein Salz gemäß einem der Ansprüche 1 bis 33, das ein Natriumsalz ist.

44. Ein Salz gemäß einem der Ansprüche 1 bis 33, das ein Lithiumsalz ist.

45. Ein Salz gemäß einem der Ansprüche 1 bis 33, das ein Glucaminsalz ist.

46. Ein Salz gemäß Anspruch 45, wobei das Glucamin N-Methyl-D-Glucamin ist.

47. Ein Salz gemäß einem der Ansprüche 1 bis 46, das aus der Peptid-Boronsäure derivierte Boronsäure-Ione umfasst und eine mit den Boronsäure-Ionen übereinstimmende Stöchiometrie aufweist, die eine einfache, negative Ladung tragen.

48. Ein Salz gemäß einem der Ansprüche 1 bis 47, wobei das Salz ein aus der Peptid-Boronsäure deriviertes Boronsäure-Ion und ein Gegenion umfasst und wobei das Salz im Wesentlichen aus einem Salz besteht, das nur eine Art von Gegenion aufweist.

49. Ein Salz gemäß einem der Ansprüche 1 bis 48, das ein Monolithium-, Mononatrium- oder Monokaliumsalz ist.

50. Ein Salz gemäß einem der Ansprüche 28 bis 33, das ein Monolithium- oder Mononatriumsalz ist.

51. Ein Salz gemäß einem der Ansprüche 49 bis 51, das ein Mononatriumsalz ist.

52. Ein Monoalkalimetallsalz der Formel Cbz-(R)-Phe-(S)-Pro-(R)-Mpg-B(OH)₂.

53. Ein Mononatriumsalz der Formel Cbz-(R)-Phe-(S)-Pro-(R)-Mpg-B(OH)₂.

54. Ein Salz gemäß einem der Ansprüche 1 bis 53, das sich in der Festphase befindet.

55. Ein Salz gemäß einem der Ansprüche 1 bis 54, das das Boronat in anhydrider Form umfasst.

56. Ein Produkt zur Verwendung als Pharmazeutikum, das ein Salz gemäß einem der Ansprüche 1 bis 55 umfasst.

57. Eine pharmazeutische Formulierung zur oralen Verabreichung, die ein Salz gemäß einem der Ansprüche 1 bis 56 umfasst.

58. Eine Formulierung gemäß Anspruch 57, die außerdem ein pharmazeutisch akzeptables Verdünnungsmittel, Bindemittel oder einen Träger umfasst.

59. Eine Formulierung gemäß Anspruch 57 oder Anspruch 58, die eine feste Formulierung ist.

60. Eine pharmazeutische Formulierung, die ein Salz gemäß einem der Ansprüche 1 bis 55 umfasst, für die orale Verabreichung angepasst wurde und folgendes umfasst:
a) eine erste Spezies, die aus (a) Boronsäuren mit der Formel (I) oder, gegebenenfalls der Formel (II), (b) Boronsäure-Anionen dieser und (c) einer Gleichgewichtsform der zuvor erwähnten (z. B. Anydrid) ausgewählt wird; und
(b) eine zweite Spezies, die aus pharmazeutisch akzeptablen Metallionen und basischen, organischen, Stickstoff enthaltenden Verbindungen ausgewählt wird, die einen pKb von ungefähr 7 oder mehr aufweisen (wobei diese Verbindungen die Verbindungen in protonierter Form umfassen sollen),
wobei die Formulierung eine beobachtete Stöchiometrie aufweist, die mit der ersten Spezies, welche aus Boronsäure-Ionen mit einer einfachen negativen Ladung besteht, übereinstimmen würde.

61. Die Verwendung eines Salzes gemäß einem der Ansprüche 1 bis 55 oder einer Formulierung gemäß einem der Ansprüche 57 bis 60 für die Herstellung eines Medikaments zur Behandlung von Thrombosen durch Prophylaxe oder Therapie.

62. Die Verwendung gemäß Anspruch 61, wobei das Medikament zur Behandlung eines venösen, thromboembolischen Ereignisses, z.B. tiefen Venenthrombosen oder pulmonaler Embolie dient.

63. Die Verwendung eines Salzes gemäß einem der Ansprüche 1 bis 55 oder einer Formulierung gemäß einem der Ansprüche 57 bis 60 zur Herstellung eines oralen Medikaments zur Vorbeugung von Thrombosen im Hämodialyse-Kreislauf eines Patienten, zur Vorbeugung eines kardiovaskulären Ereignisses bei einem Patienten mit chronischem Nierenversagen, zur Vorbeugung venöser thromboembolischer Ereignisse bei einem Patienten, der über einen Dauerkatheter chemotherapiert wird, oder zur Vorbeugung thromboembolischer Ereignisse bei einem Patienten, bei dem durch einen rekonstruktiven Eingriff Arterien in unteren Körpergliedern wiederhergestellt werden, oder zur Behandlung einer Arterienerkrankung ausgewählt aus akutem Koronarsyndrom, zerebrovaskulärer Thrombose, peripherem Arterienverschluss und arterieller Thrombose, die als Folge von Vorhofflimmern, Herzklappernerkrankung, arterio-venösen Shunts, Dauerkathetern oder Koronarstents auftreten können, durch Therapie oder Prophylaxe.

64. Die Verwendung eines Salzes gemäß einem der Ansprüche 1 bis 55 oder einer Formulierung gemäß einem der Ansprüche 57 bis 60 zur Herstellung eines Medikaments zur gerinnungshemmenden Behandlung, wenn Blut mit medizinischen Geräten außerhalb des Körpers in Berührung kommt, wie zum Beispiel während kardiovaskulären Operationen unter Verwendung einer Herz-Lungen-Maschine oder während der Hämodialyse.

65. Eine orale, pharmazeutische Formulierung, die eine Kombination (i) eines Salzes gemäß einem der Ansprüche 1 bis 55 und (ii) eines weiteren pharmazeutischen Wirkstoffs, z. B. eines weiteren Wirkstoffs zur kardiovaskulären Behandlung umfasst, beispielsweise ein lipidsenkendes Medikament, ein Fibrat, Niacin, ein Statin, einen CETP-Hemmstoff, ein Gallensäure-Maskierungsmittel, ein Antioxidans, einen IIb/IIIa Antagonisten, einen Aldosteron-Hemmstoff, einen A2-Antagonisten, einen A3-Agonisten, einen Beta-Blocker, Acetylsalizylsäure, ein Schleifendiuretikum, einen ACE-Hemmstoff, einen antithrombotischen Wirkstoff mit einem anderen Aktionsmechanismus, einen Antiplättchen-Wirkstoff, einen Thromboxan-Rezeptor und/oder Synthetase-Hemmstoff, einen Fibrinogen-Rezeptor-Antagonist, einen Prostazyklin nachahmenden Wirkstoff, einen Phosphodiesterase-Hemmstoff, einen ADP-Rezeptor (P₂T) Antagonisten, ein Thrombolytikum, ein Medikament zur Kardioprotektion oder einen COX-2 Hemmstoff.

66. Die Verwendung eines Salzes gemäß einem der Ansprüche 1 bis 55 für die Herstellung eines Medikaments zur Behandlung, zum Beispiel durch Vorbeugung, einer kardiovaskulären Störung durch gleichzeitige Verabreichung mit einem anderen Wirkstoff zur kardiovaskulären Behandlung, zum Beispiel einem der in Anspruch 55 aufgelisteten.

67. Ein orales Medikament, das ein Salz einer Boronsäure umfasst, welches ein selektiver Thrombinhemmstoff ist und einen neutralen Aminoboronsäurerest aufweist, der in der Lage ist, sich an die S1-Thrombin-Subsite zu binden, die über eine Peptidbindung mit einem hydrophoben Anteil verknüpft ist, der in der Lage ist, sich an die S2- und S3-Thrombin-Subsites zu binden, wobei das Salz eine Boronsäure-Spezies umfasst, die von der Boronsäure und einem Kation mit einer Valenz n deriviert wurde, wobei das Salz eine beobachtete Stöchiometrie aufweist, die einer fiktiven Stöchiometrie (Boronsäure:Kation) von n:1 entspricht.

68. Ein Medikament gemäß Anspruch 67, wobei die Boronsäure für Thrombin einen Ki-Wert von ungefähr 100 nM oder weniger aufweist und optional von ungefähr 20 nM oder weniger.

69. Eine pharmazeutische Formulierung, die für die orale Verabreichung angepasst wurde und eine Verbindung umfasst, die eine Quelle von Boronsäure-Spezies ist, die der Säure Cbz-(R)-Phe-(S)-Pro-(R)-Mpg-B(OH)₂ entsprechen und eine Quelle von pharmazeutisch akzeptablen Kationen ist, mit Ausnahme von Cholin und Ammonium.

70. Die Formulierung gemäß Anspruch 69, wobei die besagte Verbindung sich in der Festphase befindet.

71. Die Formulierung gemäß Anspruch 69 oder Anspruch 70, wobei die Verbindung anhydride Spezies umfasst.

72. Ein Verfahren zum Formen eines Boronsäuresalzes, wie es in Ansprüchen 1 bis 55, 67 oder 68 definiert wurde, umfassend:
Das Auflösen von Diethanolamin in einer Etherlösung eines Diolesters der Säure;
Das Zulassen oder das Anregen eines Niederschlags und die Rückgewinnung dieses Niederschlags;
Die Umwandlung des niedergeschlagenen Materials in die freie Organoboronsäure; und
Das Durchführen einer Reaktion zwischen der Organoboronsäure und einer pharmazeutisch akzeptablen Base zur Formung des Salzes.

73. Ein Verfahren gemäß Anspruch 72, wobei der Ether Diethylether ist und der Ester ein Pinacolester ist.

74. Ein Verfahren zur Herstellung einer oralen pharmazeutischen Formulierung, folgendes umfassend:
eine Boronsäure, wie in den Ansprüchen 1 bis 33 definiert, wird mit einer pharmazeutisch akzeptablen Base mit einem pKb von 7 oder mehr in Berührung gebracht; und
das entstehende Produkt wird in die Formulierung formuliert.

75. Ein Verfahren gemäß Anspruch 74, das außerdem die Gewinnung der Organoboronsäure wie folgt umfasst:
das Formen einer Lösung eines Diolesters der Boronsäure, wie in den Ansprüchen 1 bis 33 definiert;
das Kombinieren der Lösung mit Diethanolamin und das Zulassen oder Anregen einer Reaktion des Diethanolamins mit dem Ester, damit diese einen unlöslichen Niederschlag formen;
die Rückgewinnung des Niederschlags;
die Umwandlung des Niederschlags in die freie Boronsäure.

76. Ein Verfahren gemäß Anspruch 75, wobei das Lösungsmittel der besagten Lösung ein Ether ist.

77. Ein Verfahren gemäß Anspruch 75 oder Anspruch 76, wobei der Ester ein Pinacolester ist.

78. Ein Verfahren gemäß einem der Ansprüche 74 bis 77, wobei die Base eine organische, Stickstoff enthaltende Verbindung ist, die einen pKb von 7 oder mehr aufweist oder eine Base eines Metalls ist.

79. Ein Verfahren gemäß Anspruch 78, wobei die organische Base einen pKb von 7,5 oder mehr aufweist und nicht Cholin oder Ammoniak ist.

80. Ein Verfahren gemäß einem der Ansprüche 74 bis 77, wobei die Base die eines Alkalimetalls ist.

81. Ein Verfahren gemäß Anspruch 80, wobei die Base eine Natriumbase ist.

82. Ein Verfahren gemäß einem der Ansprüche 74 bis 81, wobei die Boronsäure so wie in den Ansprüchen 27 bis 32 definiert ist.

83. Ein Verfahren gemäß einem der Ansprüche 74 bis 81, wobei die Boronsäure Cbz-(R)-Phe-(S)-Pro-(R)-Mpg-B(OH)₂ ist.

84. Ein Verfahren gemäß einem der Ansprüche 74 bis 83, wobei das besagte Produkt anhydride Boronsäure-Spezies umfasst.

85. Die Verwendung eines Diolesters einer Boronsäure, wie in den Ansprüchen 1 bis 33 definiert, um eine orale pharmazeutische Formulierung zu produzieren, indem man die Boronsäure, die durch Hydrolyse des Diolesters oder eines Diethanolaminesters, der durch Transesterifizierung des besagten Diolesters hergestellt wird, mit einer pharmazeutisch akzeptablen Base, wie in Ansprüchen 78 bis 81 definiert reagieren lässt und das entstehende Produkt in eine pharmazeutische Formulierung formuliert.

86. Die Verwendung gemäß Anspruch 85, wobei der Diolester ein Pinacol-, Pinanediol- oder Diethanolaminester ist.

## Revendications

1. Sel d'addition avec une base pharmaceutiquement acceptable d'un acide boronique de formule (I): 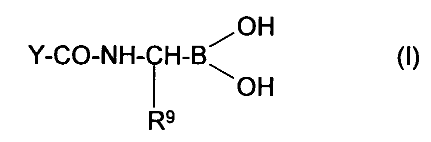 dans lequel :
Y comprend une fraction hydrophobe qui, en compagnie du résidu d'acide aminoboronique -NHCH(R⁹)-B(OH)₂, possède une affinité pour le site de liaison à un substrat de la thrombine; et
R⁹ est un groupe alkyle à chaîne droite interrompue par une ou plusieurs liaisons éther et dans lequel le nombre total d'atomes d'oxygène et de carbone est de 3, 4, 5 ou 6, ou R⁹ est -(CH₂)ₘ-W, m étant égal à 2, 3, 4 ou 5 et W étant un -OH ou un halogène (F, Cl, Br ou I),
et, lorsque YCO- est un résidu dipeptidique optionnellement protégé au niveau de la partie N-terminale, les liaisons peptidiques dans l'acide sont optionnellement et indépendamment N-substituées par un groupement hydrocarbyle en C₁-C₁₃ contenant optionnellement un azote, un oxygène ou un soufre dans la chaîne ou dans le cycle et optionnellement substituées par un substituant sélectionné parmi un halogène, un hydroxyle et un trifluorométhyle.

2. Un sel selon la revendication 1 dans lequel R⁹ est un groupement alkoxyalkyle.

3. Un sel selon la revendication 1 ou la revendication 2 dans lequel toutes les liaisons peptidiques dans l'acide sont non substituées.

4. Un sel selon la revendication 1 ou la revendication 2 dans lequel ledit groupement hydrocarbyle en C₁-C₁₃ est un groupement hydrocarbyle saturé en C₁-C₆.

5. Un sel selon l'une quelconque des revendications 1 à 4 dans lequel le YCO- comprend un acide aminé qui se lie au sous-site S2 de la thrombine, l'acide aminé étant lié par son extrémité N-terminale à une fraction qui se lie au sous-site S3 de la thrombine.

6. Un sel selon l'une quelconque des revendications 1 à 4 dans lequel le YCO- est un dipeptide optionnellement protégé au niveau de son extrémité N-terminale qui se lie aux sites de liaison S3 et S2 de la thrombine.

7. Un sel selon la revendication 6 dans lequel ledit dipeptide est protégé au niveau de son extrémité N-terminale.

8. Un sel selon la revendication 6 ou la revendication 7 dans lequel Y comprend un résidu P3 ayant une chaîne latérale de formule (B) :
-(CO)ₐ-(CH₂)_{b}-D_{c}-Cₑ(E¹)(E²)(E³) (B)
où :
a est égal à 0 ou 1;
e est égal à 1;
b est égal à 0 ou un nombre entier tel que (b+e) aille de 1 à 4;
c est égal à 0 ou 1;
D est O ou S;
E¹, E² et E³ sont chacun sélectionnés indépendamment parmi -R¹⁵ et -J-R¹⁵, J étant un cycle à 5-6 côtés et R¹⁵ étant sélectionné parmi un trialkylsilyl en C₁-C₆, -CN, -R¹³, -R¹²OR¹³, -R¹²COR¹³, - R¹²CO₂R¹³, -R¹²O₂CR¹³, et un ou deux atomes d'halogène, R¹² étant -(CH₂)_{f}- et R¹³ étant - (CH₂)_{g}H, f et g pouvant chacun indépendamment avoir une valeur de 0 à 10, pourvu que (f+g) soit égal à 1, 2, 3 ou 4.

9. Un sel selon la revendication 8 dans lequel, lorsque E¹, E² ou E³ contient un groupement R¹³, g est égal à 0 ou 1.

10. Un sel selon l'une quelconque des revendications 6 à 9 dans lequel YCO- comprend un résidu P2 qui est un résidu d'un imino-acide.

11. Un sel selon l'une quelconque des revendications 6 à 10 dans lequel le résidu dipeptide comprend un résidu d'acide aminé P3 de configuration (R)- et un résidu P2 de configuration (S)-, et dans lequel le résidu -NHCH(R⁹)-B(OH) a une configuration (R)-.

12. Un sel selon l'une quelconque des revendications 6 à 11 dans lequel l'acide boronique a un Ki vis-à-vis de la thrombine d'environ 100 nM ou moins.

13. Un sel selon la revendication 12 dans lequel l'acide boronique a un Ki vis-à-vis de la thrombine d'environ 20 nM ou moins.

14. Un sel selon la revendication 1 dans lequel l'acide boronique a la formule (II): 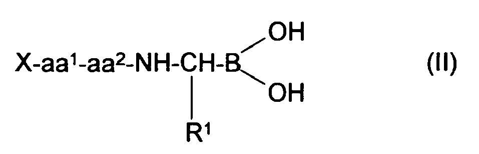 dans laquelle :
X est un H (pour former un NH₂) ou un groupe aminoprotecteur ;
aa¹ est un acide aminé ayant une chaîne latérale hydrocarbyle contenant au maximum 20 atomes de carbone et comprenant au moins un groupe cyclique ayant jusqu'à 13 atomes de carbone;
aa² est un imino-acide ayant un cycle à 4 à 6 côtés ou est Gly N-substitué par un groupement hydrocarbyle en C₃-C₁₃;
R¹ est un groupement de formule -(CH₂)ₛ-Z, s étant égal à 2, 3 ou 4 et Z étant un -OH, -OMe, - OEt ou un halogène (F, Cl, Br ou I).

15. Un sel selon la revendication 14 dans lequel aa¹ est sélectionné parmi Phe, Dpa et des analogues totalement ou partiellement hydrogénés de ces derniers.

16. Un sel selon la revendication 14 dans lequel aa¹ est sélectionné parmi Dpa, Phe, Dcha et Cha.

17. Un sel selon l'une quelconque des revendications 14 à 16 dans lequel aa¹ a une configuration (R)-.

18. Un sel selon la revendication 14 dans lequel aa¹ est un (R)-Phe ou (R)-Dpa.

19. Un sel selon la revendication 14 dans lequel aa¹ est un (R)-Phe.

20. Un sel selon l'une quelconque des revendications 14 à 19 dans lequel aa² est un résidu d'un imino-acide de formule (IV) 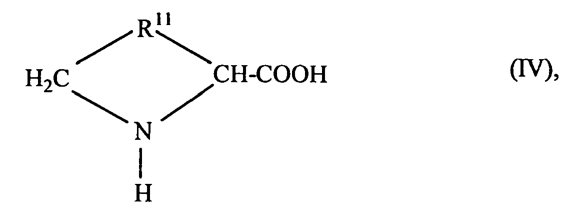 dans lequel R¹¹ est un -CH₂-, -CH₂-CH₂-, -S-CH₂-, -S-C(CH₃)₂- ou -CH₂-CH₂-CH₂-, lequel imino-acide, lorsque le cycle a 5 ou 6 côtés, est optionnellement substitué au niveau de un ou plusieurs groupements -CH₂- par de 1 à 3 groupes alkyle en C₁-C₃.

21. Un sel selon l'une quelconque des revendications 14 à 20 dans lequel aa² a une configuration (S).

22. Un sel selon la revendication 20 dans lequel aa² est un résidu de la (S)-proline.

23. Un sel selon la revendication 14, dans lequel aa¹-aa² est (R)-Phe-(S)-Pro.

24. Un sel selon l'une quelconque des revendications 14 à 23 dans lequel le résidu -NH-CH(R¹)-B(OH)₂ a la configuration (R).

25. Un sel selon l'une quelconque des revendications 14 à 24 dans lequel R¹ est un 2-bromoéthyle, 2-chloroéthyle, 2-méthoxyéthyle, 3-bromopropyle, 3-chloropropyle ou 3-méthoxypropyle.

26. Un sel selon l'une quelconque des revendications 14 à 24 dans lequel R¹ est un 3-méthoxypropyle.

27. Un sel selon la revendication 14 qui est un sel d'un composé de formule (VIII):
X-(R)-Phe-(S)-Pro-(R)-Mpg-B(OH)₂ (VIII).

28. Un sel selon l'une quelconque des revendications 14 à 27 dans lequel X est R⁶-(CH₂)ₚ-C(O)-, R⁶-(CH₂)ₚ-S(O)₂-, R⁶-(CH₂)ₚ-NH-C(O)- ou R⁶-(CH₂)ₚ-O-C(O)-, p étant égal à 0, 1, 2, 3, 4, 5 ou 6 et R⁶ étant un H ou un groupe cyclique à 5 à 13 côtés optionnellement substitué par 1, 2 ou 3 substituants sélectionnés parmi un halogène, un amino, un nitro, un hydroxy, un groupe cyclique en C₅-C₆, un alkyle en C₁-C₄ et un alkyle en C₁-C₄ contenant, et/ou lié au groupe cyclique par, un O dans la chaîne, les groupes alkyle précités étant optionnellement substitués par un substituant sélectionné parmi un halogène, un amino, un nitro, un hydroxy et un groupe cyclique en C₅-C₆.

29. Un sel selon la revendication 28 dans lequel ledit groupe cyclique à 5 à 13 côtés est aromatique ou hétéroaromatique.

30. Un sel selon la revendication 29 dans lequel ledit groupe cyclique à 5 à 13 côtés est un phényle ou un groupe hétéroaromatique hexagonal.

31. Un sel selon l'une quelconque des revendications 28 à 30 dans lequel X est R⁶-(CH₂)ₚ-O-C(O)- et p est égal à 0 ou 1.

32. Un sel selon l'une quelconque des revendications 14 à 26 dans lequel X est un benzyloxycarbonyle.

33. Un sel selon la revendication 14 dans lequel l'acide boronique est Cbz-(R)-Phe-(S)-Pro-(R)-Mpg-B(OH)₂.

34. Un sel selon l'une quelconque des revendications 1 à 33 ne comprenant pas un sel de choline ou un sel d'ammonium.

35. Un sel selon l'une quelconque des revendications 1 à 34 comprenant des ions boronate dérivés de l'acide boronique et des contre-ions monovalents.

36. Un sel selon l'une quelconque des revendications 1 à 34 qui est un sel de l'acide boronique peptidique avec un métal alcalin ou un composé organique contenant de l'azote et fortement basique.

37. Un sel selon la revendication 36 dans lequel le composé organique azoté fortement basique a un pKb d'environ 7 ou plus, p.ex. environ 7,5 ou plus.

38. Un sel selon l'une quelconque des revendications 1 à 35 qui est un sel de l'acide boronique avec un métal.

39. Un sel selon l'une quelconque des revendications 1 à 33 ou 37 qui est un sel de l'acide boronique avec un métal alcalin, un sucre aminé, une guanidine ou une amine de formule (XI): 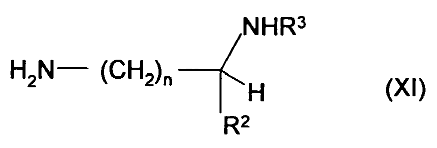 où n va de 1 à 6, R² est un H, un carboxylate ou un carboxylate dérivé, R³ est un H, un alkyle en C₁-C₄ ou un résidu d'un acide aminé naturel ou non naturel.

40. Un sel selon l'une quelconque des revendications 1 à 33 qui est un sel de la L-arginine.

41. Un sel selon l'une quelconque des revendications 1 à 33 qui est un sel de la L-lysine.

42. Un sel selon l'une quelconque des revendications 1 à 33 qui est un sel de potassium.

43. Un sel selon l'une quelconque des revendications 1 à 33 qui est un sel de sodium.

44. Un sel selon l'une quelconque des revendications 1 à 33 qui est un sel de lithium.

45. Un sel selon l'une quelconque des revendications 1 à 33 qui est un sel de glucamine.

46. Un sel selon la revendication 45 dans lequel la glucamine est la N-méthyl-D-glucamine.

47. Un sel selon l'une quelconque des revendications 1 à 46 comprenant des ions boronate dérivés du peptide acide boronique et ayant une stoechiométrie compatible avec le fait que les ions boronate portent une unique charge négative.

48. Un sel selon l'une quelconque des revendications 1 à 47 dans lequel le sel comprend un ion boronate dérivé de l'acide boronique peptidique et un contre-ion et dans lequel le sel consiste essentiellement en un sel ayant seul type de contre-ion.

49. Un sel selon l'une quelconque des revendications 1 à 48 qui est un sel monolithium, monosodique ou monopotassique

50. Un sel selon l'une quelconque des revendications 28 à 33 qui est un sel monolithium, ou monosodique.

51. Un sel selon l'une quelconque des revendications 49 à 51 qui est le sel monosodique.

52. Un sel monométallique alcalin de Cbz-(R)-Phe-(S)-Pro-(R)-Mpg-B(OH)₂.

53. Le sel monosodique de Cbz-(R)-Phe-(S)-Pro-(R)-Mpg-B(OH)₂.

54. Un sel selon l'une quelconque des revendications 1 à 53 qui est dans la phase solide.

55. Un sel selon l'une quelconque des revendications 1 à 54 qui comprend le boronate sous forme d'anhydride.

56. Product destiné à être utilisé comme produit pharmaceutique, comprenant un sel selon l'une quelconque des revendications 1 à 55.

57. Formulation pharmaceutique sous forme orale comprenant un sel selon l'une quelconque des revendications 1 à 56.

58. Formulation selon la revendication 57 comprenant en outre un diluant, excipient ou véhicule pharmaceutiquement acceptable,.

59. Formulation selon la revendication 57 ou la revendication 58 qui est une formulation solide.

60. Formulation pharmaceutique comprenant un sel selon l'une quelconque des revendications 1 à 55, adapté à une administration par voie orale, et comprenant :
a) une première espèce sélectionnée parmi (a) des acids boroniques ayant la formule (I) ou, selon le cas, la formule (II), (b) des anions boronate de celle-ci, et (c) n'importe quelle forme à l'équilibre des composés précités (p.ex. anhydride); et
(b) une deuxième espèce sélectionnée parmi des composés pharmaceutiquement acceptables organiques basiques contenant de l'azote et des ions métalliques ayant un pKb d'environ 7 ou plus (lesquels composés seront censés comprendre les composés sous forme protonée),
ladite formulation ayant une stoechiométrie observée compatible avec la première espèce, à savoir des ions boronate portant une unique charge négative.

61. Utilisation d'un sel selon l'une quelconque des revendications 1 à 55 ou d'une formulation selon l'une quelconque des revendications 57 à 60 pour la fabrication d'un médicament pour le traitement prophylactique ou thérapeutique des thromboses.

62. Utilisation de la revendication 61 dans laquelle le médicament sert à traiter un évènement thromboembolique veineux, p.ex. une thrombose veineuse profonde ou une embolie pulmonaire.

63. Utilisation d'un sel selon l'une quelconque des revendications 1 à 55 ou d'une formulation selon l'une quelconque des revendications 57 à 60 pour la fabrication d'un médicament oral pour prévenir les thromboses dans un circuit d'hémodialyse d'un patient, pour empêcher un évènement cardio-vasculaire chez un patient ayant une insuffisance rénale au stade terminal, pour empêcher un évènement thromboembolique veineux chez un patient recevant une chimiothérapie par l'intermédiaire d'un cathéter à demeure, ou pour empêcher des évènements thromboemboliques chez un patient subissant une procédure de reconstruction d'une artère d'un membre inférieur, ou pour traiter à titre thérapeutique ou prophylactique une maladie artérielle sélectionnée parmi les syndromes coronariens aigus, une thrombose cérébro-vasculaire, une occlusion artérielle périphérique et une thrombose artérielle résultant d'une fibrillation auriculaire, d'une valvulopathie cardiaque, de pontages artério-veineux, de cathéters à demeure ou de stents coronariens.

64. Utilisation d'un sel selon l'une quelconque des revendications 1 à 55 ou d'une formulation selon l'une quelconque des revendications 57 à 60 pour la fabrication d'un médicament pour un traitement anticoagulant lorsque le sang est en contact avec des dispositifs médicaux extracorporels comme lors d'une intervention cardio-vasculaire avec utilisation d'une machine coeur-poumon artificiel ou durant une hémodialyse.

65. Formulation pharmaceutique orale comprenant une combinaison (i) d'un sel selon l'une quelconque des revendications 1 à 55 et (ii) d'un principe pharmaceutiquement actif supplémentaire, p.ex. un autre agent de traitement cardio-vasculaire, par exemple un médicament hypolipémiant, un fibrate, de la niacine, une statine, un inhibiteur de la CETP, un séquestrant d'acide biliaire, un antioxydant, un antagoniste IIb/IIIa, un inhibiteur de l'aldostérone, un antagoniste A2, un agoniste A3, un béta-bloquant, l'acide acétylsalicylique, un diurétique de l'anse, un inhibiteur de l'enzyme de conversion, un agent antithrombotique avec un mécanisme d'action différent, un agent antiplaquettaire, un récepteur du thromboxane et/ou un inhibiteur de la synthétase, un antagoniste des récepteurs du fibrinogène, un agent mimétique de la prostacycline, un inhibiteur de la phosphodiestérase, un antagoniste des récepteurs ADP (P₂ T), un thrombolytique, un agent cardioprotecteur ou un inhibiteur COX-2.

66. Utilisation d'un sel selon l'une quelconque des revendications 1 à 55 pour la fabrication d'un médicament pour traiter, par exemple pour prévenir, un trouble cardio-vasculaire en co-administration avec un autre agent de traitement cardio-vasculaire, par exemple un de ceux énumérés dans la revendication 65.

67. Médicament oral comprenant un sel d'un acide boronique qui est un inhibiteur sélectif de la thrombine et a un résidu d'acide aminoboronique neutre capable de se lier au sous-site S1 de la thrombine lié par l'intermédiaire d'une liaison peptidique à une fraction hydrophobe capable de se lier aux sous-sites S2 et S3 de la thrombine, le sel comprenant une espèce boronate dérivée de l'acide boronique et un cation ayant une valence n et ayant une stoechiométrie observée compatible avec une stoechiométrie fictive (acide boronique:cation) de n:1.

68. Médicament selon la revendication 67 dans lequel l'acide boronique a un Ki vis-à-vis de la thrombine d'environ 100 nM ou moins, et optionnellement d'environ 20 nM ou moins.

69. Formulation pharmaceutique adaptée en vue d'une administration orale et comprenant un composé qui est une source d'une espèce boronate correspondant à l'acide Cbz-(R)-Phe-(S)-Pro-(R)-Mpg-B(OH)₂ et une source de cations pharmaceutiquement acceptables autres que choline et ammonium.

70. Formulation selon la revendication 69 dans laquelle ledit composé est dans la phase solide.

71. Formulation selon la revendication 69 ou la revendication 70 dans laquelle le composé comprend des espèces anhydride.

72. Méthode pour former un sel de l'acide boronique comme défini dans l'une quelconque des revendications 1 à 55, 67 ou 68
comprenant :
la dissolution de la diéthanolamine dans une solution dans l'éther d'un ester de diol de l'acide;
laisser ou faire se former un précipité et récupérer ce précipité;
la conversion du matériau précipité en l'acide organoboronique libre; et
la mise en réaction de l'acide organoboronique avec une base pharmaceutiqument acceptable pour former le sel.

73. Méthode selon la revendication 72 dans laquelle l'éther est un diéthyléther et l'ester est un ester du pinacol.

74. Méthode pour produire une formulation pharmaceutique orale, comprenant :
la mise en contact d'un acide boronique tel que défini dans l'une quelconque des revendications 1 à 33 avec une base pharmaceutiquement acceptable ayant un pKb de 7 ou plus; et
la formulation du produit résultant en la formulation.

75. Méthode selon la revendication 74 comprenant en outre l'obtention de l'acide organoboronique :
en formant une solution d'un ester de diol de l'acide boronique tel que défini dans l'une quelconque des revendications 1 à 33;
en combinant la solution avec de la diéthanolamine et en laissant ou en faisant en sorte que la diéthanolamine réagisse avec l'ester pour former un précipité insoluble;
en récupérant le précipité;
en convertissant le précipité en l'acide boronique libre.

76. Méthode selon la revendication 75 dans laquelle le solvant de ladite solution est un éther.

77. Méthode selon la revendication 75 ou la revendication 76 dans laquelle l'ester est un ester du pinacol.

78. Méthode selon l'une quelconque des revendications 74 à 77 dans laquelle la base est un composé organique contenant de l'azote ayant un pKb de 7 ou plus ou est une base d'un métal.

79. Méthode selon la revendication 78 dans laquelle la base organique a un pKb de 7,5 ou plus et n'est pas la choline ou l'ammoniac.

80. Méthode selon l'une quelconque des revendications 74 à 77 dans laquelle la base et une base d'un métal alcalin.

81. Méthode selon la revendication 80 dans laquelle la base est une base du sodium.

82. Méthode selon l'une quelconque des revendications 74 à 81 dans laquelle l'acide boronique est tel que défini dans l'une quelconque des revendications 27 à 32.

83. Méthode selon l'une quelconque des revendications 74 à 81 dans laquelle l'acide boronique est Cbz-(R)-Phe-(S)-Pro-(R)-Mpg-B(OH)₂.

84. Méthode selon l'une quelconque des revendications 74 à 83 dans laquelle ledit produit comprend l'espèce boronate anhydride.

85. Utilisation d'un ester de diol d'un acide boronique tel que défini dans l'une quelconque des revendications 1 à 33 pour produire une formulation que orale en faisant réagir l'acide boronique, produit par hydrolyse de l'ester de diol ou d'un ester de la diéthanolamine obtenu par transestérification dudit ester de diol, avec une base pharmaceutiquement acceptable telle que définie dans l'une quelconque des revendications 78 à 81 et en formulant le produit résultant en une formulation pharmaceutique.

86. Utilisation de la revendication 85 dans laquelle l'ester de diol est un ester du pinacol, du pinanediol ou de la diéthanolamine.
